(19) Europäisches Patentamt
European Patent Office
Office européen des brevets

(11) **EP 2 302 363 A2**

(12) **EUROPEAN PATENT APPLICATION**

(43) Date of publication:
**30.03.2011  Bulletin 2011/13**

(51) Int Cl.:
**G01N 21/47** (2006.01)  **G01N 33/68** (2006.01)
**G01N 33/543** (2006.01)

(21) Application number: **10183894.4**

(22) Date of filing: **05.09.2002**

(84) Designated Contracting States:
**AT BE BG CH CY CZ DE DK EE ES FI FR GB GR
IE IT LI LU MC NL PT SE SK TR**

(30) Priority: **05.09.2001  US 317543 P
12.03.2002  US 364962 P
24.04.2002  US 376049 P**

(62) Document number(s) of the earlier application(s) in
accordance with Art. 76 EPC:
**02768823.3 / 1 432 971**

(71) Applicant: **Life Technologies Corporation
Carlsbad, CA 92008 (US)**

(72) Inventors:
• **Bodzin, Leon J.
San Diego, CA 92128 (US)**

• **Yguerabide, Juan
La Jolla, CA 92037 (US)**
• **Warden, Laurence
Poway, CA 92064 (US)**
• **Anderson, Richard R.
Encinitas, CA 92024 (US)**
• **Rhodes, Kate
Poway, CA 92064 (US)**

(74) Representative: **Clarke, Jeffrey
Harrison Goddard Foote
40-43 Chancery Lane
London, Greater London WC2A 1JA (GB)**

Remarks:
This application was filed on 30-09-2010 as a
divisional application to the application mentioned
under INID code 62.

(54) **Method for normalization of assay data**

(57)  The present invention relates to a method of normalizing assay data, comprising: selecting a first population of assay data and a second population of assay data, wherein the first population comprises a dependent set of controls and the second population comprises an independent set of controls; obtaining a linear relationship between the independent set of controls and the dependent set of controls; and applying the linear relationship to the first population, thereby producing normalized assay data. The invention is of particular applicability to assays that use resonance light scattering particles.

EP 2 302 363 A2

**Description**

CROSS-REFERENCE TO RELATED APPLICATIONS

This application claims priority to provisional U.S. applications, serial no. 60/317,543, filed on September 5th 2001, entitled "Apparatus for Analyte Assays", serial no. 60/364,962, filed March 12, 2002, entitled "Multiplexed Assays Using Resonance Light Scattering Particles," and serial no. 60/376,049, filed April 24, 2002, entitled "Signal Generation and Detection System for Analyte Assays," all of which are incorporated herein by reference in their entirety.

FIELD OF THE INVENTION

The present invention generally relates to methods of processing data obtained from assay measurements on analytes. Specifically the present invention provides methods and apparatus for correlating measured light intensity with analyte concentration and for normalization of data across microarray samples. The invention is of particular applicability to assays that use resonance light scattering particles.

BACKGROUND OF THE INVENTION

Binding-pair techniques play an important role in many applications of biomedical analysis and are gaining importance in the fields of environmental science, veterinary medicine, pharmaceutical research, food and water quality control and the like. Such techniques rely upon an interaction, usually reversible, between a molecule of sample, and a label, or probe, that is based upon molecular recognition. The interaction may be highly specific, for example having the character of a ligand-receptor binding event, or may involve use of a substance that has very broad binding capabilities.

For the detection of analytes at low concentrations (less than about 1 picomole analyte per volume of sample analyzed), fluorescent, isotopic, luminescent, chemiluminescent, or electrochemiluminescent labels and accompanying specific detection methods are often used. Such methods are able to achieve detection of low concentrations of analytes by amplifying many-fold the number of luminescent molecules or photon-generating events. However, those methods suffer from a number of drawbacks, which makes the detection of analytes complicated, difficult, time consuming, and costly. Not least of these drawbacks are problems of interference of chemical or enzymatic reactions, contamination, complicated and multi-step work-up and analysis procedures, limited adaptability to single step "homogeneous", non-separation, formats, and the requirement of costly and sophisticated instrumentation.

Recently a particularly advantageous method of detecting analytes using submicroscopic (typically nanometer-sized) metal colloidal particles as labels has been developed. The detection and/or measurement of the light-scattering properties of the particles is correlated to the presence and/or amount, or absence, of one or more analytes in a sample. Analyte detection using such a technique, and an apparatus for carrying out analyte assays, are described in, for example, Yguerabide et al., U.S. Patent No. 6,214,560, and international applications PCT/US/97/06584 (WO 97/40181), PCT/US98/23160 (WO 99/20789), and U.S. provisional patent application serial number 60/317,543 (filed September 5, 2001), each of which is incorporated by reference herein in its entirety. Elements of the basic principles behind this technology known as resonance light scattering (RLS) technology, are also described in the two publications: Yguerabide & Yguerabide, Anal. Biochem., 261:157-176, (1998); and Anal. Biochem., 262:137-156, (1998). It finds application to a wide range of situations including those where, hitherto, fluorescent labels such as fluorescein have been employed.

RLS technology is based on physical properties of metal colloidal particles. These particles are typically nanometer-sized and, when illuminated with polychromatic light, preferentially scatter incident radiation in a manner consistent with electromagnetic theory known as resonance light scattering. The light produced by sub-microscopic RLS particles arises when their electrons oscillate in phase with incident electromagnetic radiation. The resulting scattered light is in the visible range and is highly intense, often being at least several orders of magnitude greater than fluorescence light when compared on a per label basis. The level of intensity and color is determined largely by particle composition, size and shape.

In contrast to the use of fluorescent labels, where the analyte binds to a fluorescent molecule, or tag, whose fluorescence is detected, the principle behind RLS is that those analytes are bound to at least one detectable light scattering particle with a size smaller than the wavelength of the illuminating light. These particles are illuminated with a light beam under conditions where the light scattered by the particle can be detected. The scattered light detected under those conditions is then a measure of the presence of the one or more analytes in a sample. The method of light illumination and detection has been referred to as DLASLPD ("Direct Light Angled for Scattered Light only from Particle Detected") in U.S. Patent No. 6,214,560 wherein a number of methods and configurations for efficient dark field illumination are described.

By ensuring appropriate illumination and maximal detection of specific scattered light, the result is an extremely sensitive method of detection that can enable detection of one or more analytes to very low concentrations. In fact, the light scattering power of a 60 nm gold particle is equivalent to the fluorescent light emitted from about 500,000 fluorescein molecules. It has been found that, in suspension, 60 nm gold particles can be detected by the naked eye, through observation of scattered light, at a concentration down to $10^{-15}$ M. Indeed, ultra-sensitive qualitative solid phase assays can be conducted with the naked eye, enabling detection of as little as $10^{-18}$ moles of analyte in 100 $\mu$l of analyte sample using integrated light intensity. Furthermore, single particle detection is possible by the human eye with less than 500 times magnification as viewed through an optical microscope. For example, individual gold particles can readily be seen in a student microscope with simple dark field illumination.

Additional benefits of RLS particles include the fact that they do not photobleach, the color of the scattered light can be changed by altering particle composition or particle size, and the particles can be coated with antibodies or DNA probes for detection of specific analyte antigens or DNA sequences. Furthermore, RLS particles offer a broad dynamic range: by judicious choice of integrated light intensity measurements or direct observation by eye, analyte can be detected over three decades of analyte concentration, and the region of dynamic range can be adjusted by changing the particle size. RLS particles are also compatible with homogeneous assays, for example in solution, or in solid phase assays wherein very high sensitivity can be obtained through particle counting. In short, ultra-sensitive quantitative assays can be conducted with relatively simple instrumentation.

Nevertheless, detection of RLS particles with conventional laser scanners entails some technical limitations because the particles emit the same wavelength light as the excitation source. Laser scanners are based on the "Stokes shift" phenomenon of fluorescence, in which the emitted light from a fluorescent molecule is of a longer wavelength than that of the excitation light. Laser scanners are designed to block the excitation spectrum from the detection system, allowing only the emission from the fluorescent molecule to be sensed by the detector. Therefore, because existing laser based systems are tailored to fluorescence labeling, specific instrumentation has been developed to analyze microarrays labeled with RLS particles. In practice, if monochromatic laser light is used, illumination in a dark field configuration works well. The principal limitation is that any incident light entering the optics on the detection side is indistinguishable from the scattered light signal because the wavelengths of the scattered light and the laser light are the same.

In essence, RLS technology can detect low concentrations of analytes without the need for signal or analyte molecule amplification. Furthermore, the method provides a simplified procedure for the detection of analytes wherein the amount and types of reagents are reduced relative to other methods in the art. The method also enables substantial reductions in the number of different tests to be carried out (e.g., through application of specially developed multiplex assays - assays that permit detection of multiple analytes using, for example, multiple labels; see for example, U.S. patent 6,214,560), thereby leading to reduced costs and lower production of waste, especially medically-related waste that must be disposed of by specialized means.

The wide range of specific light scattering signals from different particle types means that one skilled in the art can in principle detect and measure to a high degree of specificity one or more analytes in a sample. Where there is high optical resolvability of two or more different particle types there is the potential for very simple multi-analyte detection (*i.e.,* simultaneous detection of two or more different analytes) in a sample without the need for complex apparatus. Furthermore, the use of specific particle types that possess highly measurable and detectable light scattering properties in a defined assay format enables ready application of the method to micro-array and other high-throughput techniques. However, in general, mixtures of RLS particle types have a composite spectra equal to the linear summation of proportional amounts of component reference spectra, according to the relative abundance of the particles. In general, then, a process of multispectral deconvolution, or unmixing, becomes necessary to deconvolve a mixture of component spectra so that one RLS particle type can be discriminated from another.

The problem of unmixing spectra is not unlike those found in the discipline of "remote sensing" and "image exploitation". In these applications, observations of the Earth are captured with multi- and hyper-spectral satellite sensors which gather tens to hundreds of spectral images and then unmix them using linear unmixing software. However, Earth observations largely consist of unknown materials whose spectra are ambiguous. Accurate determination of material abundance from these observations is difficult. This is especially due to the variance in the reference spectra used and the number of unknown references in the image. This situation compounds the overall problem because the solution becomes under-determined since there are fewer reference materials than there are materials in the image.

Research and development in the remote sensing field is ongoing, and much of it is devoted to exploring ways to improve spectral unmixing. Example reports that highlight some of the problems and issues being encountered in the field, include: Chewings, V. H., Pickup, G., Bastin, G. N. and Pearce, G., "The potential for hyperspectral data mapping in Australian arid-zone vegetation 10th Australasian Remote Sensing and Photogrammetry Conference," Adelaide, South Australia, 851-863, (2000), in which research tested how well individual spectral bands and indices derived from discrete wavelengths were able to differentiate different tree and shrub species, and soil and litter. Results from this preliminary study indicated that major components of an arid landscape, such as green trees, dark shrubs, soil and to some extent litter, can be mapped using hyperspectral data. However, attempts to "unmix" pixels into these component parts were only partially successful, probably due to insufficient knowledge of the full range of spectral characteristics of the target objects.

Hermann, J. A. and Shettigara, V. K. and Hawthorn, C., "Full and partial unmixing of hyperspectral images for target detection," Proc. 10th Australasian Remote Sensing and Photogrammetry Conference, Adelaide, (2000), described significant efforts being expended on improving the accuracy of the unmixing processes utilized in hyperspectral sensing for target detection. It was suggested that *a priori* knowledge of the spectral signatures is essential for ensuring the success of unmixing processes. Spectral signature variability is another factor which may seriously limit the success unmixing procedures. When the entire set of spectral signatures and their variability is accurately known then successful unmixing can be achieved.

Shettigara, V. K., O'Mara, D., Bubner, T. and Kempinger, S. G., "Hyperspectral band selection using entropy and target to clutter ratio measures," Proc. 10th Australasian Remote Sensing and Photogrammetry Conference, Adelaide, (2000), demonstrated that unmixing is a popular method for target detection in hyperspectral images. The high dimensionality of the hyperspectral data is a serious challenge to the operational use of the technology. For this reason it is important to investigate if unmixing can be achieved using fewer bands.

Nevertheless, unmixing methods may have promise for, or be adaptable to, RLS particle analysis because a limited number of known materials (RLS particles) are in the image, and their reference spectra are accurately known or can be determined.

To date, the RLS particle method for analyte detection has found application to techniques including, but not limited to: *in vitro* immunoassays; *in vitro* DNA probe assays; gene expression microarrays; DNA sequencing microarrays; protein microarrays; immunocytology; immunohistochemistry; *in situ* hybridization; multiplexed (multicolor) assays; homogeneous immuno, and DNA probe assays; and microfluidic, immuno, and DNA probe assay systems as described in Bao, P., Frutos, A., Greef, C., Lahiri, J., Muller, U., Peterson, T., Warden, L., Xie, X., "High-Sensitivity detection of DNA hybridization on microarrays using resonance light scattering", Anal. Chem., 74:1792-1797, (2002). Microarray technology has been described in: Schena, M., Shalon, D., Davis, R. W., and Brown, P. O., "Quantitative monitoring of gene expression patterns with a complementary DNA microarray", Science, 270:467-70, (1995).

A particularly important area for application of microarrays is gene expression analysis. A basic problem in expression analysis is the determination of gene regulation profiles while compensating for unassociated assay and system variations. Gene regulation profiles are used to determine the degree to which a particular sample of genetic material is expressed relative to another, under controlled experimental conditions. However, independent assay and system variations conspire to obstruct accuracy in such comparative expression studies.

Sources of variation are experiment dependent. A list of commonly contributing factors from Schuchhardt, J., Beule, D., Malik, A., et al., "Normalization Strategies for cDNA Microarrays," Nucl. Acids Res., 28: e47, (2000) is included hereinbelow. This list of factors addresses fluctuations in probe, target and array preparation, in the hybridization process, background and overshining effects and effects resulting from image processing, but is not intended to be an exclusive list.

1. mRNA preparation: depending on tissue and sensitivity to RNA degradation, probes may appear different from sample to sample.
2. Transcription: reverse transcription to cDNA will result in DNA species of varying lengths.
3. Labeling: radioactive labeling may fluctuate randomly and systematically depending on nucleotide composition.
4. Amplification: clones are subject to PCR amplification, which is difficult to quantify and may fail completely.
5. Systematic variations in pin geometry: pins have different characteristics and surface properties and therefore transport different amounts of target cDNA.
6. Random fluctuations in target volume: the amount of transported target fluctuates stochastically even for the same pin.
7. Target fixation: the fraction of target cDNA that is chemically linked to the slide surface from the droplet is unknown.
8. Hybridization parameters: efficiency of the hybridization reaction is influenced by a number of experimental parameters, notably temperature, time, buffering conditions and the overall amount of probe molecules used for hybridization.
9. Slide inhomogeneities: for different reasons the probe may be distributed unequally over the slide or the hybridization reaction may perform differently in different parts of the slide.
10. Non-specific hybridization: a typical source of error that cannot be completely excluded.
11. Non-specific background and overshining: non-specific radiation and signals from neighboring spots.
12. Image analysis: non-linear transmission characteristics and saturation effects and variations in spot shape.

Although such system variations can derive from numerous sources located throughout the entire experimental process, their aggregate effect is felt most during the quantification stage. The result of quantification is usually a set of numerical measures that represent the end point of the biochemical stages of the analysis, and which are subsequently subjected to a number of data processing steps.

In particular, there are inherent difficulties in accurately correlating a measured signal with the quantity of analyte that gives rise to the signal. There is also no guarantee of reproducibility, *i.e.*, that equivalently prepared microarray features (such as spots) produce equivalently quantified expression values. Furthermore, there are problems of consistency between samples, both different samples in the same array and between samples on different arrays. Thus, quantitative analyte detection, which is so vital to areas such as gene expression analysis, is often confounded by an inability to accurately gauge analyte presence in any given sample and to make reliable comparisons between different samples.

Measured data values have absolute units and are tied to the entire population of microarray features from which they came. For the purpose of data processing, it is desirable to cancel out absolute units from the measurement in order to enable accurate comparison with expression values from other populations. This can be achieved by so-called normalization methods. In order to produce a unitless set of data, a normalization method must consistently produce a transformation. Two forms of normalization method are known in the art. One form is where controls are used to guide the normalization process, and the other is where no controls are used.

The drawback of existing normalization methods that do not use controls is that an analytic scheme must reverse-engineer a data model that itself attempts to accurately describe the underlying data. Such a model is either based on theory or is empirically derived from each data set that is considered. Ultimately, the data undergoing analysis is normalized to the theoretical or derived model, or compensated for in some way that is based on it. Subsequent ratiometric analysis is conducted relative to the model. Such an approach rests on the assumption that the theoretical or empirically derived model is in fact accurate. This assumption must be validated at some point in the analysis in order to be confident in the results. However, without controls at one's disposal to help reverse the effects of systematic and protocol induced variance, it may never be possible to have a sound model.

By contrast, an advantage of using controls is that a model can be derived directly from data that has been obtained during the course of the experiment and thus has experienced many of the variations to which the actual measured data has been subject. Nevertheless, existing normalization methods that use controls are still imperfect.

For example, Schuchhardt et al., Nucl. Acids Res., 28: e47, (2000), described the use of averaging as an approach to normalization of data across an array, but did not provide a closed solution. They described a technique in which they "divide the signal intensity of each spot by the average intensity of all control spots on the slide", a quantity referred to as a control data average. Schuchhardt *et al.* describe their technique as an "intuitive normalization procedure" because using an average intensity was expected to desensitize results from sources of noise by reducing the effect of stochastic fluctuations, such as those associated with inhomogeneities in hybridization. However, by using control data averages, there is still no guarantee that the normalized set of data will be characterized reproducibly in a consistent manner because all the control features are assumed to express equivalently. Unless this is reliably true all the time, normalization performance cannot be relied up on to guarantee a consistent solution.

Other methods of standardizing assay data using controls have been proposed. For example, in the publication, Colantuoni, C., Henry, G., Zeger, S., Pevsner, J., "Local mean normalization of microarray element signal intensities across an array surface: quality control and correction of spatially systematic artifacts," Biotechniques, 32(6):1316-20, (Jun, 2002), a method that utilizes local controls is used so that control data averages are calculated over immediate neighbors of a given spot. This method therefore suffers from the same drawback as that of Schuchhardt *et al.* A further method, Finkelstein et al., "Iterative linear regression by sector: renormalization of cDNA microarray data and cluster analysis weighted by cross homology", Critical Assessment of Microarray Data Analysis, Duke University, (2000), is based on an assumption that the data is or should have a linear relationship with respect to intensity. The method then takes an iterative approach, selectively eliminating data points that deviate substantially from linearity. This method suffers from the drawback that intensity data may not obey a linear relationship with respect to analyte concentration and also that removing data points is unsatisfactory because of the associated loss of information. Other approaches to normalization of array data have been developed, as demonstrated in the product ArrayStat®, by Imaging Research, Inc., which is directed towards quality assurance.

A hitherto unexploited advantage of controls is that an equivalence model relationship between pairs of controls can be obtained, thereby bringing validation to the fore of the analysis. In this way, the model is not assumed, but rather is enforced in the process of defining it. The model is not dependent on whether, say, intensity is proportional to RLS particle concentration, but rather only depends on the assumption that equally prepared assays should express equally. Accordingly, the art lacks a normalization method that consistently produces the same effect and guarantees that equivalently prepared microarray features will produce equivalently quantified expression values across the entire range of control features.

Thus there remains a need for a flexible, highly sensitive and automated signal generation and detection system that is capable of capturing, processing, analyzing, and comparing various samples of analytes, in a number of formats. The commercial availability of such a system would have wide applicability and would benefit research and industry in many ways including application to microarrays, biochips, genomics, proteomics, combinatorial chemistry and high throughput screening (HTS).

SUMMARY OF THE INVENTION

The present invention involves an assay system, comprising: at least one type of light scattering particles configured to be bound to an analyte of interest in a sample; and a scattered light detector configured to analyze analytes in the sample based on detected scattered light of at least first and second colors from the sample. The assay system is further such that the scattered light detector comprises: a sample holder configured to hold and position a sample containing the sample; an illumination system providing light to the sample; a light detector positioned with respect to the sample holder to detect scattered light in response to light provided by the illumination system; and an analyzer communicating with the light detector to analyze the analytes in the sample based on the detected light of the at least first and second colors.

The assay system of the present invention further comprises at least one processor and a memory, wherein the processor is configured to: accept spectral intensity data from a sample, wherein the spectral intensity data comprises signals from at least two types of light scattering particles, and wherein a first particle binds to a first analyte and a second particle binds to a second analyte; convert the spectral intensity data, using multi-spectral deconvolution, into a first intensity that corresponds to an abundance of the first particle, and a second intensity that corresponds to an abundance of the second particle; and quantify the first analyte from the first intensity and quantify the second analyte from the second intensity.

The present invention further involves An apparatus for quantifying at least two types of analytes in an assay, the apparatus comprising at least one processor and a memory, wherein the processor is configured to: accept spectral intensity data from a sample, wherein the spectral intensity data comprises signals from at least two types of light scattering particle, and wherein a first particle binds to a first analyte and a second particle binds to a second analyte; convert the spectral intensity data, using multi-spectral deconvolution, into a first intensity that corresponds to an abundance of the first label, and a second intensity that corresponds to an abundance of the second label; and quantify the first analyte from the first intensity and a concentration of the second analyte from the second intensity.

The present invention further comprises an analyzer for quantifying at least two types of analytes in multiplexed assays, comprising at least one processor and a memory, wherein the processor is configured to: accept spectral image data from a sample that includes two or more spectrally selective images, wherein the spectral image data is comprised of signals from two labels and wherein a first label binds to a first analyte and a second label binds to a second analyte; convert the two or more spectrally selective images into individual images that either contain only the first label or contain only the second label, using multispectral deconvolution; and quantify the first analyte by means of particle counting from the individual images that contain only the first label, and the second analyte by means of particle counting from the individual images that contain only the second label.

The present invention also involves a method for normalizing assay data, comprising: selecting a first population of assay data and a second population of assay data, wherein the first population comprises a dependent set of controls and the second population comprises an independent set of controls; obtaining a linear relationship between the independent set of controls and the dependent set of controls; and applying the linear relationship to the first population, thereby producing normalized assay data.

A method of ratiometric analysis, performed on assay data that comprises an array of features, the method comprising: selecting a first population of assay data and a second population of assay data, wherein the first population comprises a dependent set of controls and the second population comprises an independent set of controls; obtaining a linear relationship between the independent set of controls and the dependent set of controls; and applying the linear relationship to the first population of assay data, thereby producing a first normalized assay data; obtaining a second linear relationship between the second set of controls and the first set of controls, wherein the first set of controls is treated as an independent variable, and the second set of controls is treated as a dependent variable in the linear relationship. applying the second linear relationship to the second population of assay data, thereby producing a second normalized assay data; calculating a ratio of a value of the feature in the normalized assay data to a value of the feature in the second normalized assay data; and identifying the feature as regulated if the ratio exceeds a threshold value.

The present invention also involves a method of identifying at least one anomalous feature in assay data, wherein the assay data comprises an array of features, the method comprising: dividing the assay data into a first population and a second population, wherein the first population comprises a first set of controls and the second population comprises a second set of controls; obtaining a first linear relationship between the first set of controls and the second set of controls, including setting one set of controls as a dependent variable and the other set of controls as an independent variable; obtaining a second linear relationship between the second set of controls and the first set of controls, wherein the control set that is treated as the dependent variable in obtaining the first linear relationship is treated as an independent variable in obtaining the second linear relationship; applying the first linear relationship to the first population, thereby producing a first normalized assay data; applying the second linear relationship to the second population, thereby producing a second normalized assay data; and calculating a first ratio of a value of the feature in the first normalized assay data to a value of the feature in the second normalized assay data; and calculating a second ratio of a value of the feature in the second normalized assay data to a value of the feature in the first normalized assay data; and multiplying the first ratio by a reciprocal of the second ratio to produce a product; identifying the feature as anomalous if the product exceeds a threshold value.

The present invention provides an ultra-sensitive signal generation and detection system for multiplexed assays of analytes. The system enables simple and efficient detection using Resonance Light Scattering (RLS) particles and provides a signal generation and detection apparatus with companion software to facilitate the measurement and analysis of chemical, biological and biochemical interactions on a variety of substrates, including glass and membrane microarrays and microwell plates. Certain embodiments are designed for use with arrays, and are particularly advantageous for microarrays, in view of the high feature density and large amounts of data that can be potentially generated from even a single microarray. The present invention also provides methods for performing assays by detecting assay signals, particularly signals from microarrays, and for transforming the data using a data normalization method of the present invention. In preferred embodiments, the signals are any of the various types of assay signals, including, for example, scattered light signals, fluorescent signals, chemiluminescent signals, and radiolabel signals.

A system, according to the present invention, can be configured to carry out one or more of the following: scan a sample of a given format and detect the scattered light intensity at one or more locations within the sample; use edge detection methods to identify particles within a sample by segmenting an image into a number of regions; reconstruct a two-dimensional image of one or more regions of the sample from the detected light scattering signals at each of the detection points measured; collect images of one or more regions of the sample; detect the particles with an imaging photodetector and provide digital images of one or more regions of the sample by image processing methods; use image analysis methods to detect and measure one or more properties of the particles in the sample; identify and classify imaged particles based on one or more of the detected and measured properties, including grayscale or color based intensity values that are related to size, shape, or some combination of these properties; determine the amount of light scattering particles in a sample by counting the identified particles, summing the individual pixel intensity values in all areas of the image identified as particles, or by obtaining the sum of the mean or average intensity value for each image area identified as a particle, or some combination thereof.

In particular, use of metal particles, particularly particles made of silver or gold, permits high levels of sensitivity, thereby permitting detection of very low quantities of particles per unit area. With the use of certain combinations of particles and methods of illumination and detection, it is possible to detect a wide range of particle densities from about 0.001 to about $10^3$ particles per square micron ($\mu^2$) in a sample as a function of a particle size. By using appropriate type(s) of particles, different types of analytes in the same sample can be detected to very low levels and across very wide concentration ranges, as occur for example, in microarrays.

Use of RLS particles also permits the quantitative detection of single or multiple analytes in a sample and has found particularly forceful application to microarray based assay systems. The high sensitivity and ease of use of the signal generation and detection system ofRLS analyte detection means that one skilled in the art can by inexpensive means, detect and measure one or more analytes in a sample to extremely low concentrations without need of signal or target analyte molecule amplification methods.

Furthermore, if a sample is to be analyzed for two or more different analytes, different types of analytes exist at different concentrations in the samples. In particular, when using solid-phase related means such as array chips, some analytes may be at higher or lower concentrations from two to a few orders of magnitude more or less than other analytes in the sample. The methods of the present invention can be used to detect different analytes that are present at different concentrations in a sample by using different particle labels, each with a different inherent sensitivity based upon its light scattering power. For example, one could use a particle of greater light scattering power to detect an analyte present at a lower concentration and *vice versa.* This approach permits performing assays for analytes across a broad concentration range within the same signal generation and detection system.

The appropriate measurements can be accomplished on a single apparatus by utilizing both particle counting (at low particle densities) and integrated light intensity measurements (at high densities) on the same sample. Even wider detection ranges and greater sensitivities are possible if more powerful light sources such as lasers are used, and if more sophisticated detection methods such as confocal imaging are added to basic illumination and detection methods.

A preferred embodiment of the system of the present invention includes a signal generation and detection apparatus, or scanner, a control and analysis system, and companion software for controlling the scanner and for capturing, processing and analyzing RLS images and other data. The scanner preferably includes an illumination system having a unique shutter and aperture assembly for delivering precise patterns of light to a sample, a computer controlled X-Y stage, and a detection system comprising a CCD camera. The control and analysis system preferably comprises software instructions and algorithms for generating, capturing, processing and analyzing RLS images. For example, the control system preferably incorporates a software toolkit comprising instructions and algorithms for analyzing multiplexed assays of two or more colors, e.g., to allow separation and analysis of detected light that has scattered from 80 nm gold and 60 nm silver RLS labels. The multiplexing analysis software is preferably incorporated within the system of the present invention, and the multiplexing analysis is preferably performed in real-time during a scanning or assay procedure.

The interface between biochemical and signal processing is bridged by application of a light scattering detection and analysis instrument, as described herein.

In addition to system and apparatus, and methods for using the same, the present invention provides data normalization methods, particularly applicable to microarrays. The present invention also provides analyzers, computers, and data storage media with instructions implementing those data normalization methods embedded therein. Thus, the invention provides an analyzer for obtaining and processing signal data from microarrays. The analyzer includes a signal detector that detects signals from discrete areas of microarrays. The microarray data is derived from the signals detected by the signal detector. In preferred embodiments, the detector is configured for detecting and measuring scattered light from RLS particles. The analyzer can be configured to analyze microarray data derived from a number of alternative detection methods and using different types of labels, including but not limited to: scattered light signals from RLS particles, fluorescent signals, radiolabel signals, or chemiluminescent signals. The analyzer also comprises a computer having embedded therein a set of instructions to normalize microarray data to a regression line with a predetermined slope and $y$-intercept. The signals may be processed prior to such normalization. Preferably, the signal data is recorded on a computer readable data storage medium, and then the recorded data is operated on by the set of instructions that performs the normalization. The present normalization method transforms the data to fit a predetermined linear regression line with a particular slope and $y$-intercept. In preferred embodiments, the regression line has a slope equal to one, and a $y$-intercept equal to 0.

The results of the normalization method of the present invention may be used to enable feature data cleansing or identification. The end-effect is a net improvement for studying regulation profiles that are used to determine the degree to which a particular sample of genetic material is expressed relative to another under specified experimental conditions.

Accordingly, the invention provides a computer readable medium having recorded therein a set of instructions for performing a linear normalization method that transforms signal data from one or more microarrays to a predetermined linear regression line with a specific slope and $y$-intercept, preferably a slope equal to one and a $y$-intercept equal to 0. In preferred embodiments, the computer-readable medium is random access memory, read only memory, magnetic disk, magnetic tape, or optical disk. In preferred embodiments, the device is part of, or is connected with, a computer suitable to carry out the set of instructions on microarray assay data, e.g., scattered light, fluorescent, chemiluminescent, or radiolabel assay data.

In yet another aspect, the invention also provides a method for performing a microarray analyte assay utilizing the present normalization methods. The method involves detecting signals from a plurality of sites on one or more microarrays; transforming microarray data representing the signals with a normalization procedure that fits the data to a straight line of predetermined slope and $y$-intercept, thereby providing transformed microarray data; and recording the transformed microarray data in a data storage medium. Preferably the straight line has a slope equal to one and a $y$-intercept equal to zero.

In preferred embodiments, the method also involves displaying the transformed microarray data in a visual format. The display may, but need not, be a direct display of the data. The display can, for example, be in the form of numerical assay results, graphs, slide images, and/or biological or biochemical correlations or assay interpretations. The display format may be any of a variety of displays, including, for example, print, computer monitor displays, projected images, photographs, slides, and other hard copy images.

In preferred embodiments, the method also involves correlating at least a part of the transformed data to a biological assay result. That is, the transformed signal data is used to provide assay results, such as gene expression level, analyte level, sample source identity, and the like.

Preferably a set of instructions described herein is embedded in computer readable data storage media, but may also be implemented in hardware, in whole or in part. Such computer readable data storage media can be any of various types of media, including without limitation, volatile or non-volatile random access memory (RAM), read only memory (ROM), magnetic recording devices, such as hard disks, floppy disks, and magnetic tape, and optical devices, including, for example, WORM disks, CD-ROMs, and the like.

BRIEF DESCRIPTION OF THE DRAWINGS

These and other features, aspects, and advantages of the present invention will become more readily apparent from the following detailed description, which should be read in conjunction with the accompanying drawings in which:

FIG. 1 is a schematic view of a signal generation and detection system for analyte assays according to the present invention.

FIG. 2 is a top view of an exemplary scanner apparatus of the signal generation and detection system of FIG. 1, with the cover removed.

FIG. 3 is a cross-sectional schematic view of the illumination system of the exemplary scanner apparatus of FIG. 2.

FIG. 4 depicts a schematic view of various software components according to the present invention (FIG. 4A). FIG. 4B is a flowchart showing process steps for capturing and analyzing microarray images using the system of the present invention.

FIG. 5A shows a computer system of the present invention for use in conjunction with a scanner apparatus, or as part of a signal generation and detection system. FIG. 5B shows a schematic representation of an alternative embodiment of the system of the present invention, including a scanner and an image capture workstation connected over a network to multiple image processing workstations.

FIG. 6 is a representation of a mixed 2-color slide image produced with the system of the present invention.

FIG. 7 is a representation of four images of the 2-color slide of FIG. 6, wherein each image was taken through a different filter.

FIG. 8 is a flowchart showing steps for selecting filters to use during the multispectral linear unmixing process of FIGs. 19 and 21.

FIG. 9 is a graph showing energy spectra of gold and silver particles normalized to photon energy per mole according to step 654 of FIG. 8.

FIG. 10 shows graphs of the integral energy spectra of FIG. 9 versus wavelength for a number of different bandwidths.

FIG. 11 is a graph showing the effect of bandwidth on selectivity.

FIG. 12 is a graph showing the equal molar spectra of FIG. 10 normalized to 1nm bandwidth according to step 656 of FIG. 8.

FIG. 13 is a graph showing a difference spectra derived from the silver and gold curves of FIG. 12 according to step 658 of FIG. 8.

FIG. 14 is a graph showing the selection of filters in the regions of maxima and minima of FIG. 19 according to step 660 of FIG. 8.

FIG. 15 is a schematic representation of spots on a microarray calibration slide according to the present invention.

FIG. 16 is representation of images captured using selected filters according to step 662 of FIG. 8.

FIG. 17 shows a flowchart that describes the steps between multi-spectral images and relative abundance images.

FIG. 18 is a schematic diagram of the principle of multispectral imaging using multiple filters according to the present invention.

FIG. 19 is a schematic diagram showing the multispectral linear unmixing module according to the present invention.

FIG. 20 is a schematic drawing showing on-slide and off-slide methods for applying reference spectra to a mixture of particles.

FIG. 21 is a flowchart showing a process for unmixing a multi-color slide to produce individual intensity images for each color, or end-member.

FIG. 22 is a flow chart showing the integration of multispectral linear unmixing into the software process flow of the present invention.

FIG. 23 is a representation of individual intensity images obtained by unmixing a multicolored slide according to the process of FIG. 12.

FIG. 24 shows an $n$-particle system whereby each unmixed particle abundance value, on a pixel-by-pixel basis, is used in a ratio relative to a control abundance.

FIG. 25 shows a flow chart with steps for producing a linear transform normalization.

FIG. 26 shows the effect on data of applying the linear transform normalization method.

FIG. 27 shows a slide layout.

FIG. 28 is a schematic representation of normalization techniques utilized by the data analysis module of the system of the present invention.

FIG. 29 shows comparisons of normalization strategies for each Class, for the "Disordered" data set: FIG. 29A - slide 0; FIG. 29B - slide 1.

FIG. 30 shows data for different normalization strategies for the "Disordered" data set. FIG. 30A shows expression range for each class per normalization strategy. FIG. 30B shows Fold Change for each Class per Normalization Strategy.

FIG. 31 shows comparisons of normalization strategies for each Class, for the "Ordered" data set: FIG. 31A - slide 0; FIG. 31B - slide 1.

FIG. 32 shows data for different normalization strategies for the "Ordered" data set. FIG. 32A shows expression range for each class per normalization strategy. FIG. 32B shows Fold Change for each Class per Normalization Strategy.

FIG. 33 shows a comparison of no normalization to inter-slide normalization.

FIG. 34 shows a comparison of no normalization to inter-slide normalization.

FIG. 35 shows a comparison of linear normalization applied to reciprocal control sets: FIG. 35A shows the normalized relationships, and FIG. 35B shows the corresponding bar chart.

FIG. 36 is an image of features selected in an ArrayVision quantification analysis.

FIG. 37 shows plots, in panels A, B and C, of intensity against wavelength for samples containing varying levels of dust.

FIG. 38 shows Au concentration intensity spectra.

FIG. 39 shows before and after images (all screen stretch values: 100-500).

FIG. 40 shows corrected light scattering spectra of 60nm silver and 80 nm gold particles at $1.2 \times 10^{-12}$ M concentration in water.

FIG. 41 shows a plot of the extent to which the light scattering spectrum of 60nm silver particles overlaps the light scattering spectrum of gold, and vice versa.

FIG. 42 shows scattered light intensity from mixtures of 80nm gold and 60 nm silver particles.

FIG. 43 shows a graph of calculated percent silver or gold particles in suspension as determined by applying multiplexed vs. actual percent of silver particles in suspension.

FIG. 44 shows a layout of software in integrated Genicon separator.

FIG. 45 shows an integrated two color workflow.

FIG. 46 shows a layout for Genicon Separator Framework.

FIG. 47 shows a workflow for two-color image capture.

FIG. 48, comprising FIGS. 48A-C, shows components of Genicon Separator software.

FIG. 49 depicts a workflow for capturing a reference.

FIG. 50 depicts component software in an unmix tool.

Other features and advantages of the invention will be apparent from the following description of the preferred embodiments, and from the claims.

## DETAILED DESCRIPTION OF THE INVENTION

The structure and function of the preferred embodiments of the apparatus and methods of the present invention can best be understood by reference to the drawings. Where the same reference designations appear in multiple locations in the drawings, the numerals refer to the same or corresponding structure in those locations. While the invention will be described in conjunction with the preferred embodiments, it will be understood that they are not intended to limit the invention to those embodiments. On the contrary, the invention is intended to cover alternatives, modifications, and equivalents, which may be included within the invention as defined by the appended claims.

*Terminology*

By "light" is meant ultraviolet, visible, near infrared, infrared, and microwave frequencies of electromagnetic radiation.

By "metal-like" particles is meant any particle or particle-like substance that is composed of metal, metal compounds, metal oxides, inorganic semiconducting materials, non-organic superconducting materials, or a particle that is composed of a mixed composition containing at least 0.1 % by weight of metal, metal compound, metal oxide, semiconductor, or superconductor.

By "non-metal-like" particles is meant particles that are not composed of metal, metal compounds, metal oxides, semiconducting materials, or metal-containing superconducting materials, or mixed compositions that are not composed of at least 0.1 % by weight of metal, metal compound, metal oxide, metal-containing superconductor, or inorganic semiconductor material.

By "coated" particle is meant a particle that has on its surface a layer of additional material referred to as a coating. The coating chemically stabilizes the particle in different sample environments, and/or permits the particle to bind specific analytes according to well-understood principles of molecular recognition. The coating may also modify the optical properties of the particle. Such coatings may comprise, for example, inorganic and/or organic compounds, polymers, detergents, proteins, peptides, hormones, antibodies, nucleic acids, oligonucleotides, RNA, enzymes, fluorogenic compounds, carbohydrates, receptors, and the like.

By "analyte" is meant material evaluated in an assay. The analyte is typically just one of a complex mixture of materials but is a specific material of interest to a researcher, technician or other professional person. Such material is preferably an organic substance and is preferably detected indirectly through detection of a particle or label to which it binds. If the analyte is detected through its interaction with a particle, then the particle preferably has immobilized thereon a compound that is potentially capable of binding with the analyte. In some embodiments, an interaction between the analyte and another species is indirectly analyzed with a reporter species that specifically detects the interaction. For example, binding between an immobilized antigen and a first antibody (or visa versa) could be analyzed with a labeled second antibody specific for the antigen-first antibody complex. For applications involving hybridization between polynucleotides, the presence of hybrids could be detected by intercalating dyes, such as ethidium bromide, which have a preference for double-stranded polynucleotides. In such situations, it is the reporter species that is detected and correlated with presence of analyte.

An assay may be able to simultaneously detect the presence of more than one analyte of interest in a sample. An "assay" may also be able to identify multiple compounds which interact with an analyte of interest, such as, for example, to identify a peptide or other compound which binds an antibody, enzyme or other receptor of interest. When the immobilized compounds on particles are polynucleotides, the assays are particularly advantageous for use in hybridization-based applications such as sequencing. Furthermore, arrays suitable for use with the present invention are not limited to applications in which an immobilized compound and analyte bind another. The arrays can also be used to screen for and identify compounds which catalyze chemical reactions, such as antibodies capable of catalyzing certain chemistries, and to screen for and identify compounds which give rise to detectable biological signals, such as compounds which bind to a receptor of interest. The only requirement is that the interaction between the immobilized compound and an analyte give rise to a spatially-addressable detectable signal. Thus, the present invention is useful in any applications that take advantage of arrays or libraries of immobilized compounds, such as the myriad solid-phase combinatorial library assay methodologies described in the art.

In the context of analyte assay labels and the present methods, the term "light emitting particles" refers to particles that give off light under the conditions of the assay. It does not mean that the light needs to be generated by the particle. The light may, for example, appear to be emitted due to scattering of incident light by the particle, or by a fluorescent mechanism. Thus, emitted light may originate by a fluorescent or phosphorescent mechanism wherein incident light is absorbed by a molecule - often referred to as a label or tag - thereby exciting the molecule to an excited state such as an excited electronic, or vibrational state. The excited molecule emits light of a lower frequency than that absorbed, when it decays back to its ground state via one or more intermediate states. Alternatively, emitted light may arise from a chemiluminescent mechanism wherein, upon reaction, two or more chemical entities combine to produce a third chemical entity and to emit light. In another embodiment, light may be emitted via an electroluminescent mechanism, wherein electrons passing through a sample change from one energy level to a lower energy level, accompanied by emission of light. In yet another embodiment, emitted light may be scintillation light emitted from, for example, a substance when a radioactive emission from an isotopic label interacts with it. Radioactive decay may comprise any one or more of $\alpha$-particle (helium nucleus), $\beta$-particle (electron), or $\gamma$-ray. In a preferred embodiment of the present invention, emitted light is light scattered by a type of nanoparticles referred to herein as light scattering particles. In such an embodiment, incident light which may be polychromatic or monochromatic, is reflected by the particles in all directions and is detected. The mechanism that gives rise to the reflection is described elsewhere, see for example, U.S. patent 6,214,560, but involves an interaction between the E-field vector of the electromagnetic radiation and the electron clouds of the atoms in the particle. It is, of course, understood that other luminescent mechanisms not described herein by which a molecule, label, tag, or particle may emit detectable light are compatible with the methods of the present invention.

Likewise, the term "light scattering particles" refers to particles whose characteristics such as size, shape and composition are such that they scatter light of visible wavelengths sufficiently strongly to be useful as labels in analyte assays. For example, such particles include metal or metal-like materials as described herein. It is recognized that virtually all particles within a particular size range will scatter light to some extent.

As used herein, the term "array" refers to a plurality of sites in or on a single physical medium (also referred to as sample format or assay format), e.g., a slide, chip, or membrane. Preferably the sample format is a solid phase material with a substantially flat sample-bearing or sample-binding surface. Preferably, an array includes at least 50 separate sites. In alternate embodiments, an array can include 6, 8, 10, from about 20 to about 100, from about 100 to about 200, from about 200 to about 400, from about 400 to about 800, from about 800 to about 1,000, from about 1,000 to about 5,000, or from about 5,000 to about 10,000 separate sites wherein it is understood that the exact number of separate sites is not limited to the exact numbers presented herein, but will depend on peculiarities of array geometry and manufacture.

The term "assay format" refers to the physical medium or physical layout of the assay component in, or on which, label is detected and/or from which label is released for detection. The assay format may also be referred to as "sample format". Different assay formats include, for example, slide, chip, membrane, microtiter plate such as a 96-well plate, flow cell, cuvette, and gel (e.g., polyacrylamide or agarose gel) formats. The particles used in conjunction with the present invention are compatible with both solution and solid phase assays. In solution, the particles form a very fine suspension.

In connection with methods for extending dynamic range, the term "addressable sites" refers to physically distinguishable locations in a single assay. Typically, the locations correspond to different sampling and/or different analytes. Also, typically, the different addressable sites will have a physical separation between sites, *e.g.,* a portion of a plate or chip to which analyte does not bind. Non-limiting examples of such addressable sites include array (such as microarray) spots, microtiter wells, spots or bands in a gel, and spots on a membrane or filter, as well as distinguishable cellular components or types of cell in a tissue section.

The terms "spots" and "features" are also used to refer to sites. In connection with multiple site assay formats, the term "spot" will be used to refer to a site (*i.e.,* a spot) on an array. Often the term "feature" will be used in this manner, but can also be used to refer to other formats.

The term "image" may be used to refer to a measurement of light intensity taken over an entire microarray, a single spot, or a number of spots. An image comprises a number of pixels. A pixel is geographically restricted and is the smallest part of an image for which an independent intensity measurement may be obtained. Depending upon the magnification employed, the signal measured for a single pixel may comprise contributions from more than one RLS particle, or more than one pixel may contain a signal contribution from a single particle.

A "region of interest", as used herein, is typically a part of an array that comprises a number of spots. It may, in some circumstances, such as high levels of magnification, refer to a single spot.

As used herein, the terms "integrated intensity", "integrated light", "integrated signal" and like terms refer to the light collected from a region of interest for a period of time. Thus, the amount of light collected will depend on the collection period, the average light intensity over that period, and the collection efficiency of the collector or sensor.

The term "sample" generally refers to a biological sample in which the presence of some analyte is detected. Often a sample may comprise body fluids such as blood, serum and sputum, as well as cell extracts. It is to be understood that methods of the present invention are not limited to particular categories or classifications of sample, so that a sample may comprise biological fluids from animals other than humans, as well as plants, and other sources not originating in an organism, living or dead.

*Units*

Certain of the following abbreviations are used herein for units of measurement:

g - gram;
cm - centimeter (= $10^{-2}$ m);
mm - millimeter (= $10^{-3}$ m);
$\mu$ - micron or micrometer (= $10^{-6}$ m); $\mu$m is also used;
nm - nanometer (= $10^{-9}$ m);
$\mu$l - microliter (= $10^{-6}$ liter);
M - Molarity (moles per liter).

*Symbols and Notation*

Certain of the following abbreviations are used herein for physical quantities:

C - Molar Concentration (in M);
X - Optical Path Length (cm);
E or e - Molar Decadic Extinction Coefficient ($M^{-1}$ $cm^{-1}$);
$I_0$ - Incident Light Intensity (Photons per second);
$I_f$ - Fluorescence Intensity (Photons per second);
I - Photons per second which exit a solution after passing through a solution thickness X;
$I_s$ - Scattering Intensity (Photons/second);
$C_{sca}$ - Scattering Cross Section ($cm^2$);
a - Radius of a Particle;
N - Particle Concentration (particles/$cm^3$);
t - Turbidity of Suspension;
$\lambda_o$ - Incident Light Wavelength (nm);
$e_m$ - Dielectric constant of medium;

Before describing the particular apparatus and methods of the present invention, it is beneficial to describe some of the considerations, materials, and methods for RLS-based assays.

*Analyte Detection Apparatus*

The apparatus and methods of the present invention are advantageously applied to methods for the detection and measurement of one or more analytes in a sample, especially to analyte detection and/or quantitation methods based on the use of types of particles of specific composition, size, and shape (RLS particles), and the detection and/or measurement of one or more light scattering properties of the particles. Certain embodiments are designed for use with arrays, and are particularly advantageous for microarrays, in view of the high feature density and large amounts of data potentially generated from even a single microarray.

In typical assays, one or more types of metal-like particles are detected in a sample by measuring their color under white light or similar broad band illumination with illumination and detection methods as described herein or in U.S. provisional application serial nos. 60/317,543 and 60/376,049. For example, roughly spherical particles of gold (which may be coated with binding agent, bound to analyte, released into solution or bound to a solid-phase) of 40, 60, and 80 nm diameters, and a particle of silver of about 30 nm diameter, can easily be detected and quantified in a sample by identifying each particle type by measuring the unique color and/or the intensity of their respective scattered light. This can be carried out on a solid phase such as a microtiter well or microarray chip, or in solution. The measurement in solution can be somewhat more involved, because the particles are not spatially resolved as in the solid-phase format. For example, one can detect the different types of particles in solution by flowing the solution past a series of detectors each of which is set to measure a different wavelength or color region of the spectrum and its respective intensity. Alternatively, a series of different wavelengths of illumination and/or detection can be used with or without the flow system to detect the different particle types.

For solid-phase analytical applications, a very wide range of concentrations of metal-like particles is detectable by switching from particle counting to integrated light intensity measurements, depending on the concentration of particles as discussed hereinbelow. The particles can be detected from very low to very high particle densities per unit area.

In other assay applications, the particles which are bound to a solid substrate such as a bead, solid surface such as the bottom of a well, or other comparable apparatus, can be released into solution by adjusting the pH, ionic strength, or other liquid property. Higher refractive index liquids can be added to increase the light scattering signal and/or to reduce the intensity of background signal, and the particle light scattering properties are measured in solution. Similarly, particles in solution can be concentrated by various means into a small volume or area prior to measuring the light scattering properties. Again, higher refractive index liquids can be added prior to the measurement.

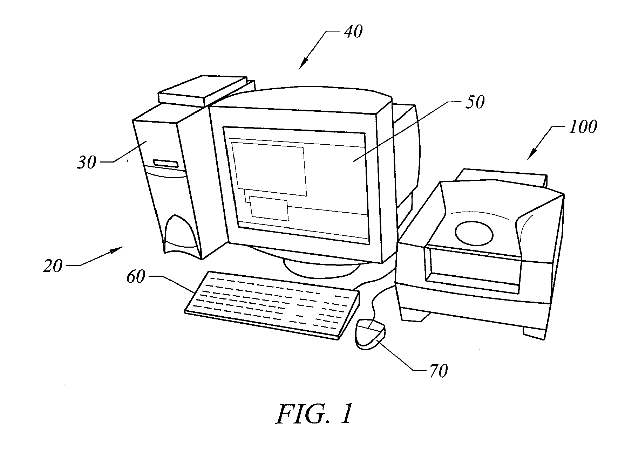

Preferably the methods of the present invention are used in conjunction with a RLS signal generation and detection system 10, as depicted in FIG. 1. Such a system generally comprises a control and analysis system 20, a signal generation and detection apparatus (also referred to as a "scanner") 100, and companion software for capturing, processing and analyzing RLS images and other data. Control and analysis system 20 generally includes at least one computing device 30 and a user interface 40, which in turn comprises a display 50 and a user interface comprising one or more user input devices such as a keyboard 60, mouse 70 and/or other devices for inputting information or commands. In an exemplary embodiment, computing device 30 is a computer system having a central processing unit (CPU), such as *e.g*., a 1 GHz or faster Intel "Pentium" processor, volatile memory, non-volatile memory, a removable media drive (such as a CD-RW drive), and an interface bus for communicating with the user interface and the scanning apparatus. One of ordinary skill in the art will appreciate that control and analysis system 20 may be any computer or other processor-based system with sufficient resources to store and implement software instructions and algorithms to control signal generation and detection apparatus 100 and to capture, process, and analyze RLS images.

Additional description and details of an exemplary system according to the present invention are included in the user manual for the Genicon Sciences Corporation GSD 501 system, entitled "GSD-501 System: Resonance Light Scattering Signal Generation and Detection, Version X2.0" and which is available at www.geniconsciences.com, the text and accompanying illustrations of which are incorporated herein by reference in their entirety.

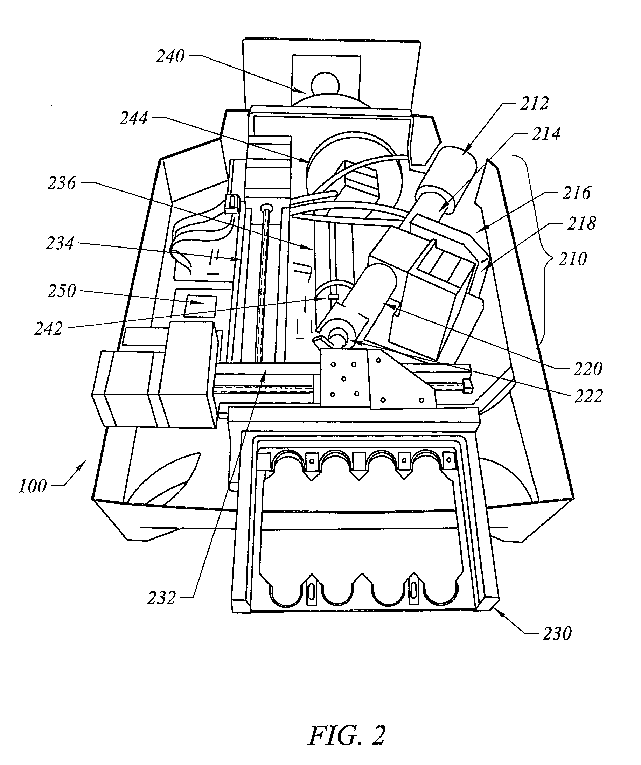

Referring to FIG. 2, a representative signal generation and detection apparatus 100 for use in conjunction with the present invention comprises an illumination system 210, a multiformat sample holder 230 (including an X-stage 232, and a Y-stage 234 together referred to as a XY-stage), a detection system 236, and control electronics 250. Apparatus 100 also preferably includes one or more communications ports, such as an RS232 port and a data port, for communication between control electronics 150 and control and analysis system 20. Scanner apparatus 100 is configured so that the sample device holder 230 holds and positions the sample device or substrate, in which the sample preferably contains one or more analytes and RLS particles; illumination system 210 provides light to a sample in the sample device, and the detection system 236 detects light scattered from any light scattering particles associated with the sample. Additionally the apparatus may use specially designed sample chambers and/or use antireflective coatings on optical components and sample chambers for optimizing any of the activities described herein.

Exemplary illumination system 210 includes a light source 212 shown as an illuminator bulb, a collimating, or focusing, lens 214, a filter wheel 216, a shutter/aperture assembly 218, an illumination imaging lens 220, and an illumination mirror 222. Illumination system 210 responds to commands from control and analysis system 20 and/or additional control electronics 250 to illuminate a discrete area of a slide or other sample substrate held in sample holder 230, such that RLS particles in the illuminated area may be detected by detection system 236. The fixed illumination and detection area set in an environment that is preferably dust-free and which is preferably set up to restrict the presence of stray light from sources external to the apparatus, The slides are moved over the fixed illumination and detection area by the high-precision XY stage comprising X-stage 232 and Y-stage 234, to capture individual images at precise locations on the sample. The captured images are combined, or stitched, for example in a mosaic fashion, to create an image of the entire slide by control and analysis system 20.

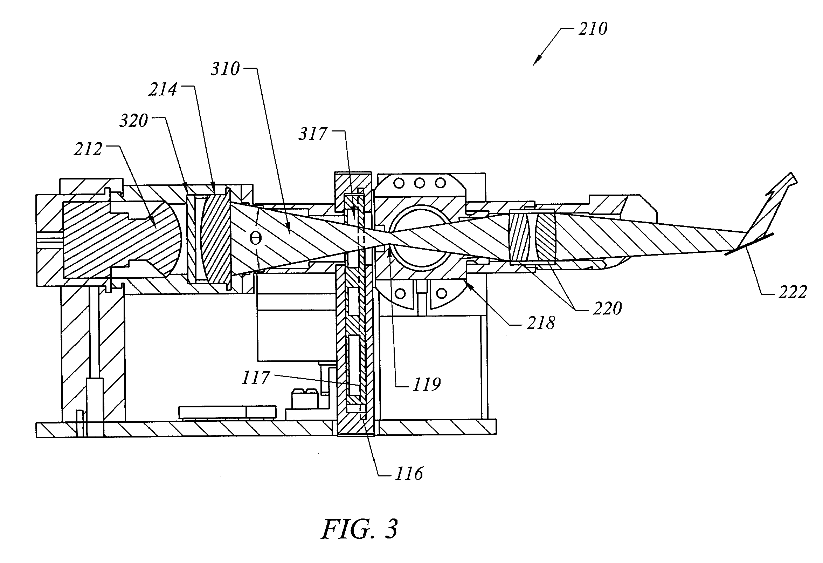

FIG. 3 illustrates the basic operation of illumination system 210 according to a preferred embodiment, the GSD-501 System available from Genicon Sciences Corporation, San Diego, which is suitable for use in conjunction with the present invention. White light from light source 212 passes through focusing lens 214 to form a cone 310 of light. Depending upon the particular application, cone 310 optionally passes through an infrared cutoff filter 320 and/or an optical band pass filter 217 before passing through an aperture in shutter/aperture assembly 218. Imaging lens set 220 then focuses the light onto illumination mirror 222 or other light-guiding system, which directs the focused light to illuminate the sample. Capture software described hereinbelow automatically controls illumination system 210 to precisely deliver a specific light pattern to a particular area of the sample for a desired exposure duration.

As disclosed in U.S. provisional patent application serial no. 60/376,049, filed April 24, 2002, a number of suitable light sources 212 are conveniently utilized in conjunction with the present invention, depending on the desired incident wavelength range. Examples include a 10W metal halide arc lamp, a laser, or a low power light source such as a filament bulb or a light emitting diode (LED). In particular, the light generated from light source 212 can be polychromatic or monochromatic, steady-state or pulsed, polarized on unpolarized, and can be coherent. When monochromatic laser light is used, it is preferably used in conjunction with a dark-field illumination system. In this case the light is preferably not evanescent light, as described by Stimpson et al., Proc. Natl. Acad. Sci. USA, 92:6379-6383, (1995). Furthermore, several colored LED's can be combined at different intensities to create a "tunable" light source to provide illumination of one or morespecific wavelengths. Alternatively, light may be delivered using a fiber-optic apparatus or a liquid light guide. In any relative orientation in a darkfield configuration, the sample can be illuminated by either "epi-" or "trans-" means. As is understood by one of ordinary skill in the art, "epi" illumination means that the incident light and detection system are both on the same side of the sample; in contrast, "trans" illumination means that the incident light and detection system are on opposite sides of sample. Trans and epi-illumination means are further described in U.S. patent 6,214, 560, and examples contained therein, incorporated herein by reference.

In various analyzers, the source of light in general need only be directed such that it allows scattered light from the particles to be detected. The illumination is introduced to the sample in a suitable manner such as at a controlled angle of incidence, to avoid incident light from entering into the detection optics, or from the sample surface, or from any other reflective surface after the sample. Spatial filtering using either kernel matrix filtering or confocal optics can be used to ensure reduction of non-specific light scatter, as would be understood by one of ordinary skill in the art. Such filtering can be supplemented by other instrumental components and sample chambers that reduce stray light.

The light is preferably directed at a sample which contains the particles, at an angle such that the light itself will not be observed by the detector unless it is scattered by the particles. Preferably, the method and apparatus differs from that of Swope, et al., U.S. Pat. No. 5,350,697, in that such scatter can be observed by eye within the critical angle, preferably within the angle of illumination. It can, however, also be detected at greater than the critical angle and outside of the intensity envelope of the forward direction of scattered light. When used with an imaging apparatus, e.g., a microscope, the present method preferably uses a detector perpendicular to the plane of the sample. However, various aspects of the present invention, including, for example, multi-format sample holders, annular light rings, tunable light sources, multi-magnification lenses, shaped apertures, immersion chambers, and analysis software, can be used in other apparatus designs, e.g., analyzers as described in Swope et al., U.S. Patent no. 5,350,697.

A description of suitable shutter/aperture assemblies 218 for the delivery of several precisely defined patterns of illumination to the sample surface while controlling the length of duration of exposure can be found in U.S. provisional patent application serial no. 60/376,049, filed April 24, 2002. Preferably, assembly 218 ensures that an even illumination of the sample at short exposures is attained.

In preferred embodiments, scattered light signals are detected from two or more different wavelengths, either from the same light source or from two or more different light sources that illuminate the sample. In preferred embodiments, the different wavelengths are peak wavelengths that correspond to a maximum in a band of wavelengths in a spectral profile. In general, the selection of wavelengths and bandwidths depends upon the relative light scattering spectral profiles of the particles in the system. The different wavelengths are preferably provided by a filtering technique, as described in U.S. provisional patent application serial no. 60/376,049, filed April 24, 2002. In one embodiment, the light is passed through illumination filter wheel 216 of FIG. 2. Alternatively, optical filtering can be performed on the detection side, for example by detection filter wheel 244 of FIG. 2. For imaging samples having two different types of RLS particles, for example, two or more filters may be used to determine the relative contributions of each particle type, as described hereinbelow.

Detection system 236 of FIG. 2, preferably includes a camera, detection optics, and optionally a detection filter wheel 244, and as described in U.S. provisional patent application serial no. 60/376,049, filed April 24, 2002, is preferably computer controlled. Additionally, the detection system can include a photodetector selected from: photodiode, photomultiplier tube, photodiode array, charge coupled device, complementary metal oxide semiconductor (CMOS), and a charge induction device. When image capture is accomplished through the use of a scientific grade CCD (charge-coupled device) camera 240 communicating with control electronics 250 and computing device 30, optical resolution is preferably at least 5 $\mu$m. CCD camera 240 can be any digital camera that utilizes a CCD for image capture. Accordingly, multiple tiles across the slide are imaged and then "stitched" together using image processing software on computing device 30. In one embodiment, in order to perform this stitching, the sample is moved in precise increments in X and Y, a movement which is preferably accomplished by a precision computer controlled XY stage with an accuracy and repeatability of less than 3 $\mu$m. Calibration of the movement of the XY stage, and the magnification of the optical system can be accomplished through the use of a specially designed photo lithographed calibration slide with features to aid in analyzing the distance between stitched panels and the orientation of the CCD to the stage. This slide is preferably used in conjunction with automated software, for example, embedded in computing device 30, to tune the stage positioning without requiring the intervention of an operator.

Detection optics 242 can include a fixed magnification lens, preferably a 2X or 4X lens, but any magnification between 0.5 to 500 times would be suitable, or a multi-position computer controlled zoom lens, allowing the operator to choose the magnification required for the experiment. The various merits of the respective lens systems are described in U.S. provisional patent application serial no. 60/376,049, filed April 24, 2002. Ideally, the fewer separate images are to be recorded, the faster is the overall time to scan a sample. An exemplary system is fitted with two fixed zoom settings at 2X and 4X, but an infinite level of zoom can be provided with computer control and encoder feedback. Ultimately, the magnification could be as great as 40 or 60X, allowing individual particle counting to be performed on individual spots, and thereby increasing the dynamic range of the system. Additionally, the apparatus may include at least a 10X objective; components for carrying out video contrast enhancement; fiber optic illumination and/or detection light path. The apparatus can be configured to use bright-field, laser confocal scanning, reflection contrast, or differential interference contrast microscopy detection.

The accuracy with which scattered light can be quantified, and the resolvability of discrete individual or populations of light scattering particles in a sample, by image analysis methods are dependent upon the spatial resolution, spectral resolution, segmentation and image processing algorithm criteria selected and the properties of the photodetector. and the properties of the photodetector. One such photodetector is a CCD camera made of individual pixels, each of which can sense the amount of light radiating from the sample at a given position. For example, in an image collected with a monochrome photodetector and an 8-bit digitizer, there are 256 gray levels that can be associated with the detected intensity signal at each pixel location of the photodetector. Using a single or multiple chip color photodetector, the red, green, and blue wavelengths (*i.e.*, the RGB components) of the detected radiated light can be measured. One of skill in the art can use higher spatial intensity CCD camera resolution including greater resolution optical digitizers to improve the resolution of the collected image. Preferably, most of the light scattering particles appear as bright point sources of light of a given color in the spectrum.

In a preferred embodiment, scanner 100 is also fitted with a second filter wheel 244 on the emission side between detection optics 242 and camera 240. This second filter wheel allows the system to measure fluorescence in addition to RLS particles for the purpose of establishing controls, or for other applications. Such a feature is useful if the sample contains fluorescent labels as well as or instead of RLS particles. The excitation and emission filter wheels can be independent units or can be combined into one larger wheel which has opposing emission and detection filters positioned in the path of illumination and detection.

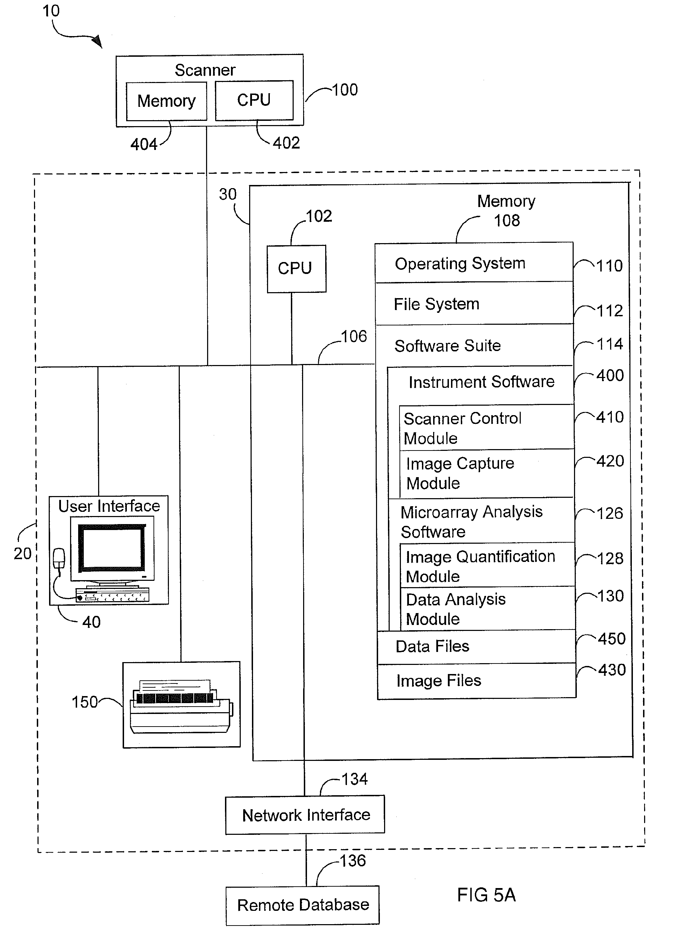

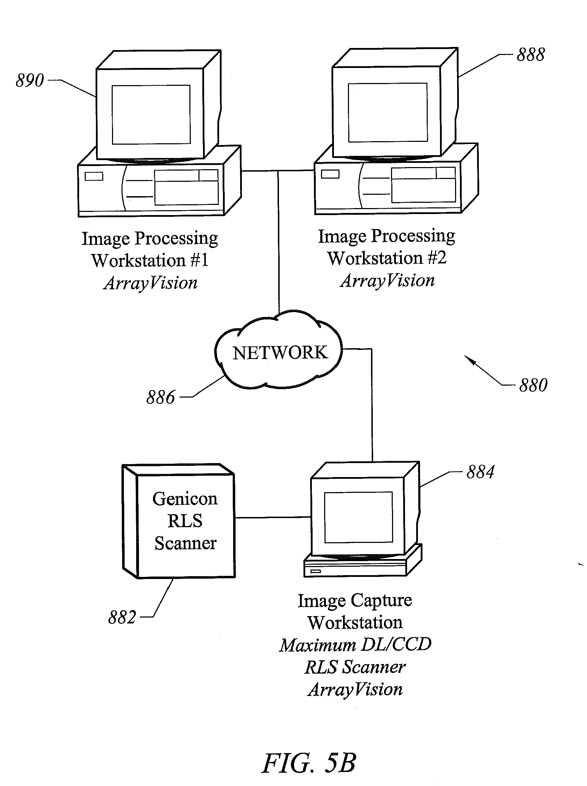

As described in U.S. provisional patent application serial no. 60/376,049, filed April 24, 2002 (see FIGS. 5A and 5B and accompanying text), sample holder 230 is preferably a multiformat sample holder that is capable holding different types of sample devices. The analyte may be present in solution or solid-phase. In a preferred embodiment, scanner apparatus 100 is particularly applicable to DNA and protein microarrays spotted on 1" X 3" glass or plastic microscope slides, patterned in 96-well microtiter plates, or spotted on immobilized membranes. Additionally, microtiter plates with microarrays spotted in each well are compatible with the present invention. Sample holder 230 is preferably fitted with a sample tray (not shown) that is designed as a removable insert, and which can accommodate different holders for each of the three substrates listed hereinabove. For example, a slide holder is designed to accommodate up to four 1"X3" slides at a time. A microtiter plate holder may accommodate one or more microtiter plates, such as 96 or 384-well clear bottom microtiter plates. In other embodiments, sample holder 230 accommodates a number of membrane carrier plates (not shown) providing an appropriate area to fit within the holder 230. Thus, for example, the membrane holder may be dimensioned to accommodate 1, 2, 4, 6, or 8 membrane carriers, as well as other numbers of carriers. One of ordinary skill in the art will appreciate that multiformat sample holder 230 may be configured to hold any other number, type, or size of samples or sample substrates. Alternative sample containers or substrates include test tubes, capillary tubes, flow cells, microchannel devices, cuvettes, dipsticks, or other containers for holding liquid or solid phase samples.

Those of ordinary skill in the art will recognize that the methods and apparatus described herein have broad utility. They can be applied in one form or another to most situations where it is desirable to use a signal generation and detection system as part of an assay system to quantify and/or detect the presence or absence of an analyte. Such analytes include industrial and pharmaceutical compounds of all types, proteins, peptides, hormones, nucleic acids, lipids, and carbohydrates, as well as biological cells and organisms of all kinds. One or another mode of practice of this invention can be adapted to most assay formats which are commonly used in diagnostic assays of all kinds. For example, these include heterogeneous and homogeneous assay formats which are of the sandwich type, aggregation type, indirect or direct, and the like. Sample types can be liquid-phase, solid-phase, or mixed phase.

The high sensitivity and ease of use of the signal generation and detection system of the present invention means that one skilled in the art can, by inexpensive means, detect and measure one or more analytes in a sample to extremely low concentrations without need of signal (label) or target analyte molecule amplification methods.

The high optical resolvability of two or more different particle types in the present invention means that very simple multi-analyte detection (*i.e.*, two or more different analytes) in a sample is possible without the need for complex apparatus).

*Instrument Calibration/Normalization*

The present invention uses a unique method to normalize images which facilitates the comparison of images between instruments, and which also compensates for drift in the illumination or detection optics. Such methods usually employ a photodetector placed in the illumination path. It is common for instruments of the type used in conjunction with the present invention to have sensors built into the illumination path which either control the output from the illumination source in the manner of a closed loop, by altering the voltage or current to the illuminator, or which measure light output and apply a factor to the data from a direct measurement of the output. The method of image normalization presented herein represents an advantage of the use RLS particles because such particles exhibit stability over time, in comparison to other types of labels such as chemiluminescent, isotopic, fluorogenic (*i.e.*, giving off fluorescent light), and fluorescent labels.

A preferred method that has been developed for a scanner used in conjunction with the present invention takes the entire optical path into consideration by making intensity measurements with the CCD camera itself. To obtain the measurements, a light scattering surface in the same image plane as a sample is moved into view by the XY-stage. The light scattering chip is mounted to the underside of the XY-stage at an extreme position of movement, out of the way of the sample holder. An image at a fixed exposure is taken, and the median pixel value is recorded. This median value is compared to a standard value taken from a calibration slide during the factory setup, and stored in non-volatile RAM ("NVRAM") in the instrument electronics. A new normalization factor is then calculated and stored in NVRAM. Each captured image tile is then multiplied by this factor before being stitched into the final composite image.

In an alternative embodiment, software would automatically modify the exposure time input by the user with the correction factor determined from the normalization test. This corrected exposure will generate an image with a normalized intensity that could be compared across instruments with different lamp intensities.

As an illustration, an operator would enter an exposure time of 1.0 second on two separate instruments, one with a relative lamp intensity of 70%, and the second with an intensity of 130%. Using the first approach, the images from the first instrument would be multiplied by a correction factor of 1.86 to be comparable to images from the second instrument which has the brighter illumination. In order to be able to multiply the image by the 1.86 correction factor and not "clip" the brightest pixels above the maximum value (64,536 counts for 16-bit images), the starting image must not have any intensities above 34,696 counts. Limiting the intensity on the first instrument because of the correction factor would lower the signal to noise performance of the first system, although the signal to noise on the second instrument would remain unchanged.

Using the second approach, the user inputs a 1.0-second exposure time into the software. The software would correct the exposure time for the first instrument by multiplying the exposure time by the 1.86 correction factor. The exposure time of the second instrument would not be modified in this approach. The resulting images would be roughly the same intensity for each instrument, and both would be using the full dynamic range available. More typically, a nominal value would be chosen around the mean of a number of instruments, and all the exposure times would be normalized to that value. The calibration slide used during factory setup as well as the light scattering chip and normalization program can further be used as a baseline value for the normalization, allowing comparisons of images between different instruments. The calibration routine is performed each time the instrument is switched on, and preferably about every 2 hours thereafter during uninterrupted operation.

*System Software*

Control and analysis system 20 of the present invention incorporates sets of software instructions and algorithms for controlling scanner apparatus 100, and for capturing, processing, and analyzing RLS images. As would be appreciated by one of skill in the art, such software, instructions and algorithms typically comprise one or more functions, subroutines, procedures and modules, and may be written in one or more of a number of computing languages, both compiled and uncompiled. It is within the scope of the software of the present invention that different functions, procedures, subroutines or modules may be written in different languages according to the developer's preference and convenience. Suitable programming languages include, but are not limited to: FORTRAN (including FORTRAN77 and FORTRAN90); BASIC; C; C++; VisualBasic; Perl; *Java;* COBOL; Python; Tcl/Tk; Ada; and scripting languages such as are available in a UNIX shell or similar operating system environment.

The methods of the present invention are preferably carried out on a computing device 30. In most cases, the sets of instructions are embedded in computer readable data storage media, but may also be implemented in hardware or firmware, in whole or in part.

Control and analysis system 20 comprises a computing device 30; a user interface 104 which may comprise a keyboard, mouse and/or touch-screen display; optionally, a network or other communication interface 134 for communicating with other computers as well as other devices; and one or more communication busses 106 for interconnecting the CPU(s) 102 to at least the memory 108, user interface 104, and network interface 134.

Computing device 30 may be a high performance machine such as a desktop workstation or a personal computer, or may be a portable computer such as a laptop or notebook or a personal digital assistant, or may also be a distributed computing array or a cluster of networked computers.

Computing device 30 comprises: one or more data processing units (CPU's) 102; and memory 108, which will typically include both high speed random access memory as well as non-volatile memory (such as one or more magnetic disk drives).

Control and analysis system 20 is preferably connected directly to scanner 100 so that RLS scattering images and data can be downloaded directly to memory 108. In less preferred embodiments, data from scanner 100 is transferred to computing device 30 via a portable storage medium such as a floppy disc, diskette, zip-disk, CD-ROM or flash-card. Scanner 100 is preferably configured for gathering micro-array data as used in gene expression analysis. Scanner 100 may be located on a bench-top or may be portable equipment for use in the field, or by mobile personnel in a laboratory environment.

Control and analysis system 20 may also access data stored in a remote database 136 via network interface 134. Remote database 134 may be distributed across one or more other computers, discs, file-systems or networks. System 20 is also optionally connected to an output device 150 such as a printer, or a device for writing to other media including, but not limited to, CD-R, CD-RW, compact flash-card, smart-media, memory-stick, floppy disk, "Zip"-disk, magnetic tape, or optical media.

The computer system's memory 108 stores instructions, procedures, and data, typically including: an operating system 110 for providing basic system services; a file system 112 for cataloging and organizing files and data; one or more application programs 114, such as user level tools for statistical analysis 118 and sorting 120. Statistical analysis tools 118 include, but are not limited to, tools for carrying out methods of normalization, regression, and sampling.

Operating system 110 may be any of the following: a UNIX-based system such as Ultrix, Irix, Solaris or Aix; a Linux system; a Windows-based system such as Windows 3.1, Windows NT, Windows 95, Windows 98, Windows ME, or Windows XP or any variant thereof; or a Macintosh operating system such as MacOS 8.x, MacOS 9.x or MacOS X; or a VMS-based system; or any comparable operating system.

Application programs 114 also preferably include a software suite 116 for analyzing and processing RLS data from scanner 100. Software suite 116 preferably comprises a number of modules, wherein a module is a function, subroutine, procedure, or a collection thereof that together, is able to carry out a task or set of tasks when in receipt of appropriate instructions. Software suite 116 preferably comprises software modules that are separately categorized as instrument software 118 and microarray analysis software 120.

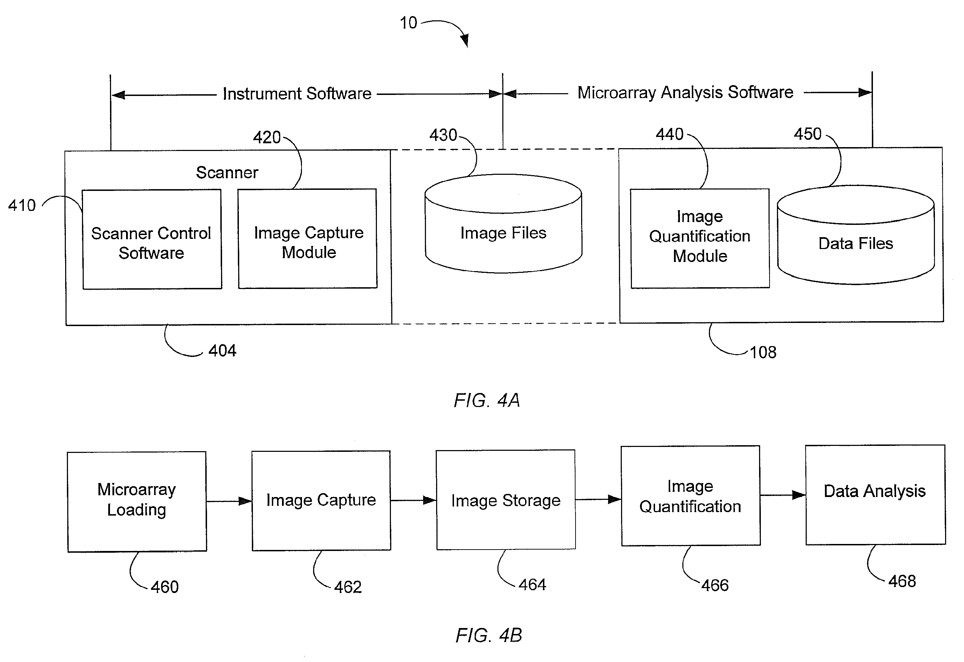

Referring to FIGs. 4A and 4B, and FIG. 5, the system 10 includes control and analysis system 20 and further includes a software suite 114, comprising instrument software 400 and microarray analysis software 126. Computing device 30 preferably comprises at least CPU 102 and memory 108. System 10 preferably includes a scanner control module 410 that controls the illumination of samples and the generation of raw images. Module 410 may be stored in memory 404 on Scanner 100, or in memory 108 on control and analysis system 20. Raw images are preferably passed to an image capture module 420, such as preferably Genicon Sciences Corporation ICS-501 software or alternatively MaxIm, (available from Diffraction Limited, Ottawa, Ontario, Canada) which captures, processes and stores the image files 430, either directly into memory 404 on scanner 100, or on memory 108 of computing device 30. Microarray analysis software, preferably stored in memory 108 on computing device 30 comprises an image quantification module 440, such as ArrayVision (available from Imaging Research, Inc., St. Catharine's, Ontario, Canada), to analyze the stored images, thereby producing one or more data files 450. Each of these various software modules is described in more detail hereinbelow.

The various steps of data collection and software analysis are described in FIG. 4B. First, samples, such as microarray slides, are loaded into scanner 100, step 460. During the operation of the scanner, images are captured, step 462. Images are processed and stored, step 464, prior to analysis and presentation of data to a user. Image quantification, step 466, includes one or more steps such as: standardization of images , for example, conversion to a standardized image format, e.g., TIFF, J-PEG, GIF, .bmp, or equivalent; carrying out one or both of particle-counting and integrated light intensity measurements; and correlating measurements of light intensity with analyte concentration. Data analysis, step 468, entails such processes as comparison of images with one another, employing a method such as a linear normalization method.

While the software is described herein as being stored in memory 108 on computing device 30, one skilled in the art will appreciate that any portion of the instructions and algorithms described herein may be stored and implemented in another computer or in memory 150 in scanner 100. In general, computer readable data storage media compatible with the present invention can be any of various types of media, including but not limited to: volatile or non-volatile random access memory (RAM); read only memory (ROM); magnetic recording devices such as hard disks, floppy disks, and magnetic tape; and optical devices, including, for example, WORM disks, CD-ROMs, and the like.

*Scanner Control Software*

In an exemplary instrument, the scanner control software 410 controls all the functions of the scanning apparatus including the stage, camera, filters, image correction and instrument setup and maintenance routines. However, it is apparent that one or more of these functions could be carried out manually, or with partial manual control and/or evaluation. Persons of ordinary skill in the art familiar with automated instrumentation are familiar with such software and associated routines, and can readily select or write suitable software or component routines to suit a particular instrument design.

The scanner control software 410 also preferably includes software routines and a graphical user interface for general scanner operation, including turning on the scanner, loading samples, setting-up a scan procedure, and other software routines (*e.g.*, for capture, processing and analysis of images). The scanner control software also preferably can operate in a number of modes, such as a "maintenance mode", a manual mode, and an automated or supervisory mode. Exemplary scanner control software is the ICS-501 software, available from Genicon Sciences Corporation, San Diego, CA.

In particular the scanner control software 410 preferably includes routines and algorithms for calibrating and/or correcting images, such as bias frame correction, flatfield correction, dark count compensation, and instrument normalization.

A bias frame correction routine is used to compensate for output variability in each pixel, thereby setting all pixels to a specified level. A need for this arises where the background is not uniform across all pixels. Several zero second exposures are taken, *i.e.*, by recording the signal without opening the shutter, and the average image stored in a secure location. This image represents the raw pixel values as read through the analog to digital electronics analysis sequence, and often has offsets of 600 counts or more, and pixel variability of 10-15 counts. The bias frame correction procedure uses the stored bias frame as a reference to correct for offsets and variability from pixel to pixel, and sets a zero length exposure to a known background value that can be offset from subsequent pixel measurements.

The present invention preferably uses a method known as flatfield correction to reduce variations in intensity across the image due to uneven lighting, or camera lens optics. Preferably pixel levels are recorded in the linear region of the response. According to a preferred embodiment, during flatfield correction, a slide is evenly coated with a film that scatters light, and is preferably imaged at an exposure level that results in pixel levels of about 1/3 the full dynamic range of the system. It is understood that other pixel levels are appropriate, such as about 2/3 or about ½ of the full dynamic range, as long as the levels are in the region of linear response.

The median pixel values over several images are extracted to reduce spurious noise. A master flatfield image is created from these median pixel values and stored for each filter and slide, microtiter and membrane modes. The collection of flatfield images is controlled by an automated routine available under the maintenance mode of the software. The stored master flatfield images are then used to correct each captured image tile using the following formula, centering the flatfield correction algorithm on the mean pixel value:

$$CI_{x,y} = \left( \frac{I_{x,y} - BL}{BI_{x,y} - BL} (M - BL) \right) + BL \qquad (E1)$$

wherein $I_{x,y}$ is a pixel value of the original image at location $(x,y)$; $BI_{x,y}$ is a pixel value of the background image at location $(x,y)$; $M$ *is* the average pixel value of the background image; $BL$ is the level of black; and $CI_{x,y}$ is the new pixel value in the corrected image.

Images are additionally subjected to dark count compensation. Dark count refers to a property of all CCD sensors whereby charge is generated in a pixel over time, even when the pixel is not exposed to light, in a manner which depends on, among other factors, the temperature. Dark count is usually expressed in electrons per unit of time at a given temperature. It can also be expressed as a current per sensor area at a given temperature. A scanner system according to the present invention preferably captures a "dark frame" at the beginning of each measurement and subtracts this value from each image to compensate for dark count.

*Image Capture Module*

Referring again to FIG. 5, the image capture module 420 includes instructions and algorithms for capturing RLS images detected by the scanner and for stitching, processing and storing the images. The basic principles and processes for capturing and processing the images have been described elsewhere, for example in Yguerabide et al., U.S. Patent 6,214,560, PCT/US/97/06584 (WO 97/40181), PCT/US98/23160 (WO 99/20789), and U.S. provisional patent application serial number 60/317,543.

Additionally, memory 108 optionally stores one or more data files 450 and one or more image files 430. In preferred embodiments of the present invention, data files 450 comprise normalized data for RLS scattering measurements on microarrays.

In certain embodiments, data files and image files may be archived, such as by storing on a separate computer, such as in a remote database 136. Alternatively, image files may be stored on one or more additional drives on a network.

*Image segmentation and processing*

The identification and classification of particles by image analysis methods is dependent on the ability to resolve the particles from the background and from other particle types by one or more properties of the detected particles. The background may consist of other light scattering particulate matter of varying size, intensity, and color, as recorded by the photodetector. For example the background can include grease and features from fingerprints, dust, bubbles, or irregularities on the surface of a microarray plate.

Whereas the eye and brain can easily detect and identify the various particles by gauging, relatively, one or more properties of the imaged light scattering particles, the accuracy and precision of such direct assessments by eye may vary according to the particular observer's capabilities. In order to detect, identify and quantify particles in a photodetected image, various image processing algorithms, preferably implemented in one or more, software components, are used to evaluate the image.

Among the image processing methods that can be used for the identification and spatial localization of light scattering particles, a form of edge detection is preferably used. Normally, an observer does not see an image as an array of discrete pixels; instead the observer perceives an image as groups of objects such as microarray features. Although such objects are made up of pixels, defining the boundary (edge) of a feature algorithmically is non-trivial when compared to what humans do naturally. Thus, a method that identifies the boundaries of regions of interest within the image is needed - even when the regions have different intensities of detected optical signal. The regions of interest are often referred to as features. In general terms, the goal is to segment an image of pixels into well-defined regions of interest, as defined by a boundary or edge, and ultimately assign to each region of interest a quantifiable result or integrated property. The edge of a spot is usually determined algorithmically from the context of the entire data set from which it is derived. All the art known methods of edge detection and image processing, (see, e.g., Djemel Ziou, Salvatore Tabbone, Edge Detection Techniques - An Overview, Technical Report, No. 195, Dept. Math & Informatique. Université de Sherbrooke, (1997), and references cited therein, incorporated by reference herein) can be adapted to the present methods and apparatus. It is within the capability of one of ordinary skill in the art to adapt such edge detection methods to the methods of the present invention.

Although edge detection methods are primarily used to identify array spots and for segmentation, edge detection can also be used for identification of individual particles. The method of edge detection groups together associated pixels of specified intensities and marks them in one form or another as an identified object, in this case a light scattering particle or multiparticle structure. The criteria for the discrimination of intensities can be varied depending on the nature of the image being analyzed. Different light scattering particle types can be identified and localized using many variations of the edge detection method for both monochrome and color images. The end result of performing an edge detection method is that specific areas of the image have been identified as light scattering particles.

For any given combination of light scattering particles, illumination source, photodetector, and optical background conditions of the sample, there is a preferred selection of the type or types of discrimination used in the edge detection routine and optimum minimum and/or maximum threshold value settings thereof. For example, in some applications a monochrome CCD photodetector is used to collect the image data. In one embodiment, it has been determined that by setting the gray scale minimum threshold value for the discrimination at 50 units( such as Counts, measured in photons/second), and the maximum gray scale threshold value at 220 units, the light scattering particles can be accurately identified in the sample, and quantified by a process known as particle counting. The threshold values and type of discrimination may vary from sample to sample.

In a manual mode of operation, the operator can directly visualize the sample and the collected image. The operator varies the method of discrimination and the limiting threshold values to determine which give the most accurate results with respect to his visual interpretation of the collected image, and with regard to identification and quantification of light scattering particles. For semi-automated or fully automated optimization of the types of discrimination and limiting threshold values used to analyze the image, several methods are possible. The values can either be preset for any sample type or calibration particles, or other materials to reflect background conditions can be used to calibrate the discrimination types and threshold settings prior to, during, or following photodetection of the sample. Based on the types of discrimination and settings of the threshold values, light scattering particles appear as groups of pixels clustered together and are identified in one form or another as a light scattering particle.

Once the light scattering particles have been identified, the particles can be further resolved, classified, and quantified by various means. The light scattering particles can be accurately classified based on many detectable properties. Any measurable property of the light scattering particle which can be used to identify different light scattering particle types and optimize the accurate identification of the particles in the sample can be used. These properties include: the sum of all pixel intensity values in the identified particle area (either gray scale, red, green, or blue wavelengths or some combination thereof); the mean value of the pixel intensities in the identified particle area (either gray scale, red, green, or blue wavelengths or some combination thereof); and the dimensions of the area of the particle identified; the shape of the particle identified. Additionally, a combination of two or more of these detectable properties can be used.

The quantification of each type of light scattering particle in the sample is important and can be accomplished by various means. For example, the number of identified particles in any given area of the sample can be obtained by counting, or by measuring integrated signal intensity from one or more particles. In another embodiment, for the particles identified as a given particle type, the sum or ratios of the aggregated values of any of the measurable light scattering particle properties described hereinabove can be used to quantify each particle type in the sample.

Thus, for each application where light scattering particles are detected, measured, and quantified by methods of image analysis, the particle detection and measurement algorithms are preferably optimized. One of skill in the art preferably determines which of the detected properties of the light scattering particles can best be used to resolve the particles in the sample. Particle identification is based on any of the measurable properties of the particles that provide the ability to specifically identify the particle. Minimum and maximum threshold values for the different particle type detection criteria are set in the algorithm to further resolve and properly identify and measure the particles. Minimum threshold values are used to set the cutoff for what is considered to be a specific light scattering particle, and maximum threshold values are used to provide a high end cutoff value to separate other particulate matter, such as dust or dirt, from being identified as specific light scattering particles.

*Specific Light Scattering Properties of Particles*

Some of the most important light scattering properties that can be used to detect analytes in types of various sample and using a variety of different assay formats, include one or more of the following: intensity, light scattering profile, peak wavelength, color, polarization, angular dependence of the scattered light, and the RIFSLIW (rotational individual fluctuations in the scattered light intensity and/or wavelengths).

Several parameters of RLS particles are relevant to the discussion presented hereinbelow.

The relative intensities of scattered light obtained from different particles irradiated with the same intensity and wavelengths of incident light can be directly compared by comparing their light scattering cross section, denoted by $C_{sca}$. This term is often also referred to as a particle's "scattering powers", or the scattered light intensity. The higher the $C_{sca}$ of a particle the greater is its scattering power. The scattering cross section of a particle ($C_{sca}$) at an incident wavelength is defined as an area surrounding the particle such that every photon which falls on that area is scattered. $C_{sca}$ is usually expressed in units of $cm^2$ or $\mu^2$ and depends on the composition, shape, size and uniformity of the particle, as well as the wavelength of incident light. The light scattering profile $C_{sca}$ versus wavelength can be calculated for any spherical particle of uniform composition using Rayleigh or Mie theory, see for example, J. Yguerabide and E. E. Yguerabide, "Light-Scattering Submicroscopic particles as Highly Fluorescent Analogs and Their Use as Traced Labels in Clinical and Biological Applications - I. Theory", Anal. Biochem., 262:137-156, (1998), which is incorporated herein by reference in its entirety.

In the quantum mechanical point of view, scattering is viewed as an absorption followed by an emission. Thus, there is a finite probability that a photon of light absorbed (wherein absorbed in this context includes both reversible and irreversible absorption) by a particle is re-emitted at the same wavelength as the absorbed photon. The photon can be re-emitted in directions different from the direction of the incident photon. That is, in the classical view, the incident photons are scattered by the particle.

The Absorption Cross Section, $C_{abs}$, of a particle is related to irreversible light absorption. Consider a particle that is illuminated by a monochromatic beam of light of wavelength $\lambda$. The absorption cross section $C_{abs}$ of the particle is defined to be an area (usually expressed in units of $cm^2$ or $\mu^2$) surrounding the particle, such that any photon falling on this area will be irreversibly absorbed by the particle. The value of $C_{abs}$ typically depends on the particle size, composition, shape and homogeneity. It also depends on the wavelength of light. A plot of $C_{abs}$ vs. wavelength gives the pure absorption profile of the particle. The $C_{abs}$ vs. wavelength profile for any spherical particle with a uniform composition can also be calculated with Rayleigh or Mie theory. The nature of $C_{abs}$ can be further understood by reference to the extinction cross section $C_{ext}$ discussed hereinbelow.

The relative absorption cross section $A_{csr}$ is defined as the ratio of the particle's absorption cross section to its physical cross sectional area, *i.e.*, $A_{csr} = C_{abs}/\tau a^2$, assuming a spherical particle of radius $\alpha$. $A_{csr}$ provides a measure of the particle's ability to irreversibly absorb photons falling on the area surrounding the particle. $A_{csr}$ can have values in the range of 0 to 6 depending on the composition, shape, and size of particle and the wavelength of light. A value greater than one means that a particle can reach beyond its physical dimensions to attract photons to it and absorb them.

The extinction cross section $C_{ext}$ of a light scattering particle is defined as the sum of its scattering cross section ($C_{sca}$) and its absorption cross section ($C_{abs}$), *i.e.*:

$$C_{ext} = C_{sca} + C_{abs} \qquad\qquad (L2)$$

$C_{ext}$ is usually expressed in units of $cm^2$ or $\mu^2$. The extinction cross section $C_{ext}$ of a particle can be readily measured at any given wavelength in a regular absorption spectrometer.

The intensity of light scattered by a particle in different directions and the state of polarization, *P*, of the scattered light depends on the wavelength and state of polarization of the incident light and on the size and shape of individual particles, and the homogeneity of the population of particles in the sample, as well as the type of particle. Furthermore, the profile of the scattered light, *i.e.*, the intensity of the scattered light as a function of wavelength (or frequency), typically comprises a band that stretches over a range of wavelengths. While it is preferable for this band to be as narrow as possible, it is understood herein that, when referring to the wavelength of scattered light, the wavelength corresponding to the peak of such a band is meant. In general, the form of the profile depends on the properties of the particle. Such properties and their influence on wavelength are further described in U.S. patent 6,214,560, incorporated herein by reference, but are summarized hereinbelow.

*Particle Type*

Coated and uncoated metal-like particles have similar light scattering properties and both have superior light scattering properties as compared to non-metal-like particles. In addition, it is relatively easy to adjust the types of light scattering properties in metal-like particles by varying in one form or another, the size, shape, composition, and homogeneity of the population of particles so that the specific light scattering attributes can be measured from the metal-like particle in different types of sample.

The metals silver (Ag) and gold (Au) are preferred for constructing the particles of the present invention. In addition, particles constructed from a core of one metal and surrounded by an outer layer of another metal, as further described in U.S. patent 6,214,560, are compatible with the methods of the present invention. Additionally, particles having a mixed composition, such that they are composed of a polymeric core, surrounded by an outer layer of a metal, may be used with the present invention.

At wavelengths of incident light in the visible region of the electromagnetic spectrum, the light scattering power (*i.e.*, $C_{sca}$) of metal-like particles is much greater than for a comparable non-metal-like particle such as polystyrene. Another important distinction between the light scattering properties of metal-like and non-metal-like particles is that for metal-like particles of the same composition but varying size in the preferred size range of 40-140 nm diameter, the profile of scattered light intensity versus incident light wavelength can be very different. This is in contrast to non-metal-like particles where for size ranges of about 10 nm diameter to hundred nm diameter, the profile is essentially the same. These differences are extremely useful to enable the detection of metal-like particles in various samples with greater specificity and sensitivity.

It has also been determined that certain mixed composition particles made from mixtures of non-metal-like and metal-like materials provide for additional light scattering properties and/or additional physical properties. These properties include the ability to manipulate the particles by applying an electromagnetic field. This property of the particles can be used in many different ways with the practice of one or more aspects of this invention.

For some particle compositions, and sizes the light scattering power decreases continuously across wavelengths from 300 to 700 nm, while for other compositions the scattering power vs. wavelength profile shows peaks or bands. When these peaks or bands are in the visible region of the electromagnetic spectrum the light scattered by the particles appears colored when the incident light is white.

*Particle Size*

Both theory and experiment indicate that for spherical particles of any composition up to about 120 nm in diameter and somewhat greater, a large proportion of the light scattered by the particle is radiated outside the envelope of the forward direction of the scattered light. As described in U.S. patent 6,214,560, detection and measurement of the scattered light at angles outside of the envelope of the forward direction of scattered light provides for a significant decrease in non-specific scattered light from the light beam and other light scattering constituents and objects being detected. This significantly increases the specific light scattering signal/non-specific light scattering ratio, which is a measure of signal-to-noise, for many samples.

Preferred sizes of particles used with the present invention range from about 20 to about 140 nm, where it is to be understood that variances in size due to the manufacturing process leads to particles a few nanometers outside those ranges but that remain eminently suitable for the methods and apparatus disclosed herein. It is to be further understood that when a preferred particle size is indicated herein, due to small imprecisions in particle manufacture among a population of such particles, a range of sizes centered on that size is contemplated. For example, a sample of 60 nm silver particles may comprise particles in the size range 59-61 nm, 58-62 nm, 56-64 nm, 55-65 nm, and less preferably a range as broad as 50 - 70 nm. Ideally, for reasons discussed hereinbelow, the range of particle sizes within a given population is as narrow as is possible.

Adjusting the size of certain types of spherical metal-like particles is a useful method to increase their detectability in various samples by using the color and/or other properties of their scattered light. By using a white light source, two or more different types of particles are easily detectable to very low concentrations. In contrast, for non-metal-like particles generally <100 nm, these particles do not possess these specific types of light scattering properties and thus it is more difficult to detect the non-metal-particles in most types of sample medium as compared to the metal-like particles.

For larger spherical particles in certain size ranges, for example from about 200 nm to about 1.2 microns in diameter, when illuminated with monochromatic incident light, the intensity of scattered light oscillates between relative values of 1 and 0 as an angle $\phi$ is changed from 90˚ to - 90˚ in a horizontal (*xz*) plane ($\theta = 0$) perpendicular to the direction of travel of the light beam. That is, if one views the scattered light for $\theta = 0$, the light intensity oscillates from bright to dark as the eye is moved from $\phi = 90˚$ to $\phi = -90˚$. This is further depicted in: J. Yguerabide and E. E. Yguerabide, "Light-Scattering Submicroscopic particles as Highly Fluorescent Analogs and Their Use as Traced Labels in Clinical and Biological Applications. II. Experimental Characterization", Anal. Biochem., 262:157-176, (1998), which is incorporated herein by reference in its entirety, as well as in U.S. patent 6,214,560. Correspondingly, for illumination with white light, the scattered light changes color as the eye is moved from $\phi = 90˚$ to $\phi = -90˚$. That is, the particles behave as diffraction gratings. Such light scattering properties are very useful for enhancing the specificity and sensitivity with which one or more analytes may be detected in many different types of samples.

The effect of increase in particle size is to increase the amplitude of the scattered light across a range of wavelengths. It is well known in the art of theoretical physics, specifically the theory of light scattering, that the scattering power of small particles increases with the sixth power of the radius up to approximately 80 nm for a gold particle. Surprisingly, this general relationship also applies to larger particles outside of the Rayleigh limit, that is, for particles with diameters larger than about 30 nm. One skilled in the art can calculate the relative scattering power of any small particle of diameter *d* from the value obtained for a 10 nm diameter particle of the same composition by multiplying the light scattering power of the 10 nm particle by $(d/10)^6$ where *d* is in nm. This approach can be used by one skilled in the art to determine the usefulness of certain particle sizes in various diagnostic assay applications where the intensity of the scattered light of a particle is used to detect the presence of an analyte. In particular, by adjusting particle size, relatively different, *i.e.*, not significant overlapping, regions of dynamic range can be achieved for different particles. For still larger particles, the light scattering power increases as $d^4$.

Preferred embodiments of the present invention use gold and silver particles. For gold particles, modest increases in size, say 10-20 nm, result in a large increase in the light scattering power of the particle. Furthermore, the incident wavelength for the maximum scattering power, $C_{sca}$, is increased significantly with particle size and the magnitude of scattered light intensity is significantly increased. For example, the incident wavelength for maximum $C_{sca}$ is around 535 nm, 575 nm and 635 nm for gold particles of diameters 40 nm, 100 nm, and 140 nm, respectively. When illuminated with white light, the 40nm gold particles strongly and preferentially scatter the wavelengths around 535 nm and the particles appear green, while the 100 nm particles appear orange-red and the 140 nm particles appear red in color. For sols of silver particles of diameters 38 nm, 47 nm, 60 nm, 75 nm, 90 nm, 118 nm, and 114 nm, the maxima of scattered light are at wavelengths: 465 nm, 472 nm, 474 nm, 572 nm, 575 nm, and 590 nm, respectively. The scattered light of such particles ranges in color from blue-violet, through green-blue to pale yellow, as wavelength increases.

Therefore, when illuminated with white light, certain metal-like particles of identical composition, but different size, can be distinguished from one another in the same sample by the color of the scattered light. The relative magnitude of the scattered light intensity can be measured and used together with the color or wavelength dependence of the scattered light to detect different particles in the same sample both specifically and sensitively, even in samples with high non-specific light backgrounds.

Smaller particles of spherical shape (in particular, those less than about 1/20 of the wavelength of the incident light in diameter) behave as isotropic dipole scatterers or emitters, that is, the light is highly polarized. For example, when such particles are illuminated with light polarized in the vertical direction, the light scattered in the horizontal direction $\phi = 0$, $\theta = 90°$ (see Figure 1 of J. Yguerabide and E. E. Yguerabide, Anal. Biochem., 262:157-176, (1998)) is one hundred percent linearly polarized (*i.e., P* = 1). This is very different to fluorescent molecules which usually behave as linear dipole emitters. In many different types of samples, the scattering property of particles allows for more specific and more sensitive detection of analytes by measuring the properties of scattered light as compared to what has been hitherto possible with fluorescent molecules.

For even larger particles (in particular, those greater than about 1/20 wavelength of light), in diameter there are certain ranges of particle sizes where the degree of polarization of the light decreases and becomes dependent on the wavelength as the size increases. As the particles become very large, the degree of polarization of the scattered light approaches 0 for the direction $\theta = 0$, $\phi = 90°$. There appear to be certain size ranges where the change in polarization changes the most, that is, the slope of degree of polarization versus size is at a maximum. Those regions where the slope is changing are preferably used in certain analytic applications, for example, agglutination or aggregation types of assays, to detect and measure one or more analytes in a sample.

*Particle Shape*

Particles used in conjunction with the present invention are preferably spherical in shape, where it is to be understood that imperfections of the manufacturing process can result in small deviations from perfect spherical symmetry.

Small nonspherical particles behave somewhat as linear dipole scatterers with the absorption and emission moments along the long axis of the particle. Applicant has observed that, under suitable illumination and detection conditions in an ordinary light microscope, when the illuminating light is linearly polarized, the non-spherical particles flicker as they rotate. The particles are most intense when their orientation is such that their long axis is oriented in the direction of polarization and is at a minimum when the moment is perpendicular to this direction. In contrast, small spherical particles do not flicker when illuminated by polarized light.

For nonspherical particles of certain compositions, the color of the scattered light (with illumination by white light) changes with the degree of asymmetry. As the asymmetry is increased, the color shifts towards longer wavelengths. For example, asymmetric particles of silver (Ag) were observed by applicant to change colors as the particles were rotating in solution when viewed with an ordinary light microscope under dark-field conditions. This property, which can be termed "RIFSLIW" (rotational individual fluctuations in the scattered light intensity and or wavelengths), can be used in various embodiments to more specifically and more sensitively detect and or measure one or more analytes or particles in a sample.

*Particle Homogeneity*

As indicated hereinabove, the intensity of scattered light can vary greatly as particle size is increased or decreased. It is preferable to use the narrowest distribution of particles possible, so that the accuracy of measurement is enhanced. Particles with narrow distributions of sizes are preferably obtained from Genicon Sciences, Corporation, San Diego, CA. Nevertheless, the variation in the intensity that is associated with variations in size must be taken into consideration when quantifying the scattered light, especially when integrated light intensity measurements are being performed. For example, using a 60 nm particle preparation with a 20% coefficient of variation in particle diameter, means that 10% of the particles have intensities about 3 times greater or less than the intensity from a 60 nm particle. In addition, the particles within the remaining 90% of the population still have varying intensities. Indeed, in applications where there are many particles being measured, the "average" integrated light intensity will probably approximate a 60 nm particle. However, at lower concentrations of particles, the statistics of such a variation may affect the accuracy of a reading from sample to sample, and correction algorithms may be needed.

*Particle Counting and Integrated Light Intensity Measurement*

Both particle counting and measurements of integrated light intensity (integrated intensity per unit surface area) have been used to detect one or more of the specific light scattering properties of the particles in a given area, with appropriate darkfield illumination and detection. It is generally useful to use the counting measurement method when the particle densities are about 0.1 particles per $\mu m^2$ or less. When the particle densities are greater than about 0.1 particles per $\mu m^2$, measuring the total integrated light intensity is a preferred method, though particle counting measurement may also be effective.

Integrated light intensity is measured by collecting the light scattered by the RLS particles in a microspot, for example, and focusing it on a non-imaging light sensing device such as a photomultiplier tube. The signal from the light sensing device, referred to as integrated light intensity, is a measure of scattered light from the whole spot. Integrated light intensity can also be measured by an imaging device such as a camera that incorporates a CCD. With such a camera, the scattered light from an RLS labeled microspot is focused by a lens onto the CCD. The camera records an image of the microspot. Integrated light intensity is evaluated by summing (integrating) the intensities of all of the camera pixels in the image.

Particle counting is based on the ability to detect individual particles using a dark field microscope, or similar arrangement. The number of RLS particles in a microspot can be evaluated by simply counting the particles (1) as seen through the microscope or (2) from an image recorded with a camera. Single particle detection is possible because the particles are very bright compared to the background.

The method of particle counting is usually more instrumentally demanding than the method of integrated light intensity measurement. However, for very sensitive detection of one or more of the light scattering properties of a particle, there are many advantages to using the counting technique. For example, fluctuations and inhomogeneities in the light source or sample chamber do not effect the particle counting measurement, whereas such variations can cause severe problems when integrated light intensity measurement is used.

The total quantity of particles in a sample may be determined by counting the number of particles per unit area and/or measuring the total integrated light intensity per unit area. In some aspects of the apparatus used in conjunction with the present invention, the scattered light from the particles is detected by a photodetector, for example, a photodiode or photodiode array, photomultiplier tube, camera, video camera or other CCD device, or the human eye. In other aspects, various types of light collection optical devices (such as a collection lens, imaging lens, mirror or similar device) can be used to collect the scattered light of the particles.

Experiments have shown that, using integrated light intensity, 80 nm gold particles can be detected on a solid transparent surface with a sensitivity of 0.005 particles/$\mu^2$ and an upper detection limit of about 10 to 20 particles/$\mu^2$. At a particle density of 0.005 particles/$\mu^2$, there are around 200 gold particles in a 200$\mu$ diameter spot. Thus, at the lower limit of integrated intensity detection, there are still many gold particles that are not being effectively detected and thereby useful information that is not being accounted for. By particle counting, however, it is possible, in principle, to detect a single particle in a microspot. Thus, the overall sensitivity for detection of 80 nm gold particles could be increased by employing particle counting in addition to integrated light intensity measurements. Experiments and theory have shown that particle counting has an upper limit of accurate detection of around 1 particle/$\mu^2$. This upper limit is determined by two principal factors: particle overlap and microscope resolution. If the resolution of the microscope is 1$\mu$, then two particles which are within 1 $\mu$ of each other appear as a single particle and would be counted as one particle. Thus, for particle densities greater than 1 particle/$\mu^2$, the number of particles counted by the particle counting method would be less than the actual number of particles in the microspot.

In a preferred embodiment, a method that achieves a smooth or seamless transition between particle counting and integrated light intensity measurements is employed. An exemplary method may be derived from observations of the factors that make particle counting more sensitive than integrated intensity measurements, even though they have a more limited detection range, as follows.

In particle counting, the spatial resolution capabilities of the eye or camera are used to separate the particles from background. Assuming that the image of a particle occupies one pixel on a CCD camera, it is easy to distinguish between signal and background pixels because a pixel that contains a particle image has a much higher intensity than one that does not (a background pixel). In fact, the particles are usually about 10 times more intense than a given background area that has the same size as the particle. Such a background area may be referred to as an object plane pixel because it represents signal from the same plane as the particles. Thus, when the eye or image analysis software detects the particles it effectively ignores object plane pixels that contain no particles. This is equivalent to throwing away background object pixels and retaining only those pixels which contain particles. By eliminating background pixels, and only counting those which contain signal, the number of particles in the microspot is simply the count of those which contain signal. The upper limit of detection for particle counting is around 1 particle/$\mu^2$. At higher particle densities, the count is no longer correct due to particle overlap and microscope resolution. By contrast, in integrated light intensity measurements, the intensities of all pixels in the sample microspot are summed and the integrated background intensity (obtained from a negative spot that contains no particles) is subtracted from the sample spot intensity to give the signal intensity. For low particle densities, such a calculation involves subtraction of two similarly sized numbers whose difference is small in comparison to their respective magnitudes. Noise in the background and sample spot intensities is responsible for limiting integrated light intensity measurements to a sensitivity of around 0.005 particles/$\mu^2$.

The particle overlap problems that arise in particle counting at higher particle densities can be eliminated by using a weighting measure, referred to herein as "intensity weighted counting", in such a manner that the use of particle counting and integrated light intensity can be streamlined at their respective detection limits.

Consider a microspot that has a particle density less than 0.005 particles/$\mu^2$, *i.e.,* is appropriate for particle counting. The count is not correct for particle densities greater than about 1 particle/$\mu^2$, because, for such high particle densities, several particles may occupy the same camera pixel but would be counted as 1. This problem can be eliminated by weighting each pixel by its intensity. Suppose that a pixel has three particle images. On the average, the intensity of this pixel is three times the value of a pixel with only one particle. From the pixel intensity it is therefore possible to tell how many particles are in a pixel. Alternatively, the intensity obtained by summing the intensities of all of the pixels in a microspot image, after subtracting background from each pixel, should be equal to the total number of particles in the spot multiplied by the average intensity per particle. This sum of pixel intensities is thus proportional to the total number of particles in a microspot and is a relative measure of particle count that is correct at all particle densities. Thus the intensity weighted count eliminates the problem of particle overlap. Moreover, the summed pixel intensity also gives the integrated light intensity which is beneficial at low particle densities because it avoids having to subtract two similar numbers from one another.

A protocol for applying such a procedure, as described hereinabove, in a preferred embodiment of the present invention is as follows.

1. Record an image of the RLS labeled microspot of interest.
2. Eliminate all pixels which have background signal only.
3. Sum the intensities of the remaining pixels.
4. The sum is proportional to particle number and gives a sensitivity that is similar to particle counting, and a detection range that is similar to integrated intensity, and permits a seamless transition of measures between low and high particle densities.

In step 2, hereinabove, in a situation where a single particle is measured by a group of pixels, a threshold for distinguishing background pixels from signal pixels can be obtained by examining a distribution plot of number of pixels vs. pixel intensity. A distribution plot is an intensity map across a group of pixels that correspond to a single particle and has an approximately bell-shaped curve. A threshold may then be determined by a user to be a number of counts (in, say, photons/sec) recorded by a single pixel, for example corresponding to an edge of the particle.

The assumption that each particle image occupies only one camera pixel is not always true in practice, however. The pixel size of a CCD in a typical digital camera is around 4 $\mu$ × 4 $\mu$. Gold and silver particles that are used in conjunction with a preferred embodiment of the present invention are submicroscopic, and the size of their image on the CCD is determined by light diffraction by the imaging lens or microscope objective. Experiments and calculations based on light diffraction theory and which have been presented elsewhere Optics, E. Hecht and A. Zayoc, Addison-Wesley Publishing Co., 1979), show that the particle image consists of a central spot and a halo. The central spot of the image has a diameter about 20 $\mu$ and occupies about 10 pixels. This size is not much affected by the magnifying power of the objective or imaging lens. The intensity is not uniform across the central spot but has an approximately Gaussian distribution. The fact that the particle occupies several pixels and the intensity is not uniform introduces the following problem. In the distribution plot of number of pixels against pixel intensity, the distribution of the pixels containing particles does not appear as a well defined band that is distinct from the background band, but instead appears as a very broad band that runs into the background distribution band. Nevertheless, it has been found that a threshold for discriminating between signal and background pixels can be established from this distribution, for example as discussed hereinabove.

As described in U.S. provisional application serial no. 60/317,543, it is important to adopt experimental conditions that lead to high levels of sensitivity and specificity in the detection of RLS particles and, in conjunction with those experimental conditions, it is preferable to employ analytical methods that ensure that those high levels of sensitivity and specificity are exploited. For example, as described in U.S. provisional application serial no. 60/317,543, appropriate experimental conditions that can be employed include: methods of refractive index enhancement for decreasing the non-specific light scattering and reflections that occur as a light beam passes from one medium to another; solid phase detection methods in conjunction with a photodetector; and detection of scattered light using a collection lens or mirror.

*Enhancements to Particle Detection*

In addition to experimental ways of improving analyte detection, there are many available software and hardware options that can be used to enhance the quality and signal to background ratios of the measured particles by both particle counting and integrated light intensity measurements. As described in U.S. provisional application serial no. 60/317,543, the signal to background ratio limits for the detection of the light scattering particles are influenced by a number of factors. In particular: by using larger diameter metal-like particles, the light scattering power of the particle can be significantly increased; by measuring the scattered light of the sample at angles outside the envelope of the forward direction of the scattered light, the signal to background ratio in either the intensity or the counting mode is substantially increased; the amount of non-specific reflected light can be substantially reduced by moving the incident light surface as far as possible away from the collection area that is being detected, thereby increasing the sensitivity of detection; and types of refractive index enhancement methods.

As also described in U.S. provisional application serial no. 60/317,543, in the performance of many assays it is highly advantageous to have a broad dynamic range over which the detection is substantially linear. That is, the signals from various samples can be detected and the presence of analyte quantified over at least several orders of magnitude of variation in analyte concentration or density. In the context of analyte assays, the term "dynamic range" refers to the range in density or concentration or absolute numbers of analyte entities that can be detected in an assay (based on the presence of specific label attached directly or indirectly to the analyte or a surrogate thereof, or released therefrom. Preferably the signal is proportional to the number of RLS particles present over a broad range which is preferably at least 4, 5, 6, 7, or even more orders of magnitude. That is, preferably, a quantifiable relationship is present between label concentration or density and signal intensity. Such a relationship is preferably linear. Broad dynamic ranges, preferably linear dynamic ranges, can be provided in various ways, for example, by various techniques in signal detection or signal processing, alone or in combination. It is highly preferable that the dynamic range is a range that can be quantified in a single performance of an assay, not requiring new sample or a dilution of concentration of an existing sample.

At a given illuminating wavelength, the useful range of scattered light intensity is usually about three orders of magnitude without the use of dynamic range extending techniques. However, this range can be extended by performing measurements at different illuminating wavelengths. In addition, at high surface densities, where the inter-particle distance becomes comparable to a wavelength of light, the light scattered by each particle is in phase with the light scattered by neighboring particles. This gives rise to specular reflection. At low particle densities the particles do not interact with each other and the particles scatter independently of each other. If the particle density is sufficiently high, a spot in a microarray displays a color in room lights and the spot looks like a diffuse dyed spot (diffuse reflection). However, at particle concentrations where the inter-particle distance is comparable to the wavelength of illuminating light, light impinging on the spot is reflected as from polished metal surface, *i.e.*, the spot behaves as a mirror.

Limitations to dynamic range generally fall into two categories. One category represents inherent assay format limitations, and includes limitations on analyte density, label density, and label interactions. Such limitations typically are significant at high analyte and/or high label densities. The second category is that of detector limitations. Such limitations include sensitivity limitations, including non-linear detector response at low signal (*e.g.*, low light) levels and problems associated with insufficient signal/noise ratio at low signal levels. The second category also includes effects occurring at high signal levels, such as overexposure limitations. An example of a sensor with limitations at both the low and high signal levels is photographic film. Similar limitations may also occur with electronic sensors, *e.g.*, CCD sensors.

Many types of light detectors collect light over an exposure time; therefore light from an assay is accumulated, rather than being recorded instantaneously. If the exposure time is too long, many of these detectors become saturated, with results such as changes in the response curve and "blooming" of the image. Sensors experience blooming when total light exposure results in excessive accumulation, with the result that both signal strength and localization discrimination are lost or at least impaired, for many such sensors. In an operational sense, blooming may also limit system performance for non-particulate or diffusing label signals. For example, blooming of signal from an intense spot to a weaker or negative spot can occur in an array system that utilizes fluorescent, chemiluminescent or isotopic detection. This effect is largely avoided when using light scattering particles because the signal is both highly intense and spatially confined. These properties provide advantages of using light scattering particles for arrays of high feature density wherein a large number of spots are distributed over a limited area.

In assays where there are multiple analyte detection sites, some sites may have strong signal and some sites may have weak signal. If a simple, single exposure time is used with a sensor that has a narrower useful response range than the difference between the weak signal and the strong signal, one or other or both of the signals will fall in a non-optimal response range. Such difficulties can be addressed in various ways, alone or in combination. Usually these techniques for providing extended dynamic range would be used in assay formats where there are a plurality of separately addressable sites at which the signal may vary. Examples of such assay formats include many solid phase and multi-well formats, such as microtiter plates, and arrays on microarrays or slides, chips, membranes, gels, and filters.

*Enhancing Dynamic Range*

As further described in U.S. provisional application serial no. 60/317,543 and in application serial no. 10/084,844, filed February 25, 2002, both of which are incorporated herein by reference, there are four basic techniques for extending dynamic range. One technique, which would typically be used to address limitations on the low signal end of the scale, is to combine integrated signal measurements for moderately strong signals, and particle counting for weak signals. Weak signals would be differentiated from strong signals through selection of an appropriate transition, or threshold. Such a transition would correspond to a particular particle density. One of ordinary skill in the art will recognize that such a transition density will depend on the detection apparatus used, the specific particles utilized, and the assay format.

An optimal or acceptable transition point for changing from integrated signal measurement to particle counting can, for example, be established based on empirical testing. Thus, for example, integrated signal measurement can be used with a selected exposure time. Preferably the exposure time is selected so that high label density sites provide an intensity that will not saturate the sensor, or otherwise push the total signal received into a range in which the sensor cannot provide readily quantifiable detection. Integrated signal measurement can then be used down to a particle density such that the sensor can also provide readily quantifiable detection of the label present at the lower density. However, the lower limit should also be at a density at which the number of particles in a site, or in at least a statistically acceptable portion of a site can be counted in a practical amount of time and with acceptable precision, *e.g.*, so that adjacent particles are not so close together that single particles cannot be reliably distinguished.

The selection of a transition point can also be made in automated fashion. Thus, for example, the exposure time can be set by computer camera controller to provide appropriate signal accumulation for quantitation. Those sites that do not provide a sufficiently strong signal at that exposure time can then be read by particle counting, *e.g.*, by increasing magnification. As indicated herein, particle counting can be performed in various ways, but is preferably performed electronically. In some cases, the lower limit of integrated signal measurement may overlap with the practical upper limit for particle counting. In such cases, the overlap region may be read by both methods, and serve as a calibration control.

Such a combination of detection modes can be implemented in many different ways. One preferred implementation for array embodiments would involve detection of the sample using spatial and/or time integration of signal. Array features (or at least portions of features) that are below a signal threshold (generally a pre-set threshold) are scanned and the particles counted. Preferably the scanning is computer controlled, and an image or images are created that are then processed with image processing software to identify and count the particles. The signals for array features above the threshold are used directly for integrated signal quantitation, and the particle counted array features are scaled to provide quantitation for an equivalent array feature area.

Such a dual mode approach can usually extend the dynamic range for RLS particles 1-2 orders of magnitude toward the lower end of the density scale, *e.g.*, for 80 nm gold particles from about 0.003 particles/$\mu$m$^2$ down to about 0.00003 particles/$\mu$m$^2$. Two orders of magnitude are achievable when the background and/or electronic noise is low. Under ideal conditions, particle counting extends down to 1 particle present in an array feature or per field of view. Under practical conditions with statistical requirements for reliable data analysis, particle counting can, for example, reliably count particle surface densities of $10^{-4}$, $10^{-5}$, or $10^{-6}$ particles per $\mu$m$^2$.

An exemplary system for carrying out such dual mode analysis utilizes a motor driven XY stage control and is described in further detail in U.S. provisional application serial no. 60/317,543. During a measurement, an initial image is captured with the zoom set for a wide field of view, preferably at the maximum field of view. The initial image is captured and processed with a grid overlay program to subtract background and extract the data from each spot. Spots that are determined to be at or below a pre-determined threshold are marked for analysis by particle counting technique. Integrated signal quantification data for spots above the threshold are used for the final report, and no particle counting of these spots is required. During the particle counting phase, the camera lens is zoomed in to a magnification allowing the visualization of particles within each spot determined from the initial image to be below the threshold. Several images are taken with small movements in XY around each of these spots. A computer program performing image analysis performs particle counting on each image, and averages the results to determine the particle density of each spot. Preferably, this image processing is performed in real-time using specially designed software and/or hardware.

Another technique for providing extended dynamic range is to use multiple exposure times. This technique involves image enhancement to provide increased dynamic range when imaging arrays or microarrays with large differences between intensities from spot to spot (feature to feature). For example, for a single assay unit with a plurality of separately addressable sites (*e.g.*, spots), at least two different exposure times are selected. It may also be beneficial to use more than two different exposure times, for example, 3, 4, 5, or even more. The number of different exposure times that are advantageous will depend, at least in part, on the dynamic range capabilities of the detector sensor and the signal range that may occur for the particular assay format and particle type selection.

In general, a short exposure time is utilized to ensure that the signal from very high density assay sites is within an acceptable quantitation range for the detector sensor, and preferably within a linear response range for the detector. With this short exposure time, sites with very low particle density will not produce sufficient signal for the detector to provide acceptable, or in some cases any, response. A long exposure time will be used that will allow the detector to accumulate sufficient signal to allow quantitation of the weak signal sites, and preferably to provide a response within the linear response region. As desired, intermediate exposure times can be utilized, *e.g.*, to maintain responses for intermediate density sites in an advantageous range for the detector.

In one embodiment, images from the camera are recorded with the exposure time set at two or more levels for both long and short time intervals. The sample is preferably kept static during the recording of all images. The data from each image can be scaled by a factor equal to the exposure difference, or the two images can be combined. Further aspects of this and other embodiments of multiple exposure time measurements are described in U.S. provisional application serial no. 60/317,543.

A third method of achieving enhanced dynamic range involves the use of non-destructive sensor reading. In a similar fashion to the use of multiple exposures, specific types of detectors can be used that are capable of providing a range of exposure times within a single actual exposure. Many types of detectors allow only a single reading, and then must be reset, *i.e.,* the process of reading the signal is destructive. However, certain detectors can be utilized that allow non-destructive reading, thus allowing multiple readings during a particular overall exposure. Further details of how to work with multiple readings are provided in U.S. provisional application serial no. 60/317,543.

A further method principally used in conjunction with integrated light intensity measurements utilizes signal or label density, to determine particle signal at high particle densities. The essence of this approach is to compensate for the deviations from linearity in the response at high particle label densities due to inherent characteristics of both the assay format and the particles. Preferably, RLS particles produce a scattered light signal that is linearly related to the number of particles present within the detection field over a broad range of particle densities, but this is usually only valid up to a density where particles begin to interfere with each other. This density will depend, at least in part, on the particle size, with larger particles interfering with each other at lower densities than smaller particles. Several different types of interferences or interactions can occur that contribute to non-linear response at high particle densities. The description presented hereinbelow is directed specifically to RLS particles, but similar effects also occur with particulate fluorescent labels, so it is to be appreciated that the present analysis can additionally be applied by one of ordinary skill in the art to the analysis of fluorescent-label based assays.

One type of interference occurs when particles become sufficiently close-packed that the surface of the aggregate of particles begins to act as a reflective surface, effectively as a mirror. This is particularly likely to occur with solid phase applications, such as arrays on chips or slides. When the particles on an array feature become close-packed, the particles effectively form a plating that acts as a reflective surface, rather than as a population of discrete scatterers.

A second type of interference is a blocking effect. Even with arrays on solid phase media, there is a certain depth to the bound particles. Thus, when the array feature is illuminated at an angle, there is some probability that a particle can be in the "shadow" of another particle. The probability of such blocking is greater the larger the particles and the more dense their packing. The light scattered from a particle can also be blocked, in part, by another particle or particles, and the magnitude or probability of this effect is also dependent on the density and size of the particles. In practice, for most assay systems this effect is generally observed under conditions wherein the detected scattered light signal has already saturated the CCD or other photodetector.

Without being limited by this explanation, whether the dominant interference phenomenon is "blocking" or "reflecting" may be different for "trans" versus "epi-" illumination. For trans-illumination, when the particle density increases to the point at which one particle can shade another from receiving the illumination from the source, the signal will actually decrease as particle density increases. In addition, particles located closer to the detection source are essentially blocking the scattered light from other particles located closer to the illumination source. In the case of epi-illumination, at sufficiently high particle densities, the particles coalesce to form a layer which acts as a reflector, similar to a layer of gold foil. So, with epi-illumination, the intensity of scattered light will remain constant after the density of particles increases to the point of complete coverage of the surface of the slide.

Yet another type of interaction is due to perturbations in light scattering properties of individual particles that occur as particles are brought close together. These perturbations can also contribute to deviations in response. As with blocking effects, in practice, for most assay systems this effect is generally observed under conditions wherein the detected scattered light signal has already saturated the photodetector.

In practice, integrated RLS signals for spotted microarrays remain linear up to about 1-10 particle per $\mu m^2$, depending on particle size, and other parameters of the detection system. Beyond about 1-10 particle per $\mu m^2$, wavelength shifts due to the close proximity of particles to one another, coupled with shadowing effects from overlapping particles, causes a reduction of signal at the peak detection wavelength of the particle. Between average densities of about 1.0 and about 10.0 particles per $\mu m^2$, corrected integrated intensities should be calculated by some method to ensure linearity. It is possible to produce corrected particle signals for a range of particle concentrations using standard curves, using analytical or semi-analytical equations, or a combination of the two.

The approach using standard curves can be carried out as follows. For densities between about 1.0 and about 10.0 particles per $\mu m^2$, corrected integrated intensities can be calculated by generating an equation from a standard curve. In establishing the standard curve, it is important for achieving good accuracy in experimental quantitations, that the density of particles in each spot be accurately determined. Such a determination may be achieved by, for example, making a preparation of spots that represent a particle dilution series down to low enough density that the particles can be counted. A standard curve is preferably obtained from data gathered for a standard dilution series of spots in the 1-10 particles per $\mu m^2$ range. The series can be obtained from measurements on a single slide or chip. Beyond about 10 particles per $\mu m^2$, the slope of the curve of scattered light intensity versus particle density approaches the horizontal, making such an approach less reliable. The standard curve can be used to determine an equation by curve fitting. A number of different curve fitting methods are suitable, as would be apparent to one of ordinary skill in the art. For example, linear regression, non-linear regression, and spline fitting, as well as others are convenient. See, for example, *Numerical Recipes,* Press *et al.,* Cambridge University Press, (1986). The equation generated from the curve can then be applied to any data collected to obtain the intensities in the non-linear, but correctable, range of signals. In applying this method, the camera exposure time is also factored into the equation to properly scale the data according to a linear relationship that is well known to one of ordinary skill in the art.

Equations for the intensities can also be obtained semi-analytically. Equations can specifically be obtained for quantifying analytes in solution and on solid surfaces. In doing so, it is beneficial to know two quantitative relations. First, the relation between light scattering intensity and particle (label) concentration or surface density, and second, the relation of light scattering intensity and analyte concentration, or in the case of RLS particles, surface density. The first relation is more fundamental because it sets the maximum ranges and sensitivities that can be achieved with a given RLS particle.

Mathematical expressions that can be used to describe these relations and which represent quantitative calibration data were obtained from the relation between light scattering intensity and particle or label, concentration in solution and on solid surfaces. The methods presented hereinbelow enable one of skill in the art to obtain experimental relations between scattered light intensity and particle density. These relations are fundamental. In practice, it is the relation between scattered light intensity and the amount of analyte detected in a given spot that is of interest.

One possessing these relations can correlate a measured signal intensity to an analyte concentration and therefore determine quantities of analyte present in a given sample. The amount of analyte can be expressed in terms of analyte surface density. If in an RLS labeling experiment, each analyte molecule on a spot binds a particle, then the particle density and analyte density are the same. The amount of analyte in the spot can be determined from the scattered light intensity and from the calibrating graph or calibrating expression relating scattered light intensity to particle density. However, it may be that not every analyte molecule on a surface binds a particle because of geometrical effects, affinity constants and other factors. In this case, scattered light intensity vs analyte concentration can be determined using a calibrating slide and by representing the results by a mathematical expression. This can, for example, be done by the methods described hereinbelow.

On the basis of conservation laws known to one of ordinary skill in the art, the intensity, $I_S$, of light scattered by particles in a volume, or on a surface S, is proportional to the incident light intensity $I_0$ (photons s$^{-1}$cm$^{-2}$), the fraction $\phi_A$ of incident photons which are absorbed by the particles and the fraction $\phi_E$ of absorbed photons which are re-emitted. Mathematically,

$$I_S = aI_0\phi_A\phi_E \qquad (C1)$$

where a is an instrumental constant. All of these parameters except *a depend* on the wavelength of the incident light. Equation (C1) assumes that the incident light is monochromatic and has a specified wavelength. The steps and approximations involved in deriving relationships between $I_S$ and particle density vary from a solution to a solid phase assay. In solution, particle density is effectively a concentration. In solid phase, particle density is effectively expressed as a number of particles per unit area.

When light scattering particles are used in solution, they form what may be called variously a colloid, a suspension or a sol. In order to obtain an explicit relation between $I_S$ and particle concentration in a suspension such as would be encountered in a solution-based assay, consider the particle concentration dependence of the various parameters in equation (C1). In general, $I_0$ is independent of particle concentration, and $\alpha$ and $\phi_E$ are independent of concentration at low particle concentrations. At low particle concentrations, only the fraction of absorbed incident photons, $\phi_A$, depends on concentration, and thus the dependence of $I_S$ on concentration is completely determined by $\phi_A$. At higher concentrations, $\phi_E$ becomes dependent on concentration due to particle-particle perturbations, and $\alpha$ also becomes dependent on concentration due to geometrical and inner filter effects discussed hereinbelow.

Assuming the validity of the Beer-Lambert law, a relation familiar to one of ordinary skill in the art, the dependence of $\phi_A$ on particle concentration is given by:

$$\phi_A = \left(1 - 10^{-\varepsilon Cx}\right) \qquad (C2)$$

where $\varepsilon$ (in M$^{-1}$ cm$^{-1}$) is the molar decadic extinction coefficient, *C* is the molar particle concentration, and *x* (in cm) is the optical path length. Thus, for particle concentrations where the distance between particles is large and there are no geometrical and inner filter effects, from equations (C1) and (C2):

$$I_S = a I_0 \left(1 - 10^{-\varepsilon Cx}\right)\phi_E \qquad\qquad (C3)$$

For low particle concentrations where $\varepsilon Cx$ is less than 1, the exponential term in equation (C3) can be expanded in a power series using a general relationship of the form (derived from the MacLaurin expansion for $e^{-z}$:

$$10^{-z} = 1 - 2.303z + \frac{(2.303z)^2}{2!} - \frac{(2.303z)^3}{3!} + \cdots \qquad\qquad (C4)$$

noting that ln(10)=2.303.

Retaining only the first two terms of equation (C4) (for $z \ll 1$), equation (C2) shows that

$$\phi_A = 2.303\varepsilon Cx \qquad\qquad (C5)$$

so that

$$I_S = 2.303 a I_0 \varepsilon Cx \phi_E \qquad\qquad (C6)$$

In practice, equations (C5) and (C6) are correct within about 5% for values of $\varepsilon Cx$ less than about 0.05.

The well known approximations presented hereinabove essentially apply to particle suspensions where the amount of light absorbed in the path-length x is very small. For this condition, the intensity of incident light is practically constant throughout the path x. However, if the suspension is itself strongly absorbing, the intensity of light is not the same at every point in the path but instead decreases from one end to the other. In the latter case it is preferable to use Equations (C2) and (C3).

From theory, $\phi_E$, the fraction of absorbed photons that are re-emitted, is expected to be particle independent when the average distance $D$ between the particles is greater than about 3 times their diameter $d$. For smaller distances, the particles interact such that their absorbance and light scattering spectra shift towards longer wavelengths, and the values of $\phi_A$ and $\phi_E$ change with increasing particle concentration. Thus, at high particle concentrations, $\phi_E$ and $\varepsilon$ are expected to become concentration dependent. Furthermore, if the particles have a tendency to associate, then, depending on the values of the association constants, $\phi_E$ can depend on particle concentration even at low particle concentrations.

If the particles are randomly distributed in suspension, then the average distance D between particles, in cm, at a concentration $c$ (in particles/cm$^3$), is given by:

$$D = \frac{0.554}{c^{1/3}} \qquad\qquad (C7)$$

That is, it is approximately equal to $(c)^{-1/3}$. From this equation, the concentration, $C$, at which $D = 3d$ can be calculated using equation (C8)

$$C = \frac{1000}{N_{av}}\left(\frac{0.554}{3d}\right)^3 \qquad\qquad (C8)$$

wherein d is in cm, and $N_{av}$ is Avogadro's number.

By way of illustration, for $d = 60$ nm, $C = 4.8 \times 10^{-8}$ M, which corresponds to an absorbance of around 1,200 for a 1 cm path length. Because of the very high extinction coefficients of a gold particle suspension used in connection with preferred embodiments of the present invention, concentrations as high as around $10^{-8}$ M are not normally used. Therefore, inter-particle perturbations are not usually important at the concentration ranges generally used. Typical particle concentrations in assays are between 0.1 and 10 optical density (OD) units. For 80 nm gold particles, a concentration of $10^{10}$ particles/OD/ml is preferred.

The scattered light intensity from a suspension of particles can also be affected by inner filter effects and the geometry of the light scattering volume, through effects contained in device parameter, $\alpha$. These effects depend on factors including the relative orientation of the illuminating beam, the direction of detection, and spatial filters that are used to reduce stray light. In preferred apparatus used for detection of particles in suspension in conjunction with the present invention, the detection system is at right angles to the direction of the illuminating beam. To minimize non-specific scattered light, the detection optics are preferably arranged such that only light scattered from a small volume in the center of the cuvet is detected. To reach this detected volume, the incident light must first cross through the intervening volume of suspension between the cuvet face on which the light is incident, and the center of the cuvet. The amount of incident light which is absorbed in the intervening volume increases with increase in particle concentration and the intervening volume thus acts as a concentration dependent inner optical filter. At very high particle concentrations, most of the light is absorbed in the intervening volume and a very small amount of light reaches the center of the cuvet. Similarly, light which is scattered towards the detector must pass through an intervening volume of suspension between the light scattering volume and the wall of the cuvet facing the detector. These effects also act as a concentration dependent inner optical filter. Because of these geometrical and inner filter effects, the scattered light intensity decreases with particle concentration at high particle concentrations. In practice, as further discussed hereinbelow, geometrical effects can be accounted for by multiplying equation (C1) by a factor which has the form $10^{-\alpha x}$.

For particles attached to a transparent surface (as in solid phase immuno- or DNA probe assays), equation (C1) also applies, and at low surface particle densities, the dependence of $I_S$ on particle density is also completely determined by $\phi_A$. Writing an equation equivalent to equation (C2) for particles on a surface depends upon how the particles are distributed on the surface. That is, the expression for $\phi_A$ depends on whether the particles are all confined to exactly the same plane or are distributed throughout a space of finite thickness (piled on top of each other). If the particles are all in the same plane, then the fraction of light that is absorbed is then simply the fraction of the total surface area occupied by the particle cross section multiplied by the particle density, as shown in equation (C9),

$$\phi_A = \rho\sigma_{ext} \qquad (C9)$$

where $\rho$ is particle density (particles/cm$^2$), and $\sigma_{ext}$ (cm$^2$) is the particle extinction cross section. The extinction cross-section represents a circular area around a particle such that every photon of light which falls on the area is absorbed and every photon outside the area is transmitted. Equation C9 can be written in terms of extinction coefficients using the relation

$$\sigma_{ext} = \frac{2.303 \times 1000\varepsilon}{N_{av}} \qquad (C10)$$

$$= 3.8 \times 10^{-21}\varepsilon. \qquad (C11)$$

The scattered light intensity from a surface in which all of the particles are in the same plane is, from equations (C1) and (C9), given by the expression

$$I_S = aI_0\rho\sigma_{ext}\phi_E \qquad (C12)$$

For solid surfaces in which the particles are not confined to a specific plane, but are distributed over a finite thickness, $\phi_A$ is given by the expression:

$$\phi_A = \left(1 - 10^{-\rho\sigma_{ext}}\right) \qquad (C13)$$

and the scattered light intensity is

$$I_S = aI_0\left(1 - 10^{-\rho\sigma_{ext}}\right)\phi_E \qquad (C14)$$

For values of $\rho\sigma_{ext} < 0.05$, equation (C14) reduces to the form of equation (C12) using a power series expansion, as in equation (C14).

Geometrical and inner filter effects are also important for light scattering from particles on a surface. The effects also depend on whether "trans" or "epi-" illumination is used. The effects are usually smaller for epi-illumination. Finally, interparticle perturbations can also affect $I_s$ when the average inter-particle distance of the particles on the surface is less than about 3$d$.

Having established the theoretical principles according to which measurements may be modelled in order to obtain particle densities, it is important to test the validity of the principles and to derive values of certain parameters that enable actual measurements to be quantified. As discussed in U.S. provisional application, serial no. 60/317,543, the applicability of the Beer-Lambert law to RLS particles can be tested by measuring the absorbance vs. particle concentration of RLS particles in suspension. In terms of absorbance $A$ (also called optical density) the Beer-Lambert law can be written as

$$A = \varepsilon Cx \qquad (C15)$$

where $\varepsilon$, $C$ and x are as defined in equation (C2). By plotting $A$ vs. $C$ for gold particles of different diameters, it was demonstrated that $A$ increases linearly with particle concentration as expected from equation (C15), and therefore that the Beer-Lambert law does apply to RLS particles and thus that the particles do not associate or perturb each other over the absorbance ranges typically encountered.

To determine the quantitative relation between scattered light intensity $I_s$ and particle concentration in suspension, measurements were taken, as described in U.S. provisional patent application, serial no. 60/317,543. If equation (C3) were to apply at all concentrations, the scattered light intensity would increase linearly with particle concentration at low particle concentrations, become nonlinear at higher concentrations and finally level off at a constant value. A graph of experimentally measured quantities displays these features, except that at high concentrations the intensity decreases with concentration instead of leveling off. This effect is attributed to inner filter and geometrical effects. Equation (C3) therefore does not give an accurate description of the experimental data at high concentrations because it ignores inner filter and geometrical effects. To take account of these effects, equation (C3) can be written in the form

$$I_S = B\left(1 - 10^{-\varepsilon Cx}\right)10^{-\varepsilon CL} \qquad (C16)$$

In equation (C16), $B = aI_0\phi_E$ and the exponential factor outside the parenthesis accounts for the inner filter effects while the term in parenthesis accounts for the amount of light absorbed by the detected light scattering volume. To determine whether equation (C16) gives a correct description of the graphs of scattered light intensity vs. particle concentration, the equation was curve-fitted by nonlinear regression analysis. The fitting parameters are $B$, $x$ and $L$, with $\varepsilon = 8 \times 10^9$ M$^{-1}$ cm$^{-1}$. Resulting values of such a fit are $B = 201.474$, $x = 0.008484$ cm and $L = 0.62754$ cm. It was seen that equation (C16) gives a very good fit to the data. At low particle concentrations, the exponential term outside the parenthesis is expected to be approximately equal to 1 and is therefore concentration independent, and the term in parenthesis reduces to $2.303\varepsilon Cx$, as suggested by equations (C4) and (C6). Thus for low concentrations, from equation (C16)

$$I_s = 2.303\, B\varepsilon Cx \qquad (C17)$$

The applicability of equation (C17) was tested at low particle concentrations and found to produce agreement within 10% for values of $C$ up to $5 \times 10^{-13}$ M. Although equation (C17) is generally applicable and can be used as a quantitative representation of experimental $I_S$ vs $C$ measurements and thus can be used to calibrate data, specific fitting parameters for different particle sizes can be found in U.S. provisional patent application, serial no. 60/317,543.

In a similar manner, experimental relations between scattered light intensity and particle surface density for solid assays were obtained, using spots of gold particles with different particle densities, as further described in U.S. provisional patent application, serial no. 60/317,543.

Plots of experimental scattered light intensity vs. particle density for 80 nm diameter gold particles on a solid phase surface show that the intensity increases linearly with particle density at low particle concentrations, becomes nonlinear at higher concentrations and decreases with increase in surface density at still higher concentrations. As with the measurements for solution-phase particles, the decrease at higher concentrations indicates that inner filter effects are important. By analogy with the results in the liquid (suspension) phase, equation (C 14) can also be used for the solid phase but preferably modified for inner filter effects, as follows:

$$I_S = B(1 - 10^{-\rho\sigma_{ext}})10^{-L\rho} \qquad (C18)$$

where B, $\sigma_{ext}$ and L are fitting parameters. Experimental data was well fitted with equation (C18) using $B = 1.73$, $\sigma_{ext} = 0.01887 \ \mu^2$ and $L = 0.00407 \ \mu^2$ with $\rho$ expressed in units of particles/$\mu^2$. These values were obtained by a nonlinear curve fit of equation (C18) to experimental data. At low particle densities, the scattered light intensity is expected to vary linearly with surface density and equation (C18) reduces to

$$I_s = 2.303 B \sigma_{ext} \rho . \qquad (C19)$$

Comparing experimental data at low particle densities with a plot of equation (C 19) using the values for B, $\sigma_{ext}$ and L presented hereinabove shows that equation (C 19) gives a very good fit to the data. Additionally, equation (C18) gives a very good representation of experimental data for other particle diameters.

It should also be noted that many electronic sensors have a response that is approximately linear throughout a range of intensities for a particular exposure time, and thus can be directly used for quantitation. However, if a sensor does not provide such linear range, or if significant deviations occur at some point(s) in the range, the recorded values can be compensated by weighting the values for different detected signals in order to correct to accurate quantitation. The correction can also be wavelength dependent, as the response of the sensor may differ at different wavelengths. In addition, for sensors that have different sensitivities to different wavelengths, the quantitation also preferably includes the use of different weightings or of different standard curves depending on the wavelength of scattered light detected. Wavelength-dependent quantification is particularly important in multiplex assays where sets of distinguishable particles are used that have different peak scattering wavelengths and so provide scattered light signals of different colors on illumination with polychromatic (preferably white) light. Compensating for differing sensitivities to different wavelengths of light permits proper quantification. Specifications on such sensitivities and deviations from linearity of response may be available from the sensor or camera manufacturer or distributor, or can be determined for each sensor with the use of standard media, *e.g.*, slides with spots of defined particle type (for defined scattered light characteristics) and defined densities.

As indicated hereinabove, the methods described herein for extending dynamic range are particularly advantageous for use with assays that utilize RLS particles. However, the methods can also be applied to other particulate labels. For example, they can be applied to fluorescent particles, *e.g.*, polystyrene beads loaded with multiple fluorophores such as fluorescein or other fluorescent dyes. In general, single small molecule fluorophores are not amenable to individual visualization in assays, but loaded beads or microspheres can be. However, applying the present techniques to fluorescent labels also presents difficulties associated with the nature of the label. For example, at high label densities, it may be necessary to deal with fluorescence quenching and bleaching, and for repeat or extended exposures it may also be necessary to deal with bleaching. Furthermore, the time course of signal decay for fluorescent labels is dependent on label density.

Thus, for methods of relating the signal to particle concentration, the mathematics is more complicated for fluorescent labels than for RLS particles. The multiple exposure technique is more limited for many assay formats involving fluorescence than for RLS particles, because the multiple exposure technique is most useful where the signals from the labels are stable over time, for example the signals are not subject to decay or photobleaching, and where the detection method has a high signal/noise ratio. In many fluorescence applications, the signal/noise ratio for example, curtails that benefit, for example, because increasing the exposure time to pull in weak signals also increases the inherent background noise.

Apparatus suitable for carrying out the assay methods with extended dynamic range as described above are compatible with the methods of the present invention. In general, the systems are configured as described in, for example,

**EP 2 302 363 A2**

U.S. provisional application no. 60/317,543. Preferably, such apparatus has encoded therein instructions for performing the calculations described hereinabove.

*Multi-Color Imaging and Deconvolution*

In some applications, the detection and measurement of only one particle type is required, while in other applications, the detection and measurement of two or more particle types in a mixture, including multiparticle structures, is desirable. It is possible and advantageous to simultaneously assay two or more analytes in a sample by using particles of two or more different types, as indicated by, for example, size or composition.

For example, in microarray based methods of analysis, the same particle type may be used to determine the amount of binding in each separate binding zone of the array. In other applications, such as biological cell based analysis, two or more types of light scattering particles may be used to identify and quantify different cellular constituents. In other applications of the present invention, the presence and amount of multiparticle structures are detected and measured. In yet other applications, the distribution of particle sizes and multiparticle structures is determined.

As an illustration, in applications to cell biology, the cell is a highly scattering object and contains intracellular components that can appear as particles of various shape, size, and color. One of skill in the art preferably uses light scattering particle type(s) which have one or more detectable and measurable properties which can be resolved from the cell. For example, particles whose radiated light color(s) are different from the scattered light radiating from the cell can be used with a color photodetector, with appropriate discrimination methods and limiting threshold values, to specifically detect, measure, and quantify the light scattering particles.

The choice of particle pair for two color RLS multiplexed assays is important. Assuming that the two analytes have the same concentration ranges, two principal criteria for selecting an optimal particle pair for two color multiplexing are as follows. First, the peaks of the bands in the light scattering spectra of the two particles should be far apart in wavelength in order to minimize spectral overlap. The amount of overlap depends on relative sensitivity and range requirement for a given analyte in an assay. For example, for one decade of concentration range, an overlap of about 2:1 is generally acceptable. Preferably the peaks are separated by at least about 100 nm, though peaks separated by as little as about 10 nm are suitable. Second, the two particle types should have similar light scattering cross sections which correlate with the intensity of the scattered light from the two particle types so that the particles have similar sensitivities for analyte detection.

For example, preferred embodiments of the present invention use gold particles. As discussed hereinabove, gold particles of different sizes scatter white light differently and appear of different colors: 40 nm gold particles scatter green light, while 80 nm particles scatter yellowish green light, and 120 nm gold particles scatter orange light. However, because the light scattering power increases rapidly with particle diameter, it is not preferred to use gold particles of different colors for two color multiplexing in the case where the two analytes have similar concentration ranges. This is because the light scattering spectra are not far enough apart in wavelength. Accordingly, preferred embodiments of two color multiplexing according to the present invention use two particles with different compositions and similar light scattering cross section.

In particular, preferred embodiments of the present invention use a 60 nm silver particle and a 80 nm gold particle because they have very similar light scattering cross sections but have light scattering spectra with a sufficiently large wavelength separation (at least 100 nm) that they constitute a good "balanced" particle pair for a two color assay. Furthermore, the wavelengths involved have been found to be well suited for two-color detection, for example because of their suitable peak separation.

In order for quantitative analysis of two or more types of particles to be reliable, it becomes important to use software that can properly separate the signals from the different particle types from one another, and to independently attribute the various signals to the concentrations of the various analytes. In methods previously known in the art, it was only possible to apply spectral deconvolution to assays that have low concentrations of analytes. A goal of the present invention is to present a deconvolution method that can be applied regardless of analyte concentration and can therefore operate at high particle density and at high levels of brightness of scattered light.

The method described herein uses as references pure samples of each of two particle types and introduces linear algebraic expressions to deduce analyte concentrations in a sample that contains a mixture of particles. Although the discussion hereinbelow uses gold and silver particles as examples, it is to be understood that the methods are generally applicable to any suitable pair or larger set of particles, chosen according to various selection criteria described hereinabove.

Certain of the following symbols, used in the mathematical derivations hereinbelow, have the following meanings unless otherwise indicated:

$n$            Designator for number of RLS particle types in a mixture;

(continued)

| | |
|---|---|
| $i, k$ | Designator for RLS particle type ($i$ and $k$ are interchangeable); |
| $j$ | Designator for spectral sample; |
| $\lambda_j$ | Wavelength of spectral sample, $j$; |
| $P_i$ | RLS particle of type, $i$; |
| $\overline{P}_i$ | Reference of RLS particle of type, $i$; |
| $P_e$ | Unmixed RLS particle of type, $e$; |
| $\overline{P}_e$ | Reference of unmixed RLS particle, $P_e$ ; |
| $M$ | Mixture of n RLS particle types; |
| $T_{P_i \lambda_j}$ | Exposure time of RLS particle of type, $i$, at wavelength, $\lambda_j$; |
| $T_{\overline{P}_i \lambda_j}$ | Exposure time of reference of RLS particle of type $i$, at wavelength $\lambda_j$; |
| $T_{P_e \lambda_j}$ | Exposure time of unmixed RLS particle of type $e$, at wavelength $\lambda_j$; |
| $T_{\overline{P}_e \lambda_j}$ | Exposure time of reference of unmixed RLS particle $P_e$, at wavelength $\lambda_j$; |
| $T_{M \lambda_j}$ | Exposure time of mixture of $n$ RLS particle types, at wavelength $\lambda_j$; |
| $C_{P_i}$ | Concentration of RLS particle of type $i$, relative to 1 convenient "unit"; |
| $C_{\overline{P}_i}$ | Concentration of reference of RLS particle of type $i$, relative to 1 convenient "unit"; |
| $C_{P_e}$ | Concentration of unmixed RLS particle of type $e$, relative to 1 convenient "unit"; |
| $C_{\overline{P}_e}$ | Concentration of reference of unmixed RLS particle $P_e$, relative to 1 convenient "unit"; |
| $C_M$ | Concentration of mixture of $n$ RLS particle types, relative to 1 convenient "unit"; |
| $a_{P_i}$ | Abundance coefficient of RLS particle of type, $i$; |
| $a_{P_e}$ | Abundance coefficient of unmixed RLS particle of type $e$; |
| $A_{P_i}$ | Relative abundance coefficient of RLS particle of type, $i$; |
| $A_{P_e}$ | Relative abundance coefficient of unmixed RLS particle of type $e$; |
| $S_{P_i \lambda_j T_{P_i \lambda_j} C_{P_i}}$ | Spectral value of RLS particle of type, $i$, at wavelength $\lambda_j$, with exposure $T_{P_i \lambda_j}$, having concentration $C_{P_i}$; |
| $S_{\overline{P}_i \lambda_j T_{\overline{P}_i \lambda_j} C_{\overline{P}_i}}$ | Spectral value of reference of RLS particle of type $i$, at wavelength $\lambda_j$, with exposure $T_{\overline{P}_i \lambda_j}$, having concentration $C_{\overline{P}_i}$; |
| $S_{P_e \lambda_j T_{P_e \lambda_j} C_{P_e}}$ | Spectral value of unmixed RLS particle of type $e$, at wavelength $\lambda_j$, with exposure $T_{P_e \lambda_j}$, having concentration $C_{P_e}$; |
| $S_{\overline{P}_e \lambda_j T_{\overline{P}_e \lambda_j} C_{\overline{P}_e}}$ | Spectral value of reference of unmixed RLS particle $P_e$, at wavelength $\lambda_j$, with exposure, $T_{\overline{P}_e \lambda_j}$, having concentration, $C_{\overline{P}_e}$; |
| $S_{M \lambda_j T_{M \lambda_j} C_M}$ | Spectral value of mixture of $n$ RLS particle types, at wavelength, $\lambda_j$, with exposure, $T_{M \lambda_j}$, having concentration, $C_M$ ; |
| $I_{\overline{P}_e \lambda_j T_{\overline{P}_e \lambda_j} C_{\overline{P}_e}}$ | Intensity value of reference of unmixed RLS particle, $P_e$, at wavelength, $\lambda_j$, with exposure, $T_{\overline{P}_e \lambda_j}$, having concentration, $C_{\overline{P}_e}$ ; |
| $I_{P_e \lambda_j T_{P_e \lambda_j} C_{P_e}}$ | Intensity value of unmixed RLS particle of type, $e$, at wavelength, $\lambda_j$, with exposure, $T_{P_e \lambda_j}$, having concentration, $C_{P_e}$ ; and |
| $I_D$ | Intensity of detector in the dark at ~0 second exposure time, $D$. |

It can be further assumed that, as would be expected by one of ordinary skill in the art, the meaning of a symbol is appropriately modified, according to various subscripts that are present. For example, where $P_i$ denotes RLS particle $i$, $P_1$ denotes RLS particle of type 1.

In general, the process of multispectral deconvolution, or unmixing, is based on a model which equates mixed spectra with the linear combination of weighted reference spectra of each RLS particle type that is present. In order to determine the amount of each RLS particle type within a mixture, the composite spectra is unmixed into proportional amounts of reference spectra. Therefore, the goal of RLS particle unmixing is to deconvolve a mixture of component spectra to discriminate one RLS particle type from another.

The unmixing method is a mathematically complete treatment of linear algebra and provides an exact solution for the relative abundance coefficients that make up the linear equations involved. However, an exact solution is only achievable in the case when all RLS particle types are known, and no variance is present in the reference spectra. Therefore, the primary focus of the unmix process is centered on the elimination of noise from the RLS particle reference spectra. Noise is classified as any and all contributors of variance to the final results. There are two primary assumptions to consider with respect to accuracy in implementing such a method. The first is the assumption that the mathematical model reflects the actual underlying physical model; and the second is that the accuracy of the reference spectra matches the materials in the mixture.

The system and method described herein is focused primarily on the accuracy of reference spectra, since the mathematical treatment of unmixing methods may be familiar to one of skill in the art from other sources. Nevertheless, the proportionality coefficients resulting from application of unmixing methods are insufficient as a final representative value for the application of the methods to microarrays. Thus, an abundance to intensity transform is also derived, as described hereinbelow.

*System and Apparatus for Spectral Unmixing*

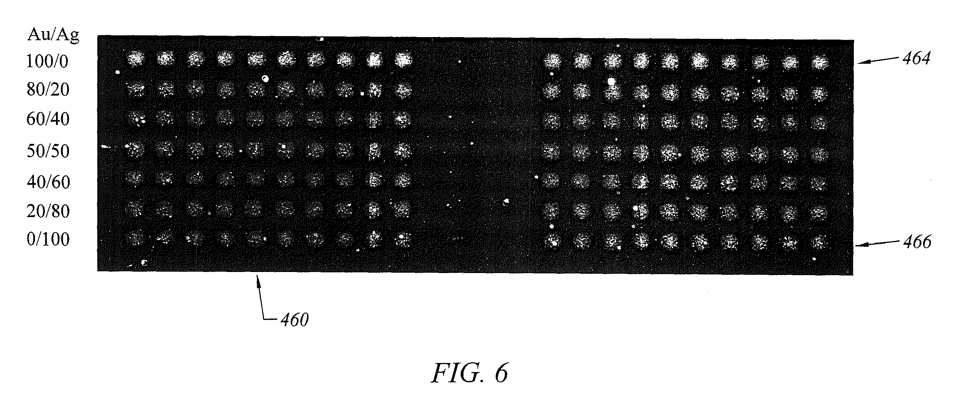

The goal of applying spectral unmixing to RLS particle-based analyte detection is to determine the proportion of each material in a mixture and to integrate the capability into software that run on a computer system. In a preferred embodiment, the system of the present invention incorporates a software toolkit comprising instructions and algorithms for performing multiplexed assays of two or more colors, *i.e.,* to allow separation and analysis of detected images that contain information from two or more particle types, wherein a particle of type $i$, is denoted $P_i$. In the examples discussed hereinbelow with respect to FIGS. 6-22, the two different types of RLS particles used are gold (*e.g.,* 80 nm in diameter) and silver (*e.g.*, 60 nm in diameter), though it is understood that particles of other compositions or sizes may be used, depending upon their RLS characteristics and the particular application.

An exemplary piece of commercially available software for unmixing is ENVI (available from Research Systems, Inc.). ENVI was designed for the field of remote sensing and image exploitation, and contains a feature called Linear Spectral Unmixing that can be adapted to the methods of the present invention.

The multi-color deconvolution software described herein is preferably incorporated within the image capture software of the present invention, and is preferably applied in real-time during a scanning or assay procedure. Alternatively, the multi-color deconvolution software may be incorporated into the analysis software described herein, or it may be a stand-alone module. In general, deconvolution is an algorithmic process of extracting underlying information from measured data that comprises contributions from a number of sources. In the present application, deconvolution attempts to ascertain the contribution of the various components from a mixture. In general, it attempts to utilize spectral intensity data, i.e., data at a number of wavelengths.

FIG. 6 shows a mixed image 460 of a microarray slide having two different types, $P_1$ and $P_2$, of RLS particles, 80 nm gold (Au) and 60 nm silver (Ag). Each row of microarray slide 460 includes spots of RLS particles composed of a particular ratio of Au/Ag, as shown. For example, row 464 has an Au/Ag ratio of 100/0, *i.e.,* is comprised entirely of gold particles, and row 466 has an Au/Ag ratio of 0/100, *i.e.,* is comprised entirely of silver particles. Because each spot (in rows other than rows 464 and 466) includes scattered light intensities from both particles, the relative intensity contributions of Au and Ag in a given spot cannot be seen in the image.

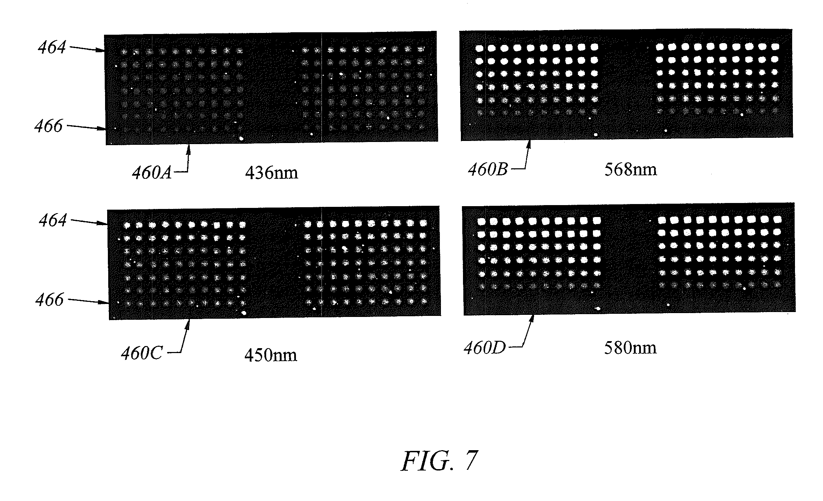

Referring to FIG. 7, four different images 460A, 460B, 460C, and 460D of the microarray slide of FIG. 6 were taken after passing the illumination light through four different bandpass filters centered at: 436 nm, 568 nm, 450 nm and 580 nm, respectively. Because Au and Ag light scattering particles have different spectral characteristics, the relative intensities of each row of spots are different in the different images 460A-D. Such multispectral images may be used to unmix the relative contributions of two or more RLS particles in a mixture and thereby produce abundance measures of the two types of particles.

In a preferred embodiment, band-images are captured from the instrument in sequence by causing the instrument to configure each filter while maintaining a fixed optical path with respect to the RLS particle media. This is important for achieving spatial alignment across each band-image, which is crucial to the success of unmixing RLS particle mixtures. If spatial alignment is not maintained between band-images, measurements will contain data from neighboring pixels, thereby corrupting the result.

Although two filters could be sufficient to perform a deconvolution of two RLS colors, a preferred embodiment uses more filters, *e.g.*, four filters, to provide an overdetermined solution. Filters are preferably used on the illumination side of the scanner device, such as filter 217 of filter wheel 216 in FIG. 2, however other positioning, such as positioning on the detection side of the scanner device may be used. In another embodiment, a single tunable filter is used.

*Selection of Filters*

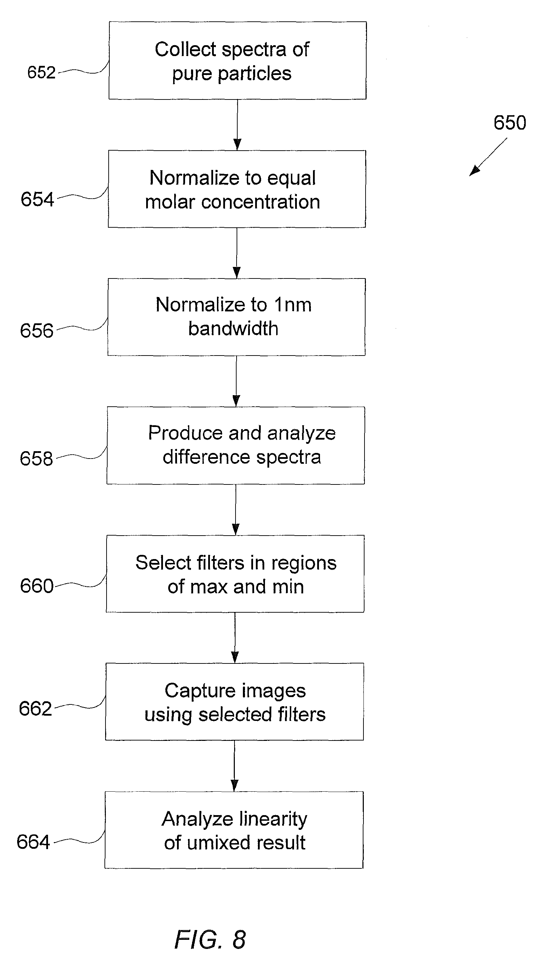

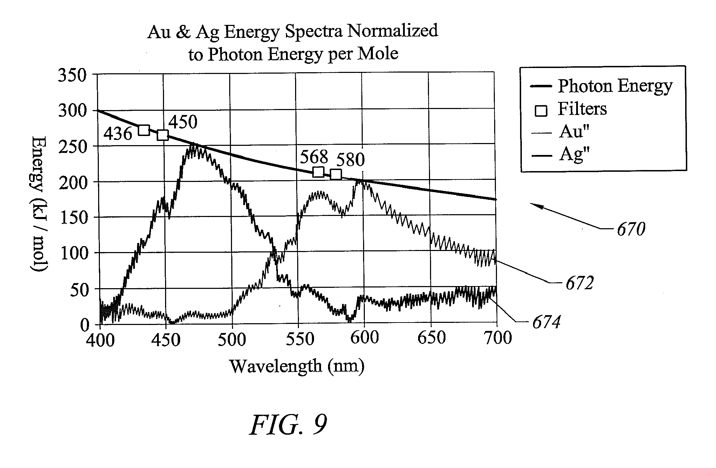

FIG. 8 shows an exemplary method 650 for selecting appropriate filters for the multispectral deconvolution processes described hereinabove. FIGs. 9-16 relate to each of the process steps 652, 654, 656, 658, 660, 662 and 664 of FIG. 8 wherein it is to be understood that the steps are not necessarily to be performed in the order shown.

In general, a spectra profile is represented by a chart that shows the response of a material to a particular wavelength, over a range of wavelengths. If it were possible to tune a filter, nanometer by nanometer, an almost continuous spectral profile would be obtained. However, in practical terms, it is preferable to obtain a few filters over a range of spectrum, and therefore to consider bands of wavelengths, for example ~70nm wide. Thus a spectral profile of a particle may be represented, effectively, as a vector of numbers, each of which is an intensity at a particular wavelength. A spectral value of a particle is typically encoded in a reference file, as discussed hereinbelow. In determining spectral profiles, then, in order to facilitate comparison of particle types, filters are preferably chosen that accentuate differences between the particles, in respect of their light scattering properties. In practice, of course, the spectral profile is affected by other factors such as concentration and exposure time. Filters may also be selected to offer a high density of coverage in areas of the spectrum where particle types exhibit similar responses. There is no requirement that the filters are equally separated from one another.

At step 652, spectra of pure particles are collected. It is preferable to carry this out for each particle type independently, and separately from the other particle types in question. The spectra of the individual types of particles are stored for subsequent use.

At step 654, the spectra of the respective particle types are normalized to equal molar concentration so that spectra for each particle type appear to represent spectra that would be obtained from solutions that have the same particle concentrations in moles per microliter. As shown in FIG. 9, spectra are also normalized to photon energy per mole. The spectra are also normalized for intensity at different wavelengths and at different concentrations. Thus, the spectra are subject to two different normalizations, one with respect to light scattering power, and another with respect to concentration.

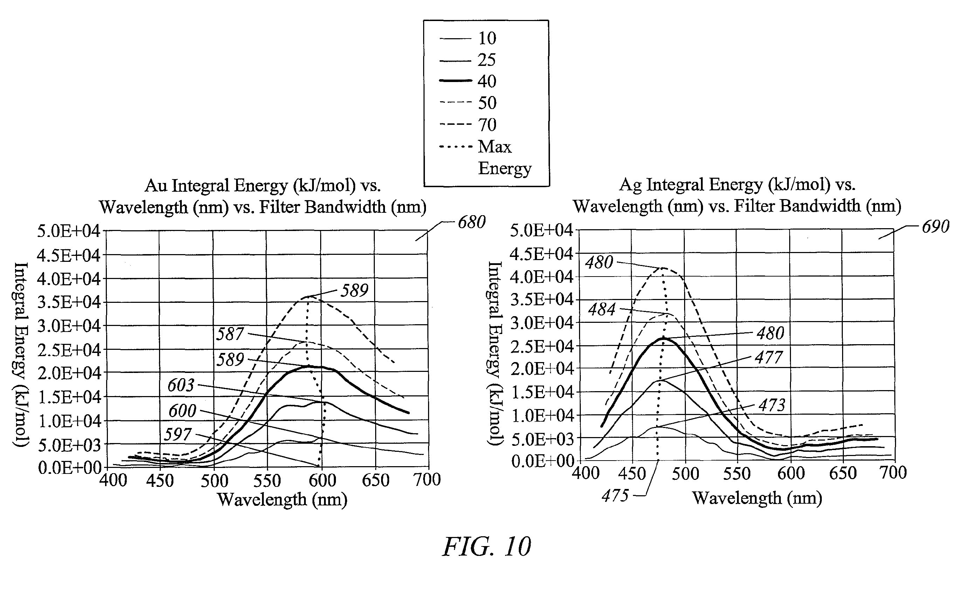

FIG. 10 shows plots of integral energy vs. wavelength for a number of filter bandwidths, for Ag and Au particles. Integral energy provides a measure of the energy of the scattered light. In FIG. 10, the integral energy is the sum of energies contributed by the RLS particles from all wavelengths within the filter bandwidth. Energy (in Joules/mole) at a wavelength) $\lambda$ is calculated from the following equation:

$$E = \frac{N_{Av}hc}{\lambda} \qquad (D0)$$

wherein h is Planck's constant and c is the speed of light.

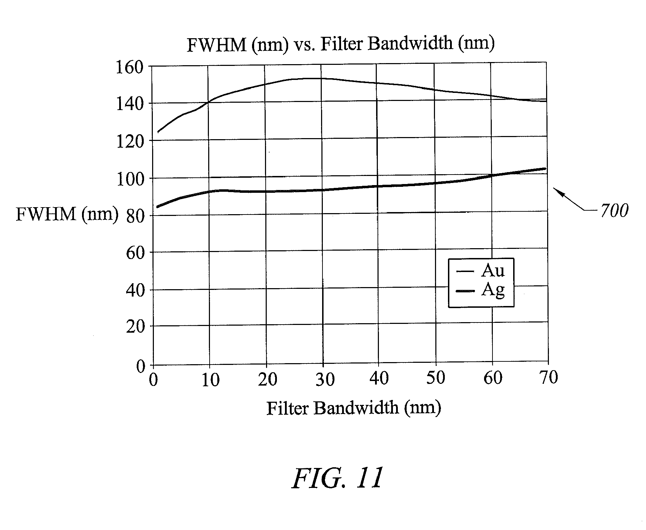

FIG. 11 shows the variation of full width at half maximum height with filter bandwidth. It can be concluded from FIG. 11 that the shape of the integral energy spectra for each type of particle, as given by the FWHM dimension of the profile, is not altered significantly by bandwidth. Since the FWHM filter characteristic is indicative of RLS particle selectivity, the invariance of this result indicates that RLS Particle selectivity is unaffected by filter bandwidth.

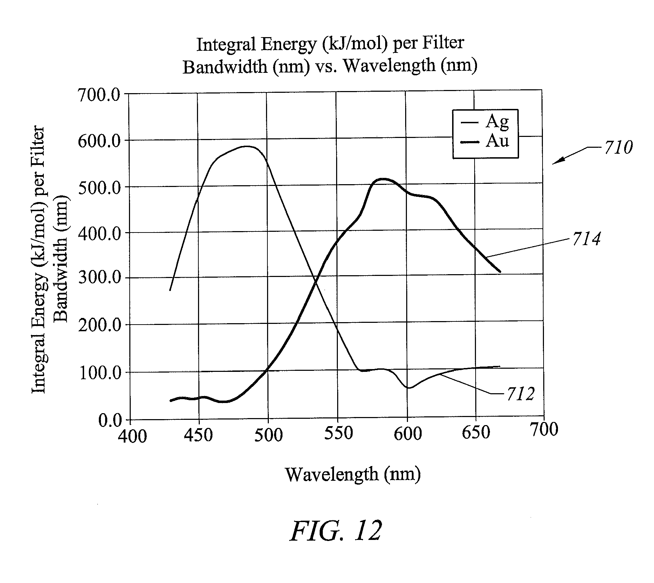

At step 656, the spectra are normalized to 1 nm bandwidth. An energy spectrum normalized to 1 nm bandwidth is calculated by determining the gradient in RLS particle integral energy over each nm bandwidth. The purpose of this calculation is to remove the effects of filter bandwidth from the analysis. In this way, conclusions drawn from the resulting spectral shapes may be extended into a practical system where one may choose to use bandwidth as an independent parameter. FIG. 12 shows the variation of the integral energy per filter bandwidth with wavelength.

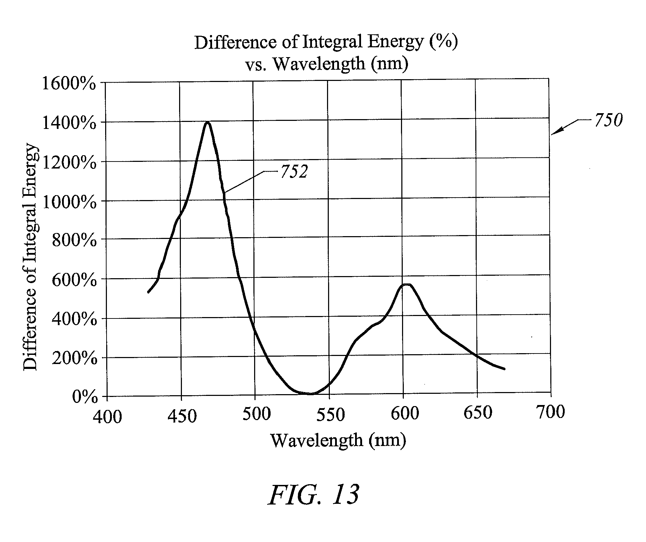

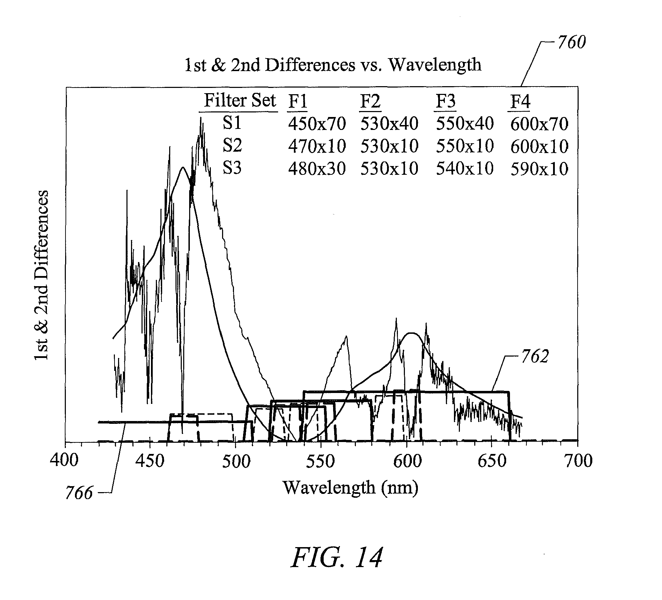

At step 658, difference spectra are produced, as shown in FIG. 13. It is preferable to select filters such that the differences between the spectral profiles of the two particle types are maximal. Difference spectra offer a way to compare spectral profiles of the particles. Analysis of such difference spectra permits selection, step 660, of filters in regions of maxima and minima, as shown in FIG. 14. It is to be noted that the spectral profiles of the different particle types are distinguishable from one another.

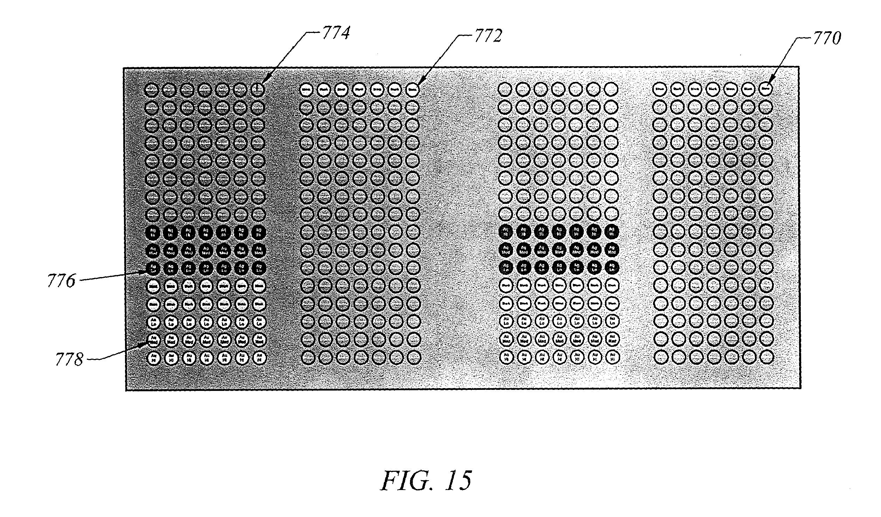

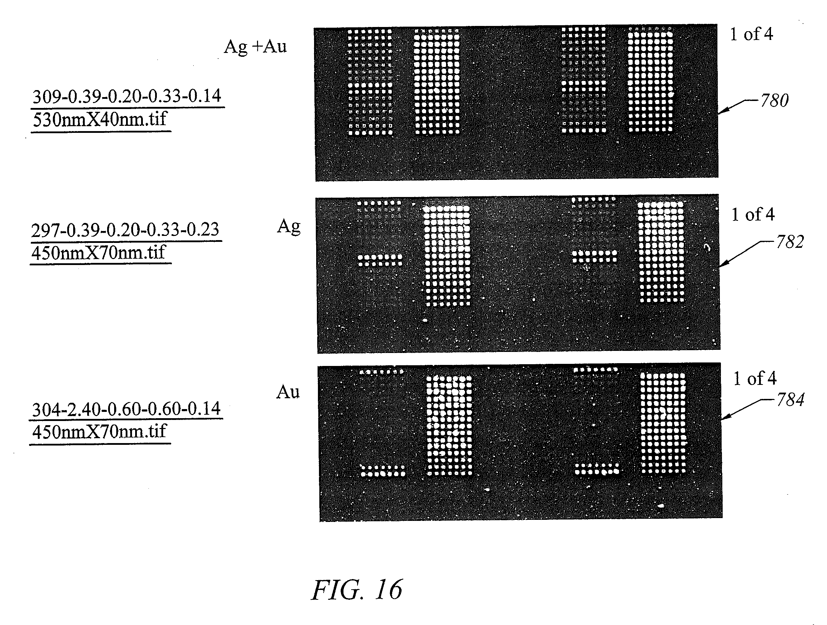

FIG. 15 shows a calibration slide as may be used in conjunction with the method of selecting filters presented herein. Having selected filters, images are then captured, step 662, representatives of which are shown in FIG. 16. Finally, the linearity of the unmixed result, step 664, is analyzed to determine filter efficacy.

*Conversion of Abundance to Intensity*

Effectively, abundance is a measure of the relative concentration at any convenient unit of a given type of RLS particle present in a sample. According to the methods of the present invention, to separate signals from a mixture of different RLS particle types into their respective components, multiple image measurements, each captured with different optical filters, are processed by linear algebra to solve for the abundance coefficients of known references. Each reference intensity is a response of a pure population of one type of RLS particle to the illumination applied to it, and is dependent on a number of factors, including: the particle type; the wavelength of light at which the intensity was measured; the length of time over which the intensity was measured; and the concentration of RLS particles in the sample. The resulting abundance coefficients, therefore, are proportional to amounts of their corresponding reference, which, when recombined for each RLS particle type in the mixture, add up to the total intensity measured.

The methods presented hereinbelow show how the derived abundance coefficients may be used to create an intensity of an image as if the image had originated from a single RLS particle type, rather than from a mixture of particle types. The principal utility of the resulting transform from abundance to intensity is to enable users, who may be more familiar with fluorescence imagery, to compare RLS particle images with one another with an equivalent level of understanding. It is to be understood that the methods presented hereinbelow are but one possible way in which a relationship between abundance and intensity may be derived and computed.

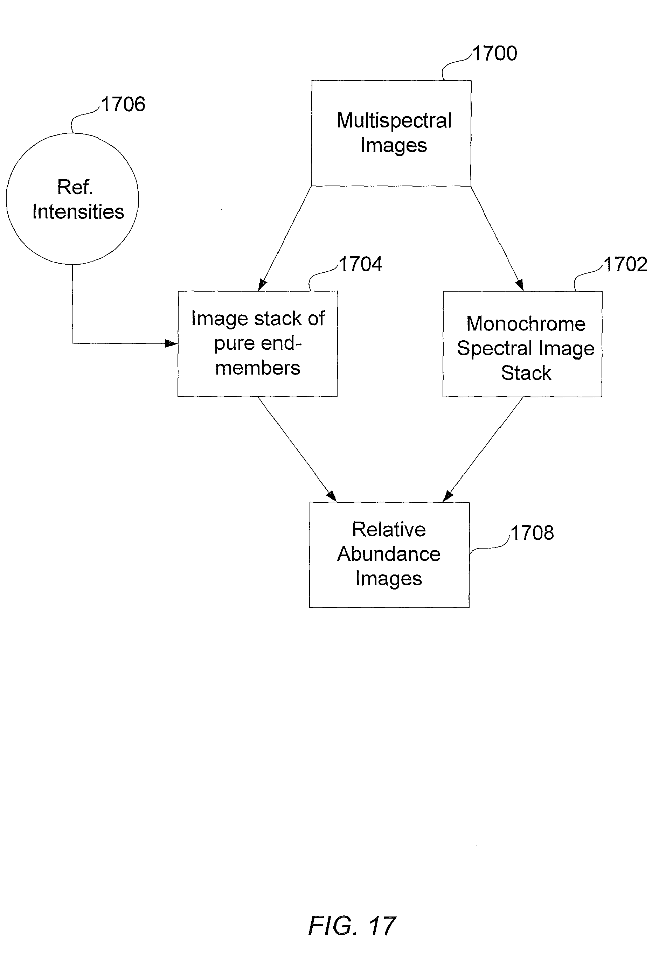

The relative abundance of each particle may be derived using a multispectral linear deconvolution process, shown in FIG. 17. Abundance is a coefficient representing a proportional amount of material, such as an RLS particle, relative to the sum of all abundance in all reference materials. An abundance coefficient is derived for each reference component using linear deconvolution of spectrally dependent measurements of intensity from an RLS mixture, and reference intensities from known concentrations of pure RLS particles. Therefore, the resulting abundance coefficients represent relative concentrations.

Overall, as shown in FIG. 17, multispectral images 1700 are deconvoluted to produce an image stack of monochrome images 1702 and an image stack of pure end-members 1704. The pure end-members are produced with the aid of a set of reference intensities 1706. Together, the monochrome spectra and the pure end-members are processed to give relative abundance images 1708.

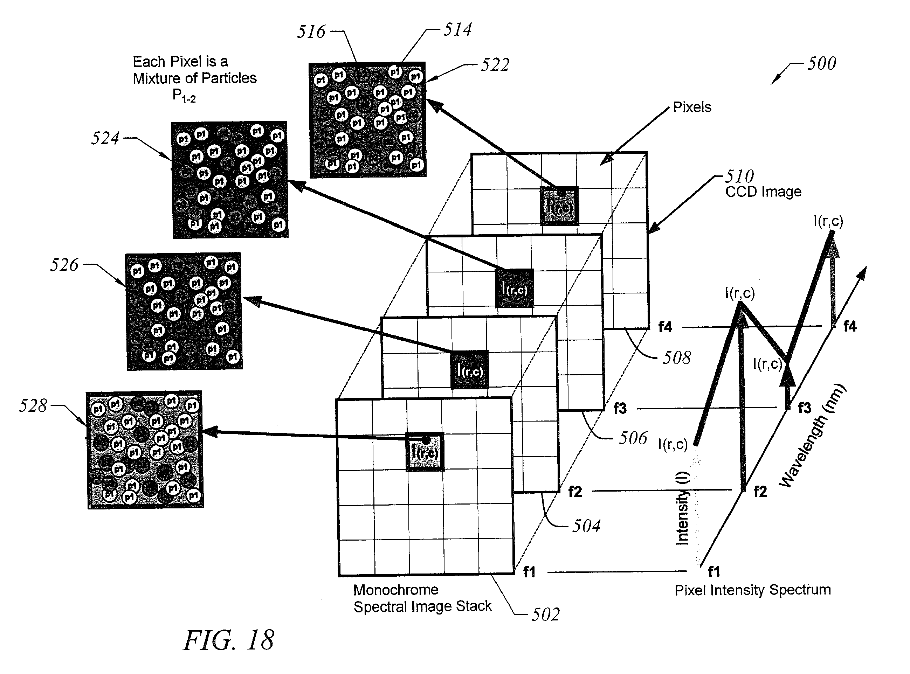

FIG. 18 illustrates a preferred arrangement 500 of bandpass filters that may be used in conjunction with methods of deconvolution, or unmixing, according to the present invention. To unmix a sample having two colors, two or more bandpass filters 502, 504, 506, and 508 (*e.g.*, as described with respect to FIG. 7) corresponding to different frequencies f1, f2, f3 and f4, respectively, are used to capture a number of multispectral images 510, one for each filter, and to create a monochrome spectral image stack, or "cube", 530. Each pixel 511 of each image 510 in cube 530 includes a particular mixture of intensities from each particle of type $P_1$ (514) and of type $P_2$ (516). The coloring and intensity of light scattered from particles of type $P_1$ and of type $P_2$ appears different after passing through each respective filter, as illustrated by representative pixels 522, 524, 526 and 528. As shown in FIG. 18, it is possible for multiple particles to contribute to the intensity of a given pixel.

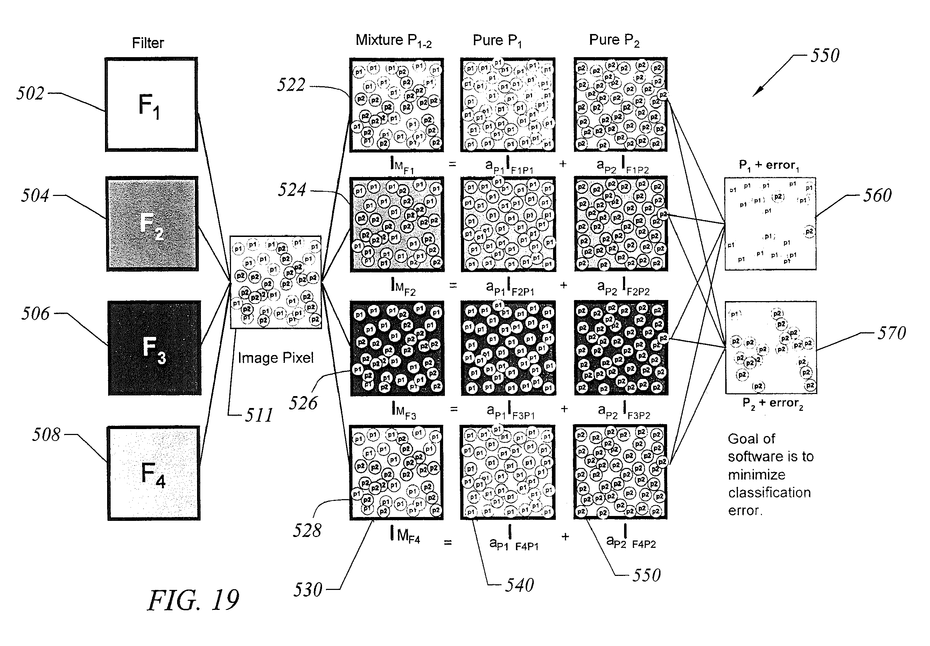

Referring now to FIG. 19, the results of applying a linear deconvolution process to an image pixel 511 are shown. Filters 502, 504, 506, 508 are used to create image stacks of pure end-member particles, *e.g.*, pure end-member $P_1$ cube 540, and pure end-member $P_2$ cube 550. End-member cubes 540 and 550 are also referred to as spectral reference image cubes because they contain signals from only one type of particle. The linear deconvolution matrix equations described hereinbelow, are then used to deconvolute the mixed pixel images, which comprise signal from both Ag and Au particles, into separate relative abundance images 560 and 570 for each particle type, $P_1$ and $P_2$, respectively. The spectral reference image cubes 540 and 550 may be generated and stored prior to the deconvolution procedure, and in some cases need only be re-created when a new filter set is used.

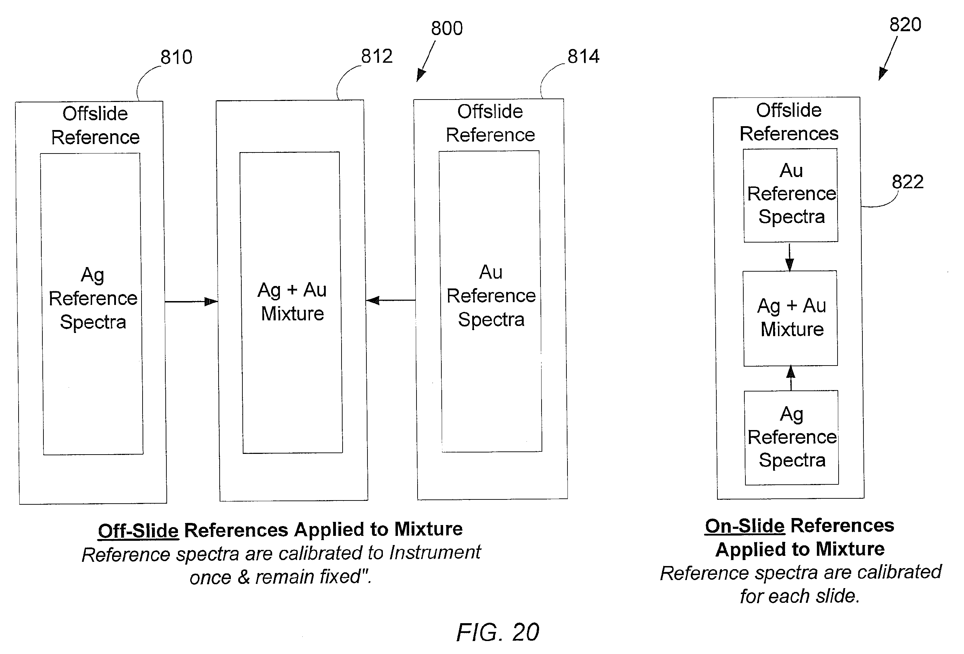

Referring to FIG. 20, the reference image cubes used in the multispectral deconvolution method of the present invention may be generated by a method 800 using off-slide references or a method 820 that uses on-slide references. For example, in off-slide method 800, the pure end-member image cubes may be generated as described hereinabove using reference slides having Au and Ag respectively, and by generating reference spectra 810 and 814 that are stored in the instrument for later application to the sample mixture 812. In contrast, the on-slide method 820 utilizes reference spectra on the same slide 822 as the mixture, and the reference spectra are calibrated for each slide.

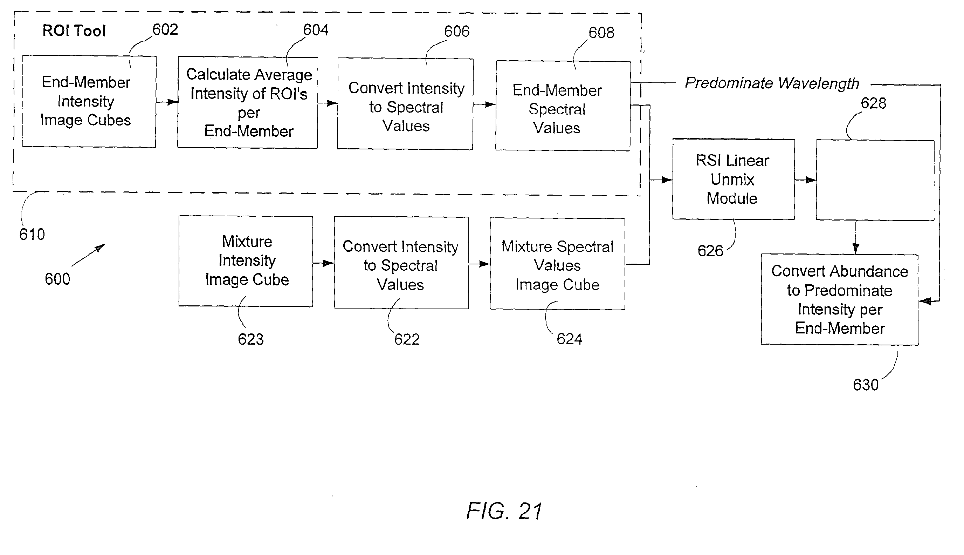

FIG. 21 shows steps in an algorithm 600 for multispectral deconvolution such as may be performed with software appropriately configured according to a preferred embodiment of the present invention. The application of algorithm 600 may result in predominate intensity per end member 630 as well as, or instead of, relative abundance 628. The predominate frequency is derived from the shape of the corrected spectrum of the spectral signature of the particles. The shape generally resembles a bell-curve in that it is continuous and has a single broad maximum of intensity. The predominate frequency is the frequency at the maximum.

In a preferred embodiment, certain steps in the algorithm are carried out using a region of interest (ROI) tool 610. In particular, the ROI tool may carry out steps 604 and 606, (see FIG. 21). The average intensities of selected regions of interest (*e.g.*, selected spots on an array) are calculated for each end-member (*e.g.*, $P_1$ and $P_2$) in step 604, using respective end-member intensity image cubes 602. The average intensity values are then converted, in step 606, into end-member spectral values 608 using equation (D1), hereinbelow.

A spectral value is analogous to an intensity value in a spectrum except that it is independent of exposure time and particle concentration. A spectral value results from transforming a raw intensity value into a unitless value that can be used to capture certain properties relative to those of a particle of another type. Exemplary steps in the transformation include subtraction of background and normalization for exposure and concentration. The resulting spectral value thus has the character of an intensity but cannot be directly perceived. Thus, the process of converting an intensity value into a spectral value is effectively a normalization method in the context of a multispectral deconvolution system since the proportional change in scattered light intensity is strictly attributed to the two parameters, time and concentration.

$S_{\overline{P}_e \lambda_j T_{\overline{P}_e \lambda_j} C_{\overline{P}_e}}$, the spectral value of a reference RLS particle type, $\overline{P}_e$, at wavelength $\lambda_j$, with exposure $T_{\overline{P}_e \lambda_j}$,

and having concentration $C_{\overline{P}_e}$, is derived from measured Intensity value $I_{\overline{P}_e \lambda_j T_{\overline{P}_e \lambda_j} C_{\overline{P}_e}}$. To derive the Spectral value

$S_{\overline{P}_e \lambda_j T_{\overline{P}_e \lambda_j} C_{\overline{P}_e}}$, the measured Intensity value $I_{\overline{P}_e \lambda_j T_{\overline{P}_e \lambda_j} C_{\overline{P}_e}}$, is compensated for detector bias noise $I_D$, is normalized to 1 second exposure and 1 concentration unit, and described by the following Equation (D1):

$$S_{\overline{P}_e \lambda_j T_{\overline{P}_e \lambda_j} C_{\overline{P}_e}} = \frac{I_{\overline{P}_e \lambda_j T_{\overline{P}_e \lambda_j} C_{\overline{P}_e}} - I_D}{T_{\overline{P}_e \lambda_j} C_{\overline{P}_e}} \qquad (D1)$$

wherein $I_{\overline{P}_e \lambda_j T_{\overline{P}_e \lambda_j} C_{\overline{P}_e}}$ is the measured intensity of reference particle of type $\overline{P}_e$, at a wavelength $\lambda_j$, with exposure time $T_{\overline{P}_e \lambda_j}$, $I_D$ is the intensity of detector $D$ in the dark at ~0 second exposure time.

The terms "unmixed" and "reference" are both used in order to distinguish the fact that the reference for a given unmixed RLS Particle is just one of many references in the system.

Similarly, intensity values for each mixture intensity image cube 620 (*i.e.,* a generalized representation of image cube 530 of FIG. 19) are converted in step 622 to spectral values to create a mixture spectral value image cube 624.

The equation used in step 622 to compute the spectral value $S_{M \lambda_j T_{M \lambda_j}}$ of a mixture of $M$ RLS particles, at wavelength $\lambda_j$, with exposure $T_{M \lambda_j}$ is:

$$S_{M \lambda_j T_{M \lambda_j}} = \frac{I_{M \lambda_j T_{M \lambda_j}} - I_D}{T_{M \lambda_j} C_M} \qquad (D2)$$

wherein the concentration, $C_M = 1$, $I_{M \lambda_j T_{M \lambda_j}}$ is the intensity of mixture $M$, at a wavelength $\lambda_j$, with exposure time $T_{M \lambda_j}$.

The concentration of the reference RLS particle is relative to 1 "unit". The unit is arbitrary but is preferably the same as the unit of abundance. In one embodiment, the unit may be particles per square micron. The expression on the right hand side of equation (D2) has a concentration of 1, implicitly, because it refers to a mixture and therefore is expressed relative to a particle reference concentration.

The resulting image cube of spectral values 624 and the end-member spectral values 608 are processed by a linear unmixing module 626 to derive the relative abundance 628 for each end-member, as shown hereinabove with respect to FIG. 19.

In a preferred embodiment of algorithm 600 of FIG. 21, the end-member abundance measures 628 are converted, in step 630, into predominate intensity values, $I_{P_i \lambda_p T_{P_i \lambda_p} C_{P_e}}$, for each end member using the following equation:

$$I_{P_e \lambda_j T_{P_e \lambda_j} C_{P_e}} = \left[ \frac{A_{P_e C_{P_e}} S_{\overline{P}_e \lambda_j T_{\overline{P}_e \lambda_j} C_{\overline{P}_e}} S_{M \lambda_j T_{M \lambda_j} C_M} T_{M \lambda_j}}{\left( \sum_i^n \left( A_{P_i C_{P_i}} S_{\overline{P}_i \lambda_j T_{\overline{P}_i \lambda_j} C_{\overline{P}_i}} \right) \right)} \right] + I_D \qquad (D3)$$

wherein $f_p$ is the predominate frequency of $P_i$ and $A_{P_i}$ is an abundance coefficient of RLS particle of type $i$. The predominate frequency is derived from the shape of the corrected spectrum of the spectral signature of the particles. The shape generally resembles a bell-curve, in that it is continuous and has a single broad maximum of intensity. The predominate frequency is the frequency at the maximum.

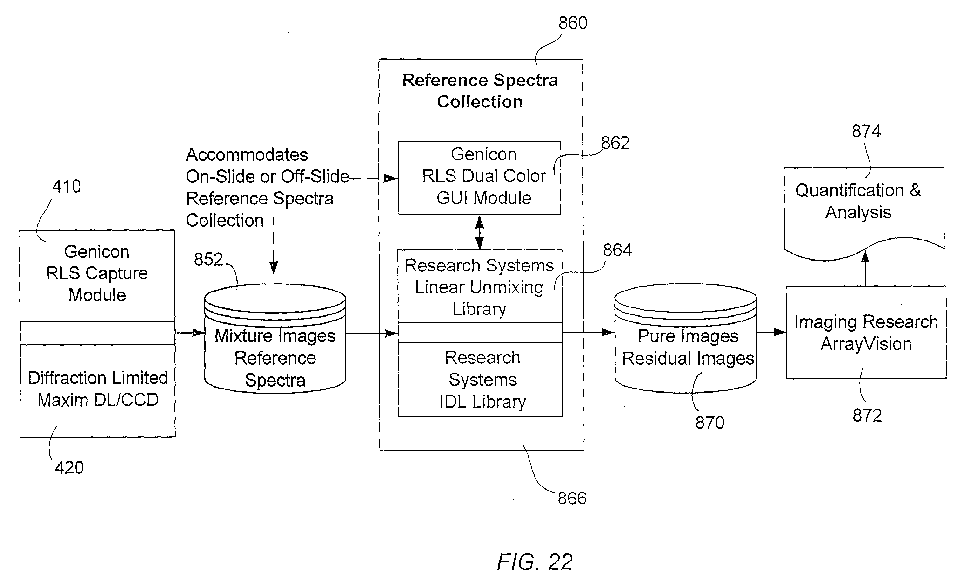

Although algorithm 600 is shown in FIG. 21 as a stand-alone module, it is preferably integrated into the overall use of scanning instrument and computer device and operates in real-time. For example, as shown in FIG. 22, the mixed RLS images 852 are captured and stored using the scanner control and capture modules 410 and 420 as described hereinabove. In particular embodiments, images are captured using Genicon ICS-501 Image Capture Software available from Genicon Sciences, Inc. and used in conjunction with a CCD. A multispectral image quantification module may be used to generate RLS Particle end-member reference files. The deconvolution module (the Genicon Separator) 860 can incorporate a graphical user interface 862 such as RLS Dual Color GUI, available from Genicon Sciences, Inc., to permit an operator to control the deconvolution algorithms and programs. Linear unmixing Library 864 may be incorporated from Research Systems, Inc., as may an IDL Library 866, which is an image description language for managing and processing image data.. In other embodiments the deconvolution algorithms and routines are implemented automatically without user intervention. The individual end-member images 870 are subsequently analyzed 874 using an analysis module 872 such as ArrayVision, available from Imaging Research, Inc., as described hereinbelow. In general, as used herein, "pure images" are the unmixed and end-member images, and "residual images" are intensity values from original mixture images that remain unassigned.

*Derivation of Spectral Amplitude from Abundance Coefficients*

In order to obtain relative abundance images for each particle type, appropriate spectral values must be obtained. The derivation presented hereinbelow produces an expression for the spectral value, $S_{P_e \lambda_j T_{P_e \lambda_j} C_{P_e}}$, of an unmixed RLS particle type, $P_e$, in terms of the spectral value, $S_{M \lambda_j T_{M \lambda_j} C_M}$, from a mixture of RLS particle types, $M$. This is achieved by determining the spectral value of the unmixed RLS particle type relative to the spectral value from a mixture of RLS particle types, using simple substitution and linear algebra, to solve simultaneous abundance equations. Abundance equations originate from the principle that the total amount of energy scattered by RLS particles in a mixture is equal to the sum of the energy scattered by each type of particle in the mixture, in proportion to its concentration. This principle was determined by Applicants during development of the present invention.

An abundance coefficient associated with each RLS particle present at a reference concentration is a mathematically derived quantity defined to represent the proportional amount of energy which that type of RLS particle has contributed to the total amount of scattered energy. Using discrete spectral observations of a RLS particle mixture, separate linear summations of abundance energies are formulated and subsequently solved for abundance coefficients which are ultimately used in representing RLS particle type component energy terms. These component energy terms are finally expressed in terms of the total energy observed in the mixture. The derivation proceeds as described hereinbelow.

Define the spectral value, $S_{P_e \lambda_j T_{P_e \lambda_j} C_{P_e}}$, of unmixed RLS particle type, $P_e$, at wavelength $\lambda_j$, with exposure $T_{\overline{P}_e \lambda_j}$, and having concentration $C_{P_e}$, as equal to the product of abundance coefficient $a_{P_e C_{P_e}}$, of unmixed RLS particle type $P_e$, having concentration $C_{P_e}$, and the spectral value, $S_{\overline{P}_e \lambda_j T_{\overline{P}_e \lambda_j} C_{\overline{P}_e}}$, of a reference RLS particle type $\overline{P}_e$, at wavelength $\lambda_j$, with exposure $T_{\overline{P}_e \lambda_j}$ and having concentration $C_{\overline{P}_e}$, as in equation (D4).

$$S_{P_e \lambda_j T_{P_e \lambda_j} C_{P_e}} = a_{P_e C_{P_e}} S_{\overline{P}_e \lambda_j T_{\overline{P}_e \lambda_j} C_{\overline{P}_e}} \qquad \text{(D4)}$$

Assume that the spectral value, $S_{M \lambda_j T_{M \lambda_j} C_M}$, of a mixture of RLS particle types, $M$, at wavelength, $\lambda_j$, with exposure, $T_{M \lambda_j}$, having concentration, $C_M$, is equal to the sum of $n$ spectral values, $S_{P_i \lambda_j T_{P_i \lambda_j} C_{P_i}}$, of unmixed RLS particle types, $P_i$, at wavelength, $\lambda_j$, with exposure, $T_{M \lambda_j}$ having concentrations, $C_{P_i}$, equation (D5):

$$S_{M\,\lambda_j T_{M\lambda_j} C_M} = \left( \sum_i^n S_{P_i \lambda_j T_{M\lambda_j} C_{P_i}} \right) \qquad (D5)$$

Substitute equation (D4) into (D5) while exchanging $i$ for $e$ to represent each unmixed RLS particle type, $P_i$. The result is that the Spectral value, $S_{M\,\lambda_j T_{M\lambda_j} C_M}$, of a mixture of RLS particle types, $P_M$, at wavelength $\lambda_j$, with exposure $T_{M\lambda_j}$, having concentration $C_M$, is equal to the sum of $n$ products of abundance coefficients $a_{P_i C_{P_i}}$ and spectral values, $S_{\overline{P}_i \lambda_j T_{\overline{P}_i \lambda_j} C_{\overline{P}_i}}$, of reference RLS particle types $\overline{P}_i$, at wavelength $\lambda_j$, with exposure, $T_{\overline{P}_j \lambda_j}$, having concentrations $C_{\overline{P}_i}$, as shown in equation (D6).

$$S_{M\,\lambda_j T_{M\lambda_j} C_M} = \left( \sum_i^n a_{P_i C_{P_i}} S_{\overline{P}_i \lambda_j T_{\overline{P}_i \lambda_j} C_{\overline{P}_i}} \right) \qquad (D6)$$

Abundance coefficients, $a_{P_i C_{P_i}}$ are solved from equation (D6) by computing a linear matrix of simultaneous equations, one equation for each spectral observation at wavelength $\lambda_j$, where $j \geq i$.

Further, define abundance coefficient, $a_{P_e C_e}$ as equal to a product of the relative abundance coefficient, $A_{P_e C_e}$, of unmixed RLS particle type $P_e$, having concentration $C_{P_e}$, and the sum, $\sum_k^n a_{P_k C_{P_k}}$, of $n$ RLS particle type abundance coefficients $a_{P_k C_{P_k}}$, of RLS particle type $P_k$, having concentration, $C_{P_k}$, contained in the mixture:

$$a_{P_e C_{P_e}} = A_{P_e C_{P_e}} \left( \sum_k^n a_{P_k C_{P_k}} \right) \qquad (D7)$$

To eliminate the abundance coefficient $a_{P_e C_e}$, from the spectral value, $S_{M\,\lambda_j T_{M\lambda_j} C_M}$, of a mixture $M$ of RLS particle types at wavelength $\lambda_j$, with exposure $T_{M\lambda_j}$, and having concentration $C_M$, equation (D7) is substituted into (D6):

$$S_{M\,\lambda_j T_{M\lambda_j} C_M} = \left( \sum_i^n A_{P_i C_{P_k}} \left( \sum_k^n a_{P_k C_{P_k}} \right) S_{\overline{P}_i \lambda_j T_{\overline{P}_i \lambda_j} C_{\overline{P}_i}} \right) \qquad (D8)$$

To eliminate the abundance coefficient $a_{P_e C_e}$, from the spectral value $S_{P_e \lambda_j T_{P_e \lambda_j} C_{P_e}}$, of unmixed RLS particle type $P_e$, at wavelength $\lambda_j$, with exposure $T_{P_e \lambda_j}$, and having concentration $C_{P_e}$, equation (D7) is substituted into (D4):

$$S_{P_e \lambda_j T_{P_e \lambda_j} C_{P_e}} = A_{P_e C_{P_e}} \left( \sum_k^n a_{P_k C_{P_k}} \right) S_{\overline{P}_e \lambda_j T_{\overline{P}_e \lambda_j} C_{\overline{P}_e}} \qquad (D9)$$

To determine the spectral value, $S_{P_e \lambda_j T_{P_e \lambda_j} C_{P_e}}$, of unmixed RLS particle type $P_e$, at wavelength $\lambda_j$, with exposure $T_{P_e \lambda_j}$, having concentration $C_{P_e}$, relative to the spectral value, $S_{M \lambda_j T_{M \lambda_j} C_M}$, of a mixture of RLS particle types $M$, at wavelength $\lambda_j$, with exposure $T_{M \lambda_j}$, and having concentration $C_M$, equation (D9) is divided by (D8):

$$\frac{S_{P_e \lambda_j T_{P_e \lambda_j} C_{P_e}}}{S_{M \lambda_j T_{M \lambda_j} C_M}} = \frac{A_{P_e C_{P_e}} \left( \sum_k^n a_{P_k C_{P_k}} \right) S_{\overline{P_e} \lambda_j T_{\overline{P_e} \lambda_j} C_{\overline{P_e}}}}{\left( \sum_i^n A_{P_i C_{P_k}} \left( \sum_k^n a_{P_k C_{P_k}} \right) S_{\overline{P_i} \lambda_j T_{\overline{P_i} \lambda_j} C_{\overline{P_i}}} \right)} = \frac{A_{P_e C_{P_e}} S_{\overline{P_e} \lambda_j T_{\overline{P_e} \lambda_j} C_{\overline{P_e}}}}{\left( \sum_i^n A_{P_i C_{P_k}} S_{\overline{P_i} \lambda_j T_{\overline{P_i} \lambda_j} C_{\overline{P_i}}} \right)} \qquad \text{(D10)}$$

Solving equation (D10) for the spectral value, $S_{P_e \lambda_j T_{P_e \lambda_j} C_{P_e}}$, of unmixed RLS particle type, $P_e$, at wavelength, $\lambda_j$, with exposure, $T_{P_e \lambda_j}$, and having concentration, $C_{P_e}$ leads to the following expression:

$$S_{P_e \lambda_j T_{P_e \lambda_j} C_{P_e}} = S_{M \lambda_j T_{M \lambda_j} C_M} \frac{A_{P_e C_{P_e}} S_{\overline{P_e} \lambda_j T_{\overline{P_e} \lambda_j} C_{\overline{P_e}}}}{\left( \sum_i^n A_{P_i C_{P_k}} S_{\overline{P_i} \lambda_j T_{\overline{P_i} \lambda_j} C_{\overline{P_i}}} \right)} \qquad \text{(D11)}$$

*Derivation of Intensity from Spectral Amplitude*

It is also important to show how the spectral values described hereinabove can be calculated from the actual measured intensity values acquired from the RLS detection instrument. A number of factors is involved. The detector is typically subject to noise in the form of a low-level bias, which preferably is first subtracted from the measured reading to exclude it from the unmixing process. Additionally, the exposure time is dependent on wavelength and must therefore be normalized across spectral observations. Finally, the relative concentration between each reference RLS particle type is preferably normalized to 1 concentration unit when measuring reference spectral values. This is carried out to enforce proportionality, which is assumed to be the condition by which the relative abundance coefficients are derived. The derivation of intensity from spectral values for reference RLS particle types and mixtures of RLS particle types is described hereinbelow.

The spectral value, $S_{\overline{P_e} \lambda_j T_{\overline{P_e} \lambda_j} C_{\overline{P_e}}}$, of a reference RLS particle type, $\overline{P}_e$, at wavelength, $\lambda_j$, with exposure $T_{\overline{P}_e \lambda_j}$, having concentration $C_{\overline{P}_e}$, can be derived from a measured intensity value, $I_{\overline{P_e} \lambda_j T_{\overline{P_e} \lambda_j} C_{\overline{P_e}}}$. To derive the spectral value, $S_{\overline{P_e} \lambda_j T_{\overline{P_e} \lambda_j} C_{\overline{P_e}}}$, the measured intensity value $I_{\overline{P_e} \lambda_j T_{\overline{P_e} \lambda_j} C_{\overline{P_e}}}$ is compensated for detector bias noise $I_D$, and is normalized to an exposure time of 1 second and 1 concentration unit, after which the spectral value is described by equation (D12):

$$S_{\overline{P_e} \lambda_j T_{\overline{P_e} \lambda_j} C_{\overline{P_e}}} = \frac{I_{\overline{P_e} \lambda_j T_{\overline{P_e} \lambda_j} C_{\overline{P_e}}} - I_D}{T_{\overline{P_e} \lambda_j} C_{\overline{P_e}}} \qquad \text{(D12)}$$

The spectral value, $S_{P_e \lambda_j T_{P_e \lambda_j} C_{P_e}}$ of an unmixed RLS particle type , may be calculated as if it were derived from a measured intensity, $I_{P_e \lambda_j T_{P_e \lambda_j} C_{P_e}}$, according to the following relationship:

$$S_{P_e \lambda_j T_{P_e \lambda_j} C_{P_e}} = \frac{I_{P_e \lambda_j T_{P_e \lambda_j} C_{P_e}} - I_D}{T_{P_e \lambda_j}} \qquad (D13)$$

where the spectral value has been compensated for detector noise, $I_D$, and normalized to a 1-second exposure.

The next step is to derive an expression for the intensity of the unmixed RLS particle type relative to the intensity of the mixture. Therefore, substitute equation (D13) into (D11):

$$\frac{I_{P_e \lambda_j T_{P_e \lambda_j} C_{P_e}} - I_D}{T_{P_e \lambda_j}} = S_{M \lambda_j T_{M \lambda_j} C_M} \frac{A_{P_e C_{P_e}} S_{\overline{P}_e \lambda_j T_{\overline{P}_e \lambda_j} C_{\overline{P}_e}}}{\left( \sum_i^n A_{P_i C_{P_k}} S_{\overline{P}_i \lambda_j T_{\overline{P}_i \lambda_j} C_{\overline{P}_i}} \right)} \qquad (D14)$$

Before solving equation (D14) for RLS particle intensity, $I_{P_e \lambda_j T_{P_e \lambda_j} C_{P_e}}$, it is important to recognize that the exposure time, $T_{P_e \lambda_j}$, of the unmixed RLS particle type, $P_e$, is equal to the exposure time, $T_{M \lambda_j}$, of the mixture of RLS particle types, *i.e.*,

$$T_{P_e \lambda_j} = T_{M \lambda_j} \qquad (D15)$$

Accordingly, substituting equation (D15) into (D14) and rearranging results in an expression for the intensity, $I_{P_e \lambda_j T_{P_e \lambda_j} C_{P_e}}$, of unmixed RLS particle type, $P_e$:

$$I_{P_e \lambda_j T_{P_e \lambda_j} C_{P_e}} = \left[ \frac{A_{P_e C_{P_e}} S_{\overline{P}_e \lambda_j T_{\overline{P}_e \lambda_j} C_{\overline{P}_e}} S_{M \lambda_j T_{M \lambda_j} C_M} T_{M \lambda_j}}{\left( \sum_i^n A_{P_i C_{P_k}} S_{\overline{P}_i \lambda_j T_{\overline{P}_i \lambda_j} C_{\overline{P}_i}} \right)} \right] + I_D \qquad (D16)$$

*Derivation of Region of Interest Spectral Amplitude from Pixel Intensity*

In order to carry out linear unmixing, step 626 of Fig. 21, a single average spectral value for representing reference RLS particle types, $\overline{P}_i$, is used and is derived hereinbelow. Each average spectral value is one amplitude value amongst others that make up a spectrum for a reference RLS particle type, $\overline{P}_i$. The spectral value is normalized for exposure time and relative concentration, and acts as the definitive signature for use in the linear unmixing matrix. The derivation of the spectral value utilizes the definition of a spectral value, as described by equation (D12).

Note that equation (D12) applies to a single intensity value, or pixel, $q$. Equation (D17) is equation (D12) restated with a subscript notation that explicitly associates a pixel, $q_U$, with a closed sub-Region of Interest ("SubROI"), *U*:

$$S_{q_U \overline{P}_i \lambda_j T_{q_U \overline{P}_i \lambda_j} C_{q_U \overline{P}_i}} = \frac{I_{q_U \overline{P}_i \lambda_j T_{q_U \overline{P}_i \lambda_j} C_{q_U \overline{P}_i}} - I_D}{T_{q_U \overline{P}_i \lambda_j} C_{q_U \overline{P}_i}} \qquad (D17)$$

First assume that each pixel, $q_U$, within SubROI $U$, may be treated equivalently. Therefore each pixel, $q_U$, contains a reference RLS particle type $\overline{P}_i$, at wavelength $\lambda_j$, with equal exposure $T_{q_U \overline{P}_i \lambda_j}$, and having equal concentration $C_{q_U \overline{P}_i}$, relative to one another throughout $U$. That is, the total number of pixels, $Q_U$, within $U$, make up a population with zero variance in both exposure and concentration. Thus, a single designation for exposure, $T_{Q_U \overline{P}_i \lambda_j}$, and concentration $C_{Q_U \overline{P}_i}$, for all the pixels, $Q_U$, within $U$, is introduced:

$$T_{q_U \overline{P}_i \lambda_j} = T_{Q_U \overline{P}_i \lambda_j} \qquad \text{(D18)}$$

$$C_{q_U \overline{P}_i} = C_{Q_U \overline{P}_i} \qquad \text{(D19)}$$

Therefore, applying equations (D 18) and (D 19) to (D17), and defining an average spectral value, $\overline{S}_{Q_U \overline{P}_i \lambda_j T_{Q_U \overline{P}_i \lambda_j} C_{Q_U \overline{P}_i}}$, based on all the pixels, $Q_U$, within $U$, containing reference RLS particle type $\overline{P}_i$, at wavelength $\lambda_j$, with exposure, $T_{Q_U \overline{P}_i \lambda_j}$, and having concentration, $C_{Q_U \overline{P}_i}$, an expression for $S$ becomes:

$$\overline{S}_{Q_U \overline{P}_i \lambda_j T_{Q_U \overline{P}_i \lambda_j} C_{Q_U \overline{P}_i}} = \frac{\sum_{q}^{Q_U} I_{q_U \overline{P}_i \lambda_j T_{q_U \overline{P}_i \lambda_j} C_{q_U \overline{P}_i}}}{T_{Q_U \overline{P}_i \lambda_j} C_{Q_U \overline{P}_i} Q_U} - I_D \qquad \text{(D20)}$$

Equation (D20) applies to SubROI, $U$, and is useful for cases where only one SubROI is found in the region of interest. It is desirable to generalize a solution for when an ROI is made up of multiple SubROI's. The reason why it would be useful to include multiple SubROI's is based on the supposition that variances in the response of RLS particle types, as a function of concentration, will be more accurately reflected in the ultimate reference Spectral value. Furthermore, it is expected that a calibrated linear dilution series of SubROI's will normally be available to provide useful data for determining the linear response range over which SubROI's apply.

Early results with the program ENVI (available from Research Systems Inc., Boulder, CO) and initial results with Genicon Separator (available from Genicon Sciences Corporation, San Diego, CA), show a high degree of sensitivity to the size of the ROI. It is worth noting that the statistical population of a SubROI, on its own, does not strictly adhere to the assumption that the pixels have a uniform concentration of reference RLS particles. Therefore, individual SubROI's already supply a statistical sampling of concentration variance, but unfortunately the variance is generally unknown. By contrast, in the approach described hereinbelow, multiple SubROI's are provided that offer an explicit description of concentration variance, by virtue of the known relative concentration values of the dilution series from which they originated. Overall, the increase in population of pixels across a wide variation of concentrations is statistically robust because increasing the population increases the sample set and the variance therein.

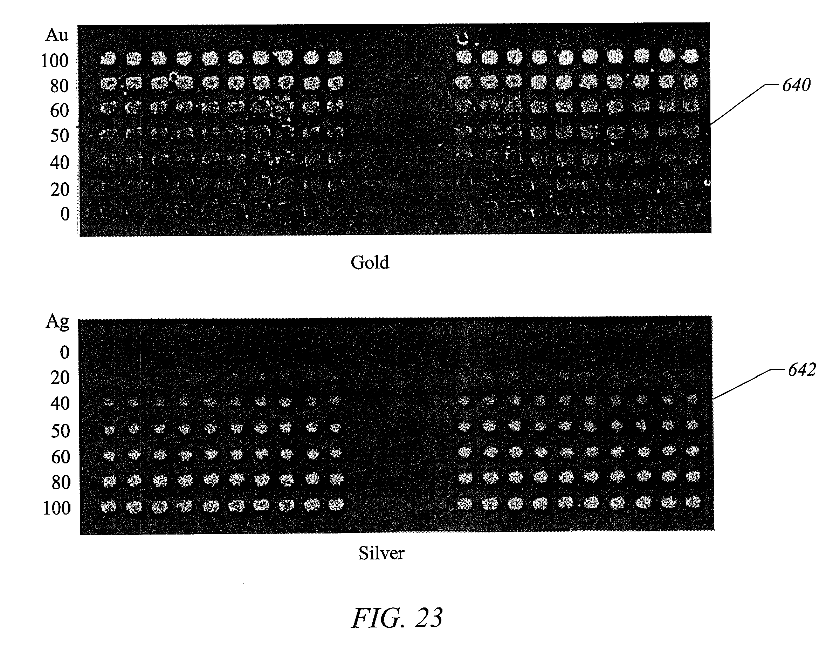

FIG. 23 shows results of unmixing of 2 particle titration spots.

Proceeding with the derivation, a group of SubROI's, called a Region of Interest (ROI), $R$, is introduced, wherein each SubROI has the same reference RLS particle type, $\overline{P}_i$, and wavelength, $\lambda_j$. The average ROI spectral value, $\overline{S}_{Q_R \overline{P}_i \lambda_j T_{Q_R \overline{P}_i \lambda_j} C_{Q_R \overline{P}_i}}$, is based on all spectral values from all SubROI's. Using the notation introduced by equation (D17), the spectral values, $S_{q_U \overline{P}_i \lambda_j T_{q_U \overline{P}_i \lambda_j} C_{q_U \overline{P}_i}}$, of all pixels from the SubROI's, of the ROI, are summed together, then divided by the total number of pixels. This calculation can be carried out without any further weighting, because each pixel's spectral value is already normalized for detector bias noise, $I_D$, normalized to 1 second exposure and to 1 concentration unit. Thus:

$$\overline{S}_{Q_R \overline{P}_i \lambda_j T_{Q_R \overline{P}_i \lambda_j} C_{Q_R \overline{P}_i}} = \frac{\sum\limits_{u}^{U_R} \sum\limits_{q}^{Q_U} S_{q_u \overline{P}_i \lambda_j T_{q_u \overline{P}_i \lambda_j} C_{q_u \overline{P}_i}}}{\sum\limits_{u}^{U_R} \sum\limits_{q}^{Q_U} q_u} \qquad (D21)$$

Equation (D21), together with equation (D17), enables the calculation of a normalized average spectral value for each reference RLS particle type.

*Derivation of Direct Comparative Intensity Constraint*

It is also possible to determine the conditions that permit the intensity $I_{P_e \lambda_j T_{P_e \lambda_j} C_{P_e}}$, of unmixed RLS particle type $P_e$, at wavelength $\lambda_j$, with exposure $T_{\overline{P}_e \lambda_j}$, having concentration $C_{P_e}$, to be directly compared with intensity $I_{P_s \lambda_j T_{P_s \lambda_j} C_{P_s}}$, of unmixed RLS particle type $P_s$, at wavelength $\lambda_j$, with exposure $T_{P_s \lambda_j}$, having concentration $C_{P_s}$. Direct comparison of intensities is useful because it enables a user to use ratiometric values (for example for use in 2 color gene expression array analysis), without the need to normalize the intensities prior to generating the ratio. The absence of normalization is an important step in achieving transparency in ordinary laboratory operation, a feature that is absent from current practice in competing technologies, such as assay-based fluorescence labeling and detection.

The purpose of deriving a condition that permits the unmixed intensities of each RLS particle to be directly compared, is to explicitly show that the presentational intensity ratios are equivalent to the underlying abundance coefficient ratios. In the end, both ratios should be the same, so this method offers an opportunity to exploit the differences between the two ratios as a means for enhancing system performance.

Initially, a concentration ratio, $R_{C_{P_s} C_{P_e}}$, is defined as the ratio of the concentration $C_{P_s}$, of unmixed RLS particle type $P_s$, to the concentration $C_{P_e}$, of unmixed RLS particle type $P_e$, where unmixed RLS particle types $P_s$ and $P_e$, are from the same mixture:

$$R_{C_{P_s} C_{P_e}} = \frac{C_{P_s}}{C_{P_e}} \qquad (D22)$$

It is asserted that the concentration $C_{P_e}$, of unmixed RLS particle type $P_e$, and the concentration $C_{P_s}$, of unmixed RLS particle type $P_s$, are equivalent to respectively, the relative abundance coefficient $A_{P_e C_{P_e}}$ of unmixed RLS particle type $P_e$, having concentration $C_{P_e}$, and the relative abundance coefficient $A_{P_s C_{P_s}}$ of unmixed RLS particle type $P_s$, having concentration $C_{P_s}$:

$$C_{P_e} = A_{P_e C_{P_e}} \qquad (D23)$$

$$C_{P_s} = A_{P_s C_{P_s}} \qquad (D24)$$

Thus, by combining equations (D22), (D23) and (D24), the concentration ratio, $R_{C_{P_s} C_{P_e}}$, may be stated as:

$$R_{C_{P_s}C_{P_e}} = \frac{A_{P_sC_{P_s}}}{A_{P_eC_{P_e}}} \qquad (D25)$$

The intensity ratio $R_{I_{P_s}I_{P_e}}$, is defined as the ratio of intensity $I_{P_s\lambda_jT_{P_s\lambda_j}C_{P_s}}$ of unmixed RLS particle type $P_s$, and the intensity $I_{P_e\lambda_jT_{P_e\lambda_j}C_{P_e}}$, of unmixed RLS particle type $P_e$, both of which are independently compensated for detector bias noise $I_D$, wherein the unmixed RLS particle types $P_s$ and $P_e$, are from the same mixture:

$$R_{I_{P_s}I_{P_e}} = \frac{I_{P_s\lambda_jT_{P_s\lambda_j}C_{P_s}} - I_D}{I_{P_e\lambda_jT_{P_e\lambda_j}C_{P_e}} - I_D} \qquad (D26)$$

Equation (D16) is substituted into equation (D26) to derive a simplified expression for the concentration ratio:

$$R_{I_{P_s}I_{P_e}} = \frac{\left[\dfrac{A_{P_sC_{P_s}}S_{\overline{P_s}\lambda_jT_{\overline{P_s}\lambda_j}C_{\overline{P_s}}}S_{M\lambda_jT_{M\lambda_j}C_M}T_{M\lambda_j}}{\left(\sum_i^n\left(A_{P_iC_{P_i}}S_{\overline{P_i}\lambda_jT_{\overline{P_i}\lambda_j}C_{\overline{P_i}}}\right)\right)}+I_D-I_D\right]}{\left[\dfrac{A_{P_eC_{P_e}}S_{\overline{P_e}\lambda_jT_{\overline{P_e}\lambda_j}C_{\overline{P_e}}}S_{M\lambda_jT_{M\lambda_j}C_M}T_{M\lambda_j}}{\left(\sum_i^n\left(A_{P_iC_{P_i}}S_{\overline{P_i}\lambda_jT_{\overline{P_i}\lambda_j}C_{\overline{P_i}}}\right)\right)}+I_D-I_D\right]} = \frac{A_{P_sC_{P_s}}S_{\overline{P_s}\lambda_jT_{\overline{P_s}\lambda_j}C_{\overline{P_s}}}}{A_{P_eC_{P_e}}S_{\overline{P_e}\lambda_jT_{\overline{P_e}\lambda_j}C_{\overline{P_e}}}} \qquad (D27)$$

Equation (D28) is now introduced to achieve a condition whereby either the concentration ratio $R_{C_{P_s}C_{P_e}}$, or the intensity ratio $R_{I_{P_s}I_{P_e}}$, will produce the same results, *i.e.*:

$$R_{C_{P_s}C_{P_e}} = R_{I_{P_s}I_{P_e}} \qquad (D28)$$

Substituting equations (D25) and (D27) into (D28) gives:

$$\frac{A_{P_sC_{P_s}}}{A_{P_eC_{P_e}}} = \frac{A_{P_sC_{P_s}}S_{\overline{P_s}\lambda_jT_{\overline{P_s}\lambda_j}C_{\overline{P_s}}}}{A_{P_eC_{P_e}}S_{\overline{P_e}\lambda_jT_{\overline{P_e}\lambda_j}C_{\overline{P_e}}}} \qquad (D29)$$

Simplifying equation (D29) gives:

$$\frac{S_{\overline{P}_s \lambda_j T_{\overline{P}_s \lambda_j} C_{\overline{P}_s}}}{S_{\overline{P}_e \lambda_j T_{\overline{P}_e \lambda_j} C_{\overline{P}_e}}} = 1 \qquad \text{(D30)}$$

The conclusion drawn from equation (D30) is that the spectral value, at wavelength $\lambda_j$, of reference RLS particle type $\overline{P}_s$ with exposure $T_{\overline{P}_s \lambda_j}$, and having concentration $C\overline{P}_s$, must be equal to the spectral value of reference RLS particle type $\overline{P}_e$, with exposure $T_{\overline{P}_s \lambda_j}$, having concentration $C\overline{P}_e$, for the condition of equivalency between concentration ratio $R_{C_{P_s} C_{P_e}}$, and the intensity ratio $R_{I_{P_s} I_{P_e}}$, equation (D28), to be met. In practice, this can be achieved by determining the wavelength $\lambda_j$, at which both the spectral value of reference RLS particle type $\overline{P}_s$, and the spectral value of reference RLS particle type $\overline{P}_e$, are equal. This particular spectral wavelength is referred to as the "isosbestic" wavelength.

In practice, the isosbestic wavelength is conventionally the center wavelength of a spectral band defined by the bandwidth, or full-width at half maximum height, associated with the optical filter used in measuring the intensity signal. However, absolute equivalency is hard to achieve, so the goal of implementing a system with this requirement is preferably to minimize the error between the reference RLS particle spectral values within the isosbestic band. This can be achieved by modifying the center wavelength, bandwidth or RLS particle characteristics. As described hereinabove, monitoring the difference between the underlying abundance coefficient ratio and the intensity ratio offers a means for meeting system convergence. Thus, if we have an isosbestic wavelength, it is possible to use the observed difference as a measure of convergence, in an iterative manner. As such, the error between the reference RLS particle spectral values within the isosbestic band can be minimized through modification of the isosbestic filter characteristics, while keeping the RLS particle characteristics constant.

The use of an isosbestic band for transforming abundances into intensities is required to enable direct ratiometric comparison of intensities, as if they were abundances. This is an important constraint in an RLS detection system, and helps to reduce systematic workflow requirements. Furthermore, having a closed solution for transforming data between these two representations by way of an optical processing element (the isosbestic band filter) offers a novel way to uniquely exploit microarray imagery. Also shown was a means to directly access abundance data, on a pixel-by-pixel basis, offering an opportunity to generate microarray regulation imagery. Subsequent use of such imagery for quantification and segmentation applications is possible.

*Application of Abundance Ratio to Regulation Imagery*

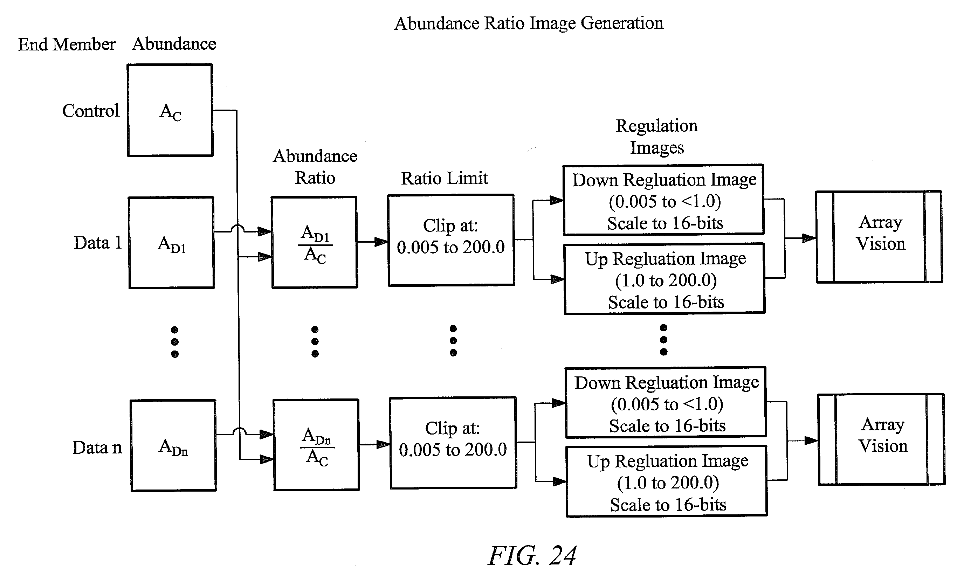

It is also possible to apply Abundance coefficient ratios to directly generate regulation imagery (*i.e.*, images that provide visual insight into regulation and automated quantification of gene regulation - as described in FIG. 24), for segmenting microarrays on a pixel-by-pixel basis, prior to transformation into intensity imagery. Such an approach would effectively partition signal obtained from a microarray feature into an "up" and "down" regulation set of intensity images, that act to partition the microarray image data prior to analysis. Since any one microarray feature, or spot, is normally associated with either "up" or "down" regulation as a whole, this new capability would act to filter out those pixels within an individual feature that are not performing as expected. Presumably, those filtered pixels represent noise or morphological structure that is undesirable in a quantitative analysis.

FIG. 24, shows an n-particle system whereby each unmixed particle abundance value, on a pixel-by-pixel basis, is used in a ratio relative to a control abundance. The ratios are clipped to some expression-level of interest and scaled into the appropriate "up" or "down" regulation image. Any number of follow-on applications are implied by quantification using a computer program such as ArrayVision. For example, the regulation images may be quantified by themselves to provide average "up" or "down" regulation values, on a feature-by-feature basis; or may be used as a pixel mask to filter intensity imagery prior to presentation and quantification.

*Artifact Rejection Techniques*

Artifact rejection techniques operate in a similar manner to the de-convolution method described above, in that filtered images are analyzed at the peak wavelength of the particle of interest, and at one or more off-peak wavelength positions. Gold or silver RLS particles will have the greatest signal at the peak wavelength of the particle type, while dust, salt and other artifacts will scatter more efficiently at other or multiple wavelengths. Off-peak wavelength images are compared to the peak wavelength images from the particle, and pixels containing signals identified as contamination can be flagged and later removed from quantification processes. Expand this to cover example 5

While described herein for RLS, the approaches of the present invention could also be applied to improve performance and data quality for other signal generation and detection system wherein increased resolution and improved quantitation of signal/background in cases where having a level of spectral overlap among system labels or among system labels and sources of background is desired.

In summary, equations (D11) and (D16) represent the final useful expressions for transforming Abundances into Intensities. Equations (D21) and equation (D17), are used to prepare the reference RLS particle type spectral values. Equation (D16) is used to transform the unmixed RLS particle type spectral values into intensity values as if the RLS detection instrument had acquired the intensity values directly. The expression (D16) may be used in preferred RLS detection system(s) to directly transform the output from the deconvolution software, *e.g.*, Genicon Separator software, on a pixel-by-pixel basis. Overall, this transform should eliminate most, if not all, of the negative abundance coefficients resulting from the unmix process. Further improvement in end-member representation can be achieved by incorporating a linear normalization strategy as discussed hereinbelow against the concentration dilution series used in the SubROI's. This can readily be achieved using the data collected from the images and the relative concentration values provided by the user.

*Data Analysis Module*

The images that result from, *e.g.*, spectral deconvolution, effectively capture the state of a microarray assay by digitally sampling the end product of various biochemical processes. Within a digital image, each pixel encodes individual sensory readings, which are eventually classified into signal categories. It is preferable to carry out a transformation from image space to feature space. In this transformation, an image is represented by pixels, and an example of a feature is a spot on a microarray slide. Such a transformation is referred to as segmentation. Determining whether a pixel contains one form of signal or another is part of such a segmentation process. For example, a pixel may contain dust particles as well as RLS particles. The overall segmentation process is an algorithm-based statistical selection that results in a fully qualified spot - absent artifacts. A fully qualified spot is one that has been quantified with only those pixels that are valid as coming from something of interest, for example a RLS particle, and not dust or some other artifact. Quantifying the spot results in a single integrated value that can be combined with the quantified results from a second microarray sample, usually a control sample, and is then expressed in the form of a ratio. Ultimately, it is this ratio that is used when carrying out quantitative analysis of the various spots. However, before ratios can be compared within a given study, spatially diverse controls, *i.e.*, controls at various locations on the assay slide, must be examined for equivalence, and adjustments applied to non-control spots based upon the outcome of such an examination.

According to the methods of the present invention, a systematic, mathematically derived solution to this problem can be used with a number of different assay methods. While this solution is preferably applied to RLS-based assay methods, it can also be used with other types of labeling and detection methods, such as fluorescence-labeling and detection.

*Overview of linear normalization*

One type of data processing involves data normalization. Such methods are particularly applicable to microarray data analysis. Normalization methods address the issue of how to conduct relative comparisons among large numbers of samples that have been measured under heterogeneous experimental conditions. It is to be understood that heterogeneous experimental conditions are those for which one or more parameters is not constant from measurement to measurement. Examples of such parameters include temperature, intensity of illuminating beam, presence of impurities, concentration of analyte, concentration of RLS particle, and presence of moisture in the ambient air. Therefore, the method of the present invention corrects experimental data for such heterogeneous effects before undertaking further analysis.

The normalization method of the present invention normalizes quantitative data from microarray assays, thereby effectively compensating for distortion from numerous sources of variation both inside and outside the system. Such distortions are typically caused by uniformly additive effects in the sense that they apply equally to all samples and are independent of one another. The normalization method of the present invention may work with spectral values, for example as computed by methods described hereinabove, or directly with raw intensity data.

Quantifying a microarray feature results in a single integrated value that may be compared with the quantified results from a second microarray sample, usually a control sample. Such a comparison can be expressed in the form of a ratio. However, in respect of variance, the control sample typically is no different from the experimental data sample. The method of linear normalization of the present invention uses regression analysis, which makes an underlying assumption that the control, or independent variable, is non-variant. Therefore, whether one chooses to use linear normalization or not, the question remains, which of the two data sets is better to use for the independent control when expressing a comparative result in the form of a ratio? The answer is that either data set is adequate, but that the two results are only meaningful if they exist in the same spatial coordinate framework. Linear normalization introduces such a framework to facilitate comparative expression analysis. It is important to note that the linear relationship that the method introduces does not rest on any assumption of linearity within the data, but merely expresses a relationship in a linear form for mathematical convenience and ease of representation.

The normalization method described herein assumes a knowledge of integrated light intensity values from fully qualified replicate control spots within and across the microarrays under study. Replicate control spots are paired up, between control and sample. For example, there is an equivalence between a feature in a first population and a replicate feature in a second population. Such control spots are referred to as spatially diverse controls and serve to provide a representative sample of various heterogeneous conditions that might distort the assay data. The method for correcting for these effects described herein is applicable if these spatially diverse controls are available in the assemblage of microarray spot data. Preferably the controls are distributed across a slide or microarray in a fashion that corresponds to the way in which the slide was prepared. For example, there can be one or more systematic errors in the spotting process of preparing a microarray or in its fabrication, that arise temporally or geographically across the array. It is preferable for the control spots to account for such variations. Thus, in a less preferred embodiment, the control spots are distributed randomly.

In general, the normalization process of the present invention involves applying a corrective model derived from control features, to adjust data from non-control features. When applied to microarrays, the non-control features are microarray elements whose expression intensities are measured under ordinary experimental conditions. Control features are typically specific microarray elements that express at theoretical *a priori* known levels, relative to other elements in the same microarray or to another external reference. Differences between actual and theoretical expression levels within the control data set are exploited by deriving a linear model through use of simple statistical methods. The resulting linear model typically varies from the ideal because of heterogeneous conditions that occur throughout the experiment.

The derived linear model from the control set, when re-applied to the control set, results in ideal conditions set forth by theoretical *a priori* expression levels. This linear model is a mathematical relationship between experimental and theoretical results, and produces a transform that corrects the control data in this way. Therefore, this normalization method applies the mathematically derived linear transform to the non-control set, as if the non-control set had been affected by the same heterogeneous conditions as had the control set. Once the non-control data set is corrected, normal analytical procedures, such as ratiometric expression analysis, can then proceed with higher confidence. The net effect of this method is to orient each data set into a common coordinate space before comparing the sets to one another. Thus, variations due strictly to misalignment of independent coordinate systems are eliminated. The result is that remaining variations found in a comparative analysis are more likely to be the result of actual variation due strictly to the experimental conditions under study.

When carrying out ratiometric analysis, it is preferred that a ratio of interest is at least 2:1, though in other embodiments ratios of 10:1 1 and 50:1 1 are useful.

*Linear Transform Normalization*

In the present linear transform normalization method, replicate control spots are interspersed within and across microarray assays to compensate for fluctuations that arise from biochemical processes and measurement uncertainties. In the normalization method of the present invention, there is no assumption of linearity in the intensity data. Two primary assumptions are that replicate control spots express equally, and that assay and system variations contribute uniformly. In real situations, as described hereinabove, there are many internal and external sources that contribute to undesirable system variations. However, by including an ideal set of scattered replicate control spots, the normalization method presented herein is likely to compensate for the additive effect of such contributions. The actual genetic makeup of the spots is not considered to be especially relevant to the method, although it is of course important to the researcher in designing a suitable assay.

Nevertheless, normalization is not intended to replace good experimental practice nor instrumentation design. Therefore, because the accuracy of regression analysis depends on the degree of scatter in the data, the more linear the data in the first place, the more accurate the regression model. As is understood by one of skill in the art, linear regression uses the method of least squares for determining the best-fit line for the given data. Thus, as described hereinabove, the regression model assumes that independent sources of fluctuation are applied uniformly across the data elements (spots) and add linearly. Given such an assumption, replicate control spots are used to determine a least-squares regression line, represented by a slope ($m$), and $y$-intercept ($b$). These values are then used in a linear transform equation that, when applied to the original dependent replicate control spots, results in a new regression line that has a slope exactly equal to 1 and a $y$-intercept exactly equal to 0. A derivation of the linear transform equation is outlined as follows.

A given data set comprises data points $X_n$ and $Y_n$ such that $x_m$ is a subset of independent variable $X_n$, and $y_m$ is a subset of dependent variable $Y_n$. Assuming that $x_m$ and $y_m$ are related sets (replicate control spots) such that for

each value of $x_m$, there is a corresponding value of $y_m$ that is expected to experimentally express equally to $x_m$, the best-fit linear regression line for the related data $\{x_m: y_m\}$ is:

$$y_m = m\,x_m + b \qquad (\text{N1})$$

wherein $x_m$ = spot intensity of independent variable, $y_m$ = spot intensity of dependent variable, $m$ = slope of replicate control spot regression line; and $b$ = intercept of replicate control spot regression line.

The calculations for $m$ and $b$ are based on the following best-fit least-squares formulae:

$$m = \frac{n\left(\sum xy\right)\left(\sum x\right)\left(\sum y\right)}{n\left(\sum x^2\right)-\left(\sum x\right)^2} \qquad (\text{N2})$$

and

$$b = \frac{\left(\sum y\right)\left(\sum x^2\right)-\left(\sum x\right)\left(\sum xy\right)}{n\left(\sum x^2\right)-\left(\sum x\right)^2} \qquad (\text{N3})$$

wherein $n$ = number of spots included in analysis, $x$ = independent variable, and $y$ = dependent variable, as would be known to one of ordinary skill in the art.

Let there exist a best-fit linear regression line:

$$y'_m = m'\,x_m + b' \qquad (\text{N4})$$

wherein $x_m$ = spot intensity of independent variable; $y'_m$ = normalized spot intensity of dependent variable; $m'$ = normalized slope of replicate control spot regression line; and $b'$ = normalized intercept of replicate control spot regression line.

Solving (N1) for $x_m$:

$$x_m = (y_m - b)/m \qquad (\text{N5})$$

Solving equations (N4) and (N5) simultaneously, results in a relationship between the original linear regression line and a new regression line:

$$y'_m = (m'/m)(y_m - b) + b' \qquad (\text{N6})$$

Let there exist a new normalized regression line having a slope of 1, and a $y$-intercept of *0, i.e.,*

$$m' = 1 \qquad (\text{N7})$$

$$b' = 0 \qquad (\text{N8})$$

Solving equations (N6), (N7) and (N8) simultaneously results in a linear transform equation that, when applied to the original replicate control spots, dependent variable ($y_m$) will produce a linear regression line that has a *y*-intercept equal to 0 and a slope equal to 1:

$$y'_m = ( y_m - b ) / m \qquad (N9)$$

Equation (N9) represents a linear transform model which estimates the characteristics of the linearly additive effects that cause variation across the replicate control spots. To complete the normalization process, the transform is applied to the balance of dependent variable data set, $Y_n$, thereby producing normalized assay data.

While the present method is designed to produce a linear regression line with slope equal to 1, and intercept equal to zero, the method could be modified to produce linear regression lines with different slopes and *y* intercepts. Nevertheless, lines whose slopes are equal to 1 and whose *y*-intercepts are equal to zero are preferred.

Although the method of linear normalization described hereinabove is normally applicable to replicate control spots paired across experiments, it can also be beneficially applied in the context of a single experiment. That is, by adding replicate control spots to a single experiment, normalization would be applied in the same manner to compensate for local intra-experiment fluctuations. Localized areas of reduced signal within an experiment may be spatially dependent and may therefore fall outside the general assumption of linearity. Such anomalies are preferably excluded from analysis and flagged as indicators for use in quality control of processes that are carried out prior to the analysis.

Once outliers are removed from consideration in the analysis, and additionally, or in the alternative, quality control measures are used to correct spatial or other non-linear effects, intra-experimental quantification values can be normalized reliably. To correct for global inter-fluctuations that occur between experiments, the same linear normalization procedure is reapplied across all experiments under investigation, using the paired replicate control spots, and the respective normalized intensity values. Overall, such a procedure results in a more finely controlled effect as compared to a single application of the process. Whether normalization is pursued from a biochemical, instrumentation, or quantification perspective, the process is highly beneficial.

It is useful to relate the general concept of a coordinate system in which all data resides, to the normalization strategies described hereinabove, in order to better understand why normalization is so important. The best-fit regression line through a data set represents the principal axis of the data set. Each data set has a principal axis. (In data sets that include mixed classes of data it can be shown that more than one principal axis exists.) Without normalization, when one data set has a different axis from another data set, comparisons made between the two sets are questionable. The goal, therefore, is to bring each data set into a common coordinate system by manipulating the individual principal axes into coincidence. Thus, one can visualize the process as a translation and rotation of the original data set into a coincident coordinate system that is the same as that of the control data set.

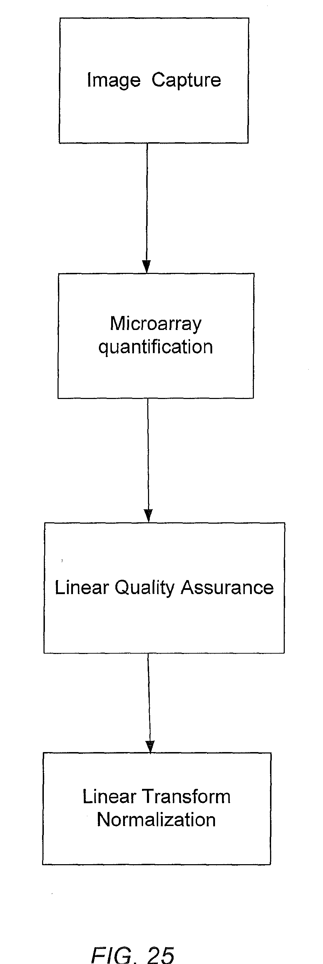

Nevertheless, with regard to detecting outliers in intra- or inter-experimental data, it is desirable to determine the statistical sampling requirements for the paired replicate control spots in order to attain robust results from the normalization process. Replicate specification is important for enabling the detection of a desired fold change, determining which replicate spots are poorly reproduced, and overall, which expression changes are statistically significant. In the context of an assay, a fold change is the level of measured difference in gene expression level for a given gene in a sample relative to a control sample. For example a 10 fold change in expression would be found if the EGF receptor is expressed in cancer at a level that is 10 times that which is measured in normal tissue. The method for determining the specifications for the replicates is iterative in nature, because the results of a study are first required to determine the degree to which the specification succeeds at producing linearity. Therefore, this part of the overall quantification process, as shown in FIG. 25, is preferably addressed by a separate set of analysis steps. Without the linear quality assurance step, there are opportunities to misinterpret results. Even without such a systematic approach, there are ways, however, to proceed with normalization, for example, as described hereinbelow.

Using such a method, one can inspect (preferably automatically) each quantification value to ascertain whether it exceeds a predetermined threshold, expressed as some number of absolute standard deviations, in order to detect outliers. Thereafter, the outliers can be excluded before applying the method to the data again. Once all of the outliers are excluded, normalization continues as described hereinabove. However, this approach requires a fixed threshold.

As indicated hereinabove, normalization of microarray data is important for many applications, *e.g.*, gene expression studies, because bioinformatic queries and an ability to understand expression relationships depend on accurate expression profiles. Attaining accurate measures of expression data requires robust biochemical procedures, reliable instrumentation, and sound methods of data processing. The importance of developing validation criteria along the process workflow is very important. The resulting utility of biological research depends in large part on accuracy of data as products are integrated with public and private bioinformatic and other database or information sources.

Signal generation and detection systems, in general, should address these elements in the context of a complete system. For the case of data processing, the normalization method described hereinabove offers an approach to data normalization that linearly compensates for inhomogeneities in hybridization, and other sources of fluctuation in micro-array assays. The process described is a linear model based on the estimation that signal fluctuations are uniformly added to experimental assays by undesirable internal and external sources. The proposed linear transform normalization method described hereinabove, uses paired replicate controls across experiments to provide a measure of these effects. The resulting best-fit least-squares regression formula provides metric parameters used to modify the data set dependent variables. The modified data is assumed to be corrected for the fluctuations characterized by the derived transform. Other estimation models are feasible. It is therefore reasonable to suggest the possibility of modifying this model to transform the data using a non-linear model to one that is linear.

In summary, the normalization method of the present invention assumes using paired replicate control spots across experimental microarray assays (and within a microarray assay for intra-experiment normalization) that vary in intensity across, and beyond, the entire range of intensities expressed by the assay. Normalization only concerns itself with the data and does not directly make use of concentration values or other characteristics of the assay such as the actual genetic content of the spots.

Overall, the method of the present invention provides a consistent means for normalization that can be used across experiments, as well as within a single experiment. It is recommended that expression profiles of known models be validated alongside experimental results to gain confidence in this or any other technique considered. For example, applying the approaches to published and widely used expression study data sets from well characterized biological systems having gene sets with known functional properties and interactions, as a control system, the stability and reproducibility of a given experimental process can be validated.

As indicated hereinabove, the normalization techniques can be implemented in software and/or hardware. Persons familiar with methods of computer programming can readily prepare the code for carrying out the functions involved. For example, ArrayStat® (by Imaging Research, Inc., St. Catharine's, Ontario, Canada) is designed to work in conjunction with ArrayVision®, a microarray quantification package (which is suitable for use with the detection and analysis system of the present invention). With ArrayVision®, methods for investigating the quantification results can be used to judge various solutions to the issues discussed hereinabove.

*Anomalous Feature Detection using Bi-Linear Normalization*

It is further possible to apply the method of linear normalization discussed hereinabove to quality control, in particular to the detection of anomalous samples among a large population of samples that has been subject to heterogeneous experimental conditions. A systematic solution to this problem has been developed in which a single body of comparative data is transformed into two separate, but equally valid, representations. The resulting separation is then exploited by comparing one representation to the other in order to determine if there is compliance with expectations. Thus, using this process, a researcher can identify specific members of a population that merit closer analysis to determine possible factors that contribute to the anomalous behavior. The meaning of the behavior itself is not interpreted as being either "good" or "bad", but instead that the feature is beyond the threshold of expectations. Furthermore, as would be obvious to one skilled in the art of data analysis, detection of deviant behavior is based on a threshold, and that such a threshold is typically set to some number of standard deviations above and below the average or median of the data population under study. This threshold is at the discretion of the analyst and set based on the context of the study.

The preferred linear normalization technique used in this process is a method described hereinabove by which a corrective model is derived from control features and is subsequently applied to adjust data from non-control features. In the context of microarray analysis, features are elements from a microarray which are measured for expression intensity under experimental conditions. Control features are specific microarray elements that express at theoretical *a priori* known levels relative to other elements in the same microarray or to other external reference. Differences between actual and theoretical expression levels within the control data set are exploited by statistically deriving a linear model by linear regression analysis.

The derived linear model of the control set, when applied back to the control set, results in ideal conditions set forth by theoretical a priori expression levels. This linear model is a mathematical relationship between experimental and theoretical results, and will always produce a transform that corrects the control data in this way. The concept of linear normalization is to apply the mathematically derived linear transform to the non-control set, as if the non-control set had undergone the same inhomogeneous effects of the control-set. Once the non-control data set is corrected, normal analytical procedures, such as ratio analysis, can then proceed with higher confidence. The net effect of the approach is to orient each data set into a third common coordinate space before comparing to one another. Thus, variations due strictly to disorientation between independent coordinate spaces are eliminated. The result is that remaining variation found in a comparative analysis is more likely to be the result of actual variation due strictly to the experimental conditions under study. However, the selection of which data set is independent and which is dependent for the purpose of regression analysis is arbitrary. It is this arbitrariness that is exploited by the method of bi-linear normalization described hereinbelow.

The process is referred to as "bi-linear normalization" because linear normalization is performed on the same data set under two spatial orientations. Each linear transform results in a relative change that orients the data set into a third external coordinate space. This new coordinate space allows both data representations to be examined on an equal basis, thereby eliminating variance strictly due to non-coincident spatial frameworks. It is the comparison of the resulting relative orientations within this common framework that enables anomalous behavior in the features to be revealed. In preferred embodiments, the bi-linear normalization is applied to microarray anomalous feature detection.

The reason that it is an arbitrary decision as to which data set is independent is because there is no standard to which to compare. A basic assumption of linear regression analysis is that there exists an independent and a dependent data set which are paired to one another, and that the independent data set has zero uncertainty, or is a "standard". In the situations to which the present methods are applied, this assumption is not true: either data set can act as the independent data set. Thus, either pairing situation is an arbitrary selection by the researcher. It is this arbitrariness that is exploited in the present method of bi-linear normalization, wherein in certain situations, the ratios of the paired normalized data values can be used to detect anomalies in the overall population of features. That is, since the relationship connecting the separated data is reciprocal, the product of the two ratios, one from each representation, should be equal to 1. Variation from 1 for any particular data member indicates anomalous behavior. The anomalies can then be excluded from the overall analysis or focused in upon for further study as to why they exhibit anomalous behavior.

As indicated hereinabove, utilization of RLS detection methods can involve various techniques of data processing, typically utilized for the purpose of providing such enhancements as greater signal to noise ratios, increased sensitivity, extended dynamic range, comparability within and/or between assays, and to identify particular features. These data processing techniques can be incorporated as sets of instructions embedded in software and/or hardware that is part of an analyzer and/or embedded in a computer with a data connection to an analyzer. Such software is typically recorded in an ordinary computer storage medium, including, but not limited to: volatile or non-volatile random access memory (RAM), read only memory (ROM), magnetic tape, hard or floppy disks, and optical storage media such as compact disks (CD, CD-ROM). Further assay methods, preferably array-based methods and especially microarray assay methods, can advantageously utilize such software and/or hardware.

The practice of the present invention is now illustrated by means of several examples. It would be understood by one of skill in the art that these examples are not in any way limiting in the scope of the present invention and merely illustrate representative embodiments.

EXAMPLES

Example 1: Linear normalization of microarray data.

Application of the linear normalization method is demonstrated using artificial data generated in a Microsoft Excel® Spreadsheet, shown in the following Table 1. Data sets X and Y are representative of microarray expression intensity values acquired from two experiments. The actual values are from a random number generator. The first nine rows at the top of the table represent replicate control spots. The replicates were used to calculate a best-fit least-squares linear regression line, as described hereinabove. The last 14 rows of Table 1 represent replicate experimental spots. Column 3 of Table 1 was generated by the resulting linear transform equation. Table 1 includes ratios obtained both before and after normalization, along with their associated percentage change.

Table 1.

| Data Set X | Data Set Y | Normalized Data Set Y' | Ratio (Y/X) | Normalized Ratio (Y'/X) | Change In Normalized Ratio |
|---|---|---|---|---|---|
| 68 | 81 | 107 | 1.19 | 1.58 | 24% |
| 54 | 70 | 88 | 1.03 | 1.29 | 20% |
| 62 | 32 | 22 | 0.47 | 0.32 | -47% |
| 44 | 6 | -24 | 0.09 | -0.35 | 125% |
| 79 | 65 | 79 | 0.96 | 1.17 | 18% |
| 19 | 75 | 97 | 1.10 | 1.42 | 22% |
| 73 | 69 | 86 | 1.01 | 1.27 | 20% |
| 37 | 26 | 11 | 0.38 | 0.17 | -130% |
| 18 | 12 | -13 | 0.18 | -0.19 | 191% |

(continued)

| Data Set X | Data Set Y | Normalized Data Set Y' | Ratio (Y/X) | Normalized Ratio (Y'/X) | Change In Normalized Ratio |
|---|---|---|---|---|---|
| 40 | 11 | -15 | 0.16 | -0.22 | 174% |
| 76 | 73 | 93 | 1.07 | 1.37 | 22% |
| 13 | 60 | 71 | 0.88 | 1.04 | 15% |
| 20 | 72 | 92 | 1.06 | 1.35 | 21% |
| 37 | 35 | 27 | 0.51 | 0.40 | -30% |
| 66 | 53 | 58 | 0.78 | 0.86 | 9% |
| 39 | 87 | 118 | 1.28 | 1.73 | 26% |
| 85 | 70 | 88 | 1.03 | 1.29 | 20% |
| 74 | 72 | 92 | 1.06 | 1.35 | 21% |
| 25 | 51 | 55 | 0.75 | 0.81 | 7% |
| 42 | 87 | 118 | 1.28 | 1.73 | 26% |
| 94 | 54 | 60 | 0.79 | 0.88 | 10% |
| 42 | 43 | 41 | 0.63 | 0.60 | -5% |
| 89 | 96 | 133 | 1.41 | 1.96 | 28% |

The linear equation obtained is represented by a slope of 0.5733 and a *y*-intercept of 19.5269. Accordingly, using equation (N9) hereinabove, the resulting linear transform equation is:

$$Y' = (Y - 19.5269) / 0.5733$$

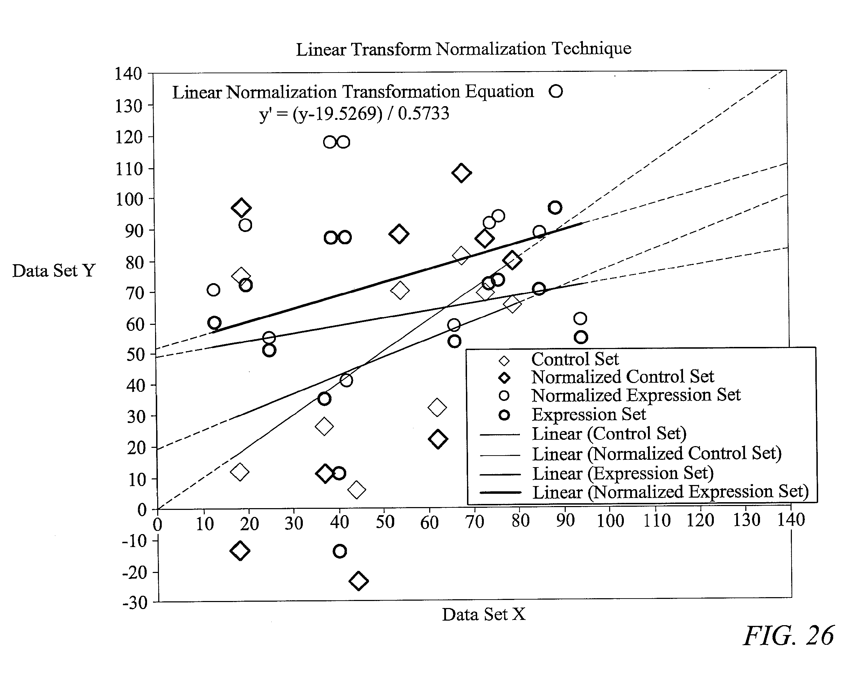

FIG. 26 shows the effect on data of applying the linear transform normalization method. The original replicate control spots have a regression line as described hereinabove. The transformed values of the same spots are shown having a regression line with slope equal to 1 and a *y*-intercept equal to 0, as derived. The balance of the data (*i.e.,* that which is not the controls) is shown before and after the normalization transform is applied. The variation between the resulting regression lines indicates the degree to which data values have been modified. Table 1 includes ratios of before and after normalization along with associated percentage change.

Example 2

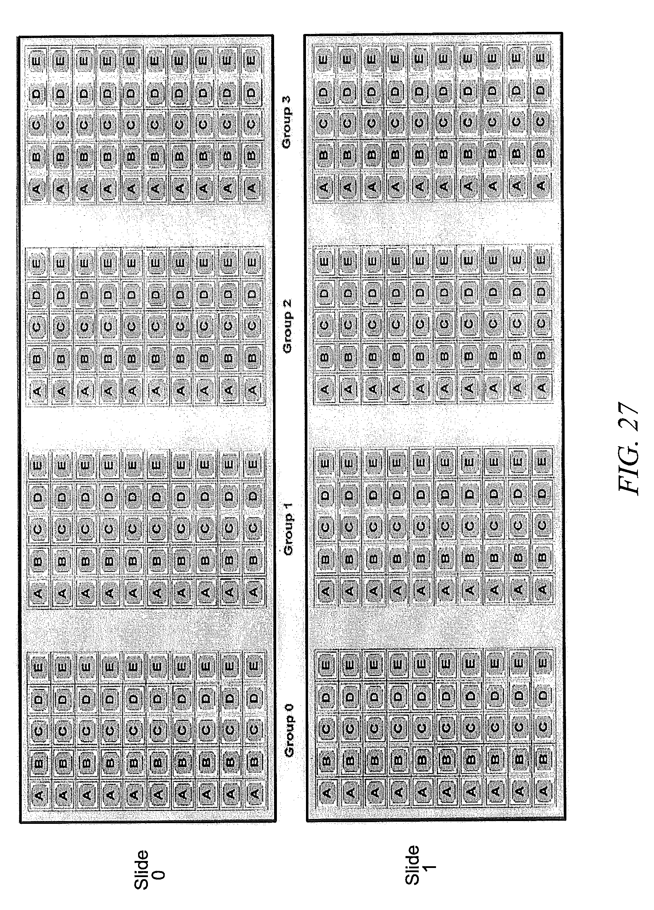

This example describes a study using microarray data from four slides: a set of two excellent-quality slides, referred to as "Ordered", and another set of two poor-quality slides, referred to as "Disordered". Each set of slides matches one another, feature-for-feature, and are expected to equally express across slides and within each slide. The slide layout depicted in FIG. 27 shows two slides, each having one array comprised of four sub-arrays. Each sub-array contains five classes of features replicated ten times in a column. The nomenclature used herein includes: Slides 0-1 (S0, S1); Groups 0-3 (G0, G1, G2, G3); and Features A-E. Each feature has an associated background value used in calculating an average background for each slide.

The purpose of analyzing two sets of data, Ordered and Disordered, is to demonstrate the degree to which this normalization technique affects high-variance data as compared to low-variance data. Normally, only high quality data should be used in an expression study, but determining excellence requires measures of quality assurance. The normalization process produces a statistical measure ($R^2$), which can help to determine the goodness of fit. Ultimately, a threshold at which quality assurance is compared, should preferably be determined so that a standard against which other fits can be measured is established.

The graphs associated with various microarray images are MTM density values of each group on slides 0 and 1, for both the Ordered and Disordered data sets. MTM density (the Median-based Trimmed Mean density) is the quantification value measured by ArrayVision, and is defined as follows.

The reported MTM Density (definition below) value for each spot represents the mean of all the pixels remaining in a defined region of interest, after first removing pixels with density values that exceed a user-defined threshold value median absolute deviations (MAD's) above or below the median. (By default, the threshold is 4.0 MAD's.) This measure removes the effect of image artifacts (*e.g.*, dust particles) on density estimation.

The objective of this study was to demonstrate the effect of applying the linear normalization technique to a realistic data set using several spatially related strategies. These strategies were investigated in parallel to explore the effect of using normalization to compensate for local (group-to-group) and global (slide-to-slide) fluctuations. Presently, the simplest of the spatial relationships (InterSlide) will be available in the Genicon Sciences' version of Imaging Research ArrayVision® software.

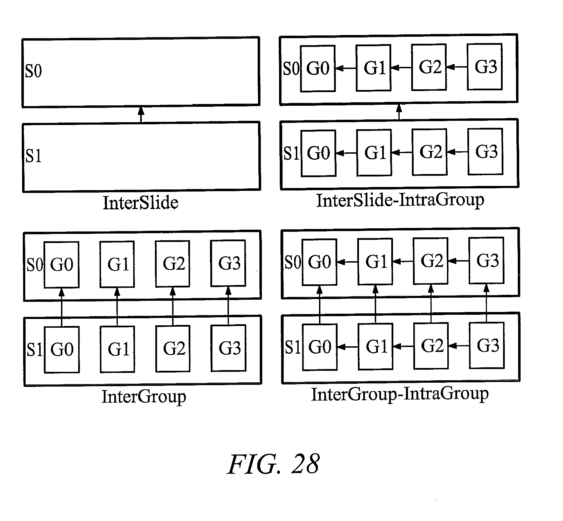

Four spatially related strategies for applying the linear normalization technique were implemented and are depicted in FIG. 28. The first strategy, referred to as "InterSlide," treats all of the data in slide S0 as a population set, paired with all the data in slide S1 as another population set. Three other general strategies can also be used to exploit spatial relationships that provide finer granularity. Fine-grained statistical analysis leads to improved accuracy by compensating for local effects. In order of complexity, the additional strategies are referred to respectively as "InterGroup", "InterSlide-IntraGroup", and "InterGroup-IntraGroup". The prefixes "Inter-" and "Intra-" refer to "Slide-to-Slide" and "Group-to-Group" relationships, respectively. To implement the InterSlide normalization strategy into software such as ArrayVision, modifications are expected to be minor when compared to those required to implement the alternative strategies.

Each of the normalization strategies is now briefly described with reference to FIG. 28.

*No Normalization*

Data from S0 and S1 are compared using no normalization. In one normalization approach, as suggested by Schuchhardt et al., Nucl. Acids Res., 28: e47, (2000), analysts will divide by a "normalization factor" based on the average of all features in a slide. However, such a term applied across the data set has no guarantee of normalizing the data set in an equalized, repeatable and consistent manner.

*InterSlide Linear Normalization*

All features from S0 are paired with corresponding features from S1. Regression analysis of the resulting data-pairs produces linear normalization parameters, subsequently used to correct the original data values from S1, thereby producing normalized InterSlide S1'. A comparative study is performed between S0 and S1'.

*InterGroup Linear Normalization*

All features from S0G0 are paired with corresponding features from S1G0. Regression analysis of the resulting data-pairs produces linear normalization parameters, subsequently used to correct the original data values from S1G0, thereby producing normalized InterGroup S1'G0. This InterGroup normalization process is repeated for each group to produce normalized InterGroups: S1'G0, S1'G1, S1'G2, and S1'G3. Comparative study is performed between the cumulative populations of S0G (made up of S0G0 + S0G1 + S0G2 + S0G3) and S1'G (made up of S1'G0 + S1'G1 + S1'G2 + S1'G3).

*InterSlide-IntraGroup Linear Normalization*

All features from S0G0 are paired with corresponding features from S0G1. Regression analysis of the resulting data-pairs produces linear normalization parameters, subsequently used to correct the original data values from S0G1, thereby producing normalized IntraGroup S0G1'. All features from S0G1' are paired with corresponding features from S0G2. Regression analysis of the resulting data-pairs produces linear normalization parameters, subsequently used to correct the original data values from S0G2, thereby producing normalized IntraGroup S0G2'. This IntraGroup normalization process is repeated for each Group to produce normalized IntraGroups: S0G0, S0G1', S0G2' and S0G3'. The entire IntraGroup normalization process is performed on Slide S1 in an equivalent manner, thereby producing normalized IntraGroups: S1G0, S1G1', S1G2' and S1G3':

Finally, InterSlide normalization is performed: all features from the cumulative populations of S0G' (*i.e.*, S0G0 + S0G1' + S0G2' + S0G3') are paired with the features from the corresponding cumulative populations of S1G' (*i.e.,* S1G0 + S1G1' + S1G2' + S 1 G3'). Regression analysis of the resulting data-pairs produces linear normalization parameters, subsequently used to correct the original data values from the cumulative populations of S1G', thereby producing a normalized InterSlide S1'G'. Comparative study is performed between the cumulative populations of S0G' and S1'G'.

*InterGroup-IntraGroup Linear Normalization*

All features from S0G0 are paired with corresponding features from S0G1. Regression analysis of the resulting data-pairs produces linear normalization parameters, subsequently used to correct the original data values from S0G1, thereby producing normalized IntraGroup S0G1'. All features from S0G1' are paired with corresponding features from S0G2. Regression analysis of the resulting data-pairs produces linear normalization parameters, subsequently used to correct the original data values from S0G2, thereby producing normalized IntraGroup S0G2'. This IntraGroup normalization process is repeated for each Group to produce normalized IntraGroups: S0G0, S0G1', S0G2' and S0G3'. The entire IntraGroup normalization process is performed on Slide S1 in an equal manner, thereby producing normalized IntraGroups: S1G0, S1G1', S1G2' and S1G3'.

Finally, InterGroup normalization is performed: all features from S0G0 are paired with corresponding features from S1G0. Regression analysis of the resulting data-pairs produces linear normalization parameters, subsequently used to correct the original data values from S1G0, thereby producing a normalized InterGroup S1'G0. All features from S0G1' are paired with corresponding features from S1G1'. Regression analysis of the resulting data-pairs produces linear normalization parameters, subsequently used to correct the original data values from S1G1', thereby producing a normalized InterGroup S1'G1'. This InterGroup normalization process is repeated for each Group to produce normalized InterGroups: S1'G0, S1'G1', S1'G2' and S1'G3'. Comparative study is performed between the cumulative populations of SOG' (*i.e.,* S0G0 + S0G1' + S0G2' + S0G3') and S1G' (*i.e.*, S1'G0 + S1'G1' + S1'G2' + S1'G3').

In alternative embodiments, intra-slide groups are created as a function of the particular pin used for spotting the group. For example, reference features used in the normalization process are preferably grouped so that sub-groups of features on the slide can be related back to the pin used in spotting. This allows sub-populations to be normalized to minimize pin-to-pin variability, which can be a significant source of spotting variability. In still other embodiments, reference features can be distributed so that temporal or position dependence variability during array printing can be corrected for.

The linear transform normalization algorithm is the same in each strategy, only the granularity of the problem setup changes. By granularity is meant the fact that the data can be expressed as a hierarchy of classes, from groups of slides, to individual slides, to groups within slides. Exploiting granularity is an approach to statistical analysis which subtly captures inherent relationships that can be missed (averaged out) when the data is included in a larger population. Of course, to take advantage of such inherent relationships means there has to be structure to the data. A microarray has structure, but is preferably specified and translated into a form suitable for use by the algorithm. It should be noted that the amount of computer processing increases as the granularity becomes finer. However, with the present state of desktop computing power, this is not an immediate concern.

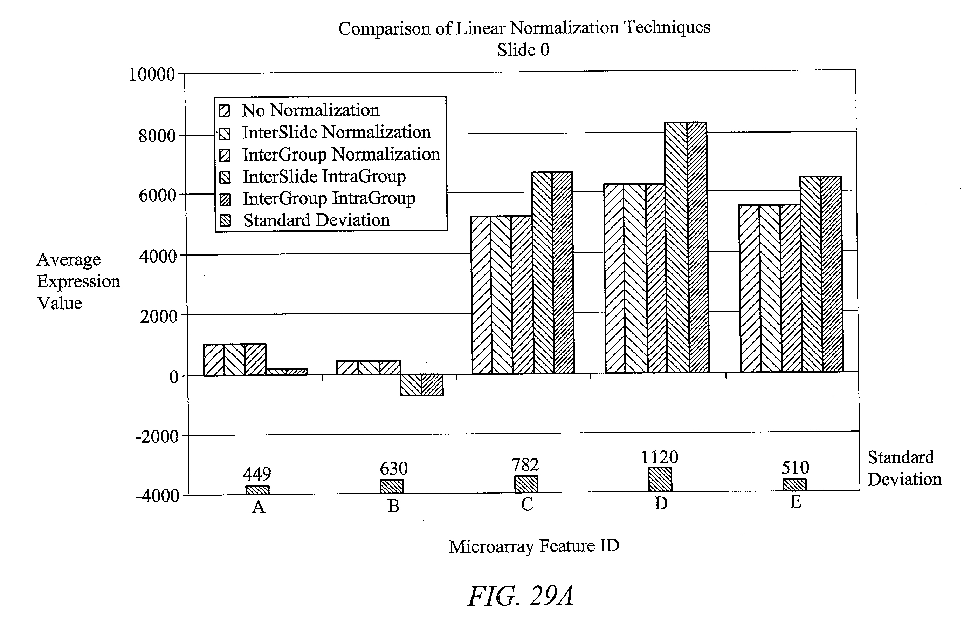

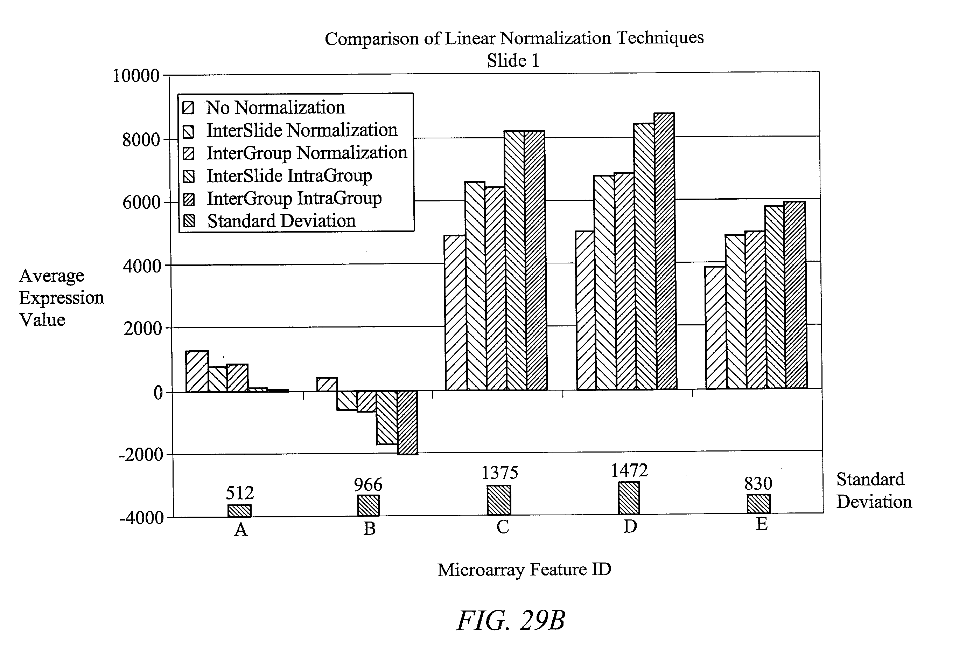

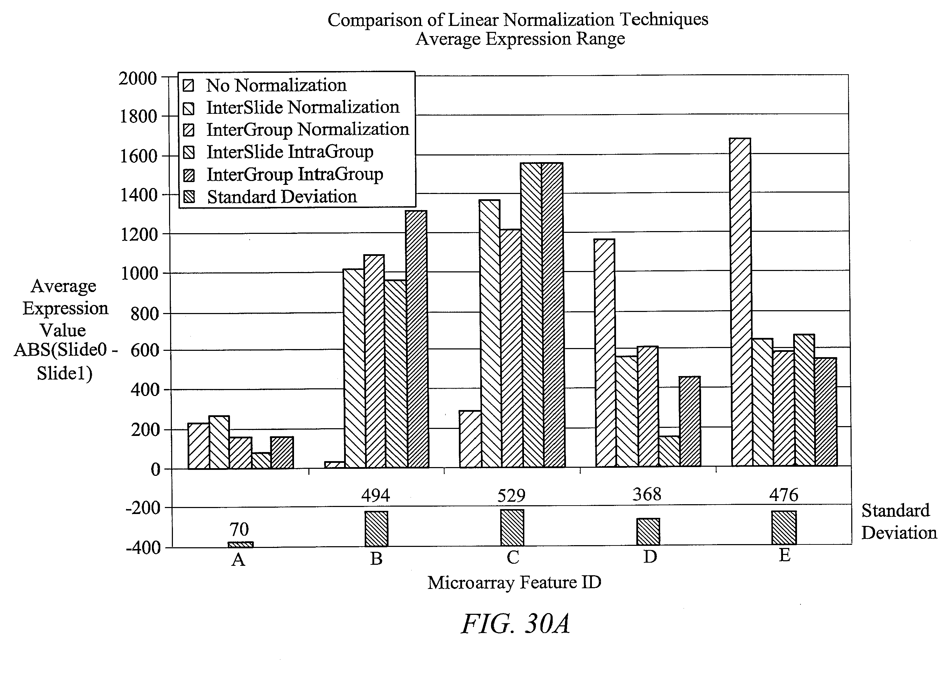

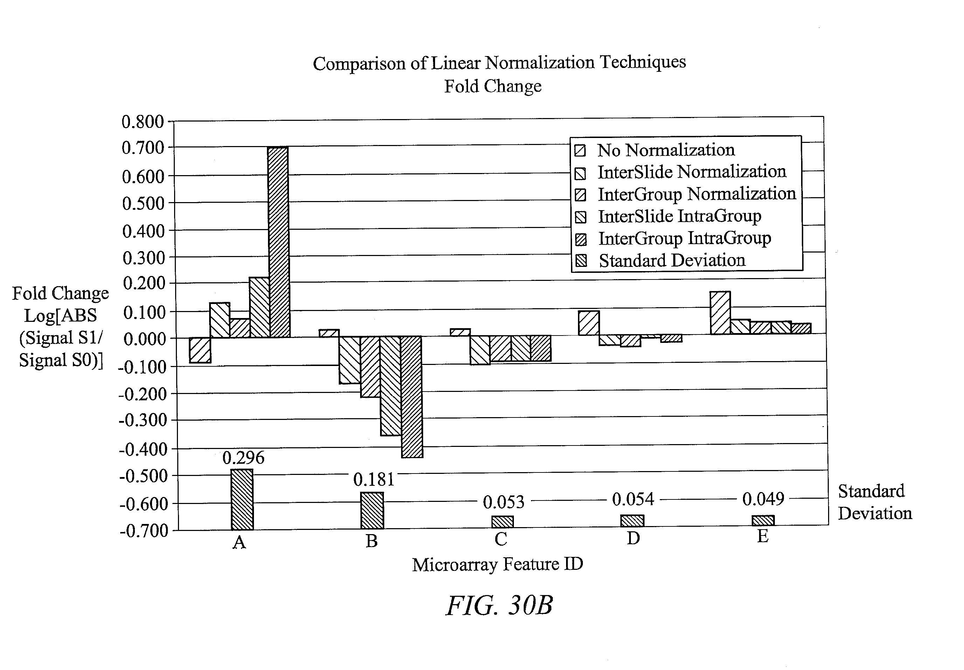

FIGs 29 and 30 show a side-by-side performance comparison between the five normalization strategies for the Disordered data set. As shown in FIGs 29A and 29B, the average expression values for each class vary according to normalization strategy. The standard deviation amongst techniques is included below each comparative set for each feature. Slide 0 shows an average standard deviation of 698 across normalization strategies, while Slide 1 shows 1,031. This implies that Slide 1 is affected more by the method of normalization than is Slide 0. The magnitude of the deviation is high due to the already established high variance associated with the Disordered data set. Once linear normalization is complete, comparative expression analysis is performed resulting in Figures 30A and 30B.

FIG. 30A shows the average expression range between Slide 0 and Slide 1, indicating the amount of new signal that is contributed to measured density values as a result of normalization. Ideally, in this study, the average expression across slides should be equal and therefore the difference should be zero. It is interesting to note that for feature A, normalization is in agreement; for features B and C, the normalization caused an increase in range; and for features D and E, the normalization caused a decrease in range, relative to no normalization. This behavior is not unusual, and was noted in the discussion hereinabove on data distribution shifts and class separation. The average standard deviation of average expression ranges across normalization strategies is 387. This indicates that normalization has a nominal contribution to measured density values. Comparative expression in terms of ratios is shown in FIG. 30B. Similarly, the expected performance of the study should be that relative expression is one-to-one which produces a fold change whose log is equal to zero. FIG. 30B represents the log fold change having an average standard deviation of 0.1266 fold across normalization techniques. Thus, comparative analysis of the Disordered data set consistently shows high levels of variance throughout the analysis, as expected from the onset. The next section repeats this entire process of analysis based upon the Ordered data set.

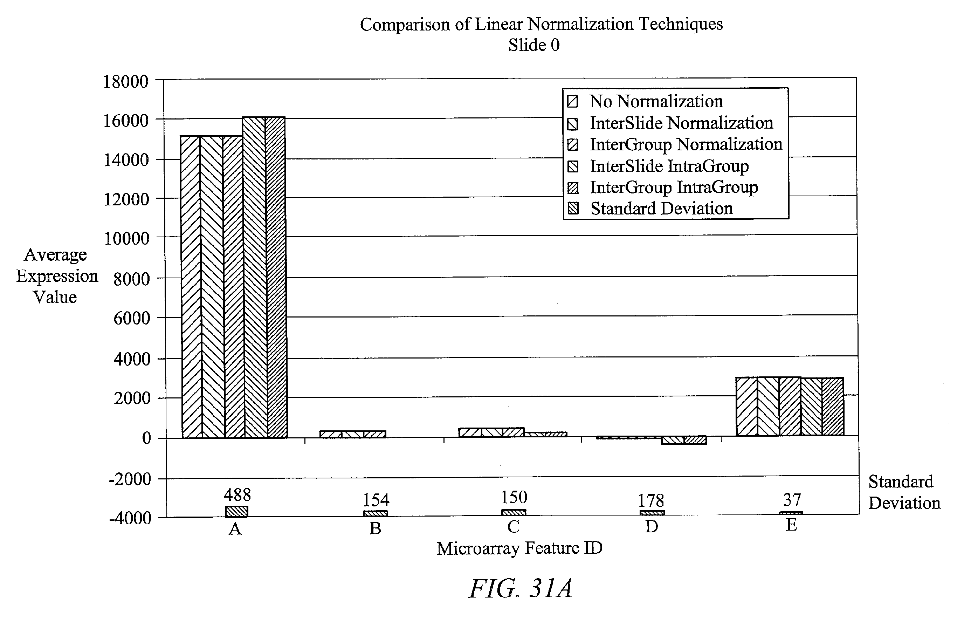

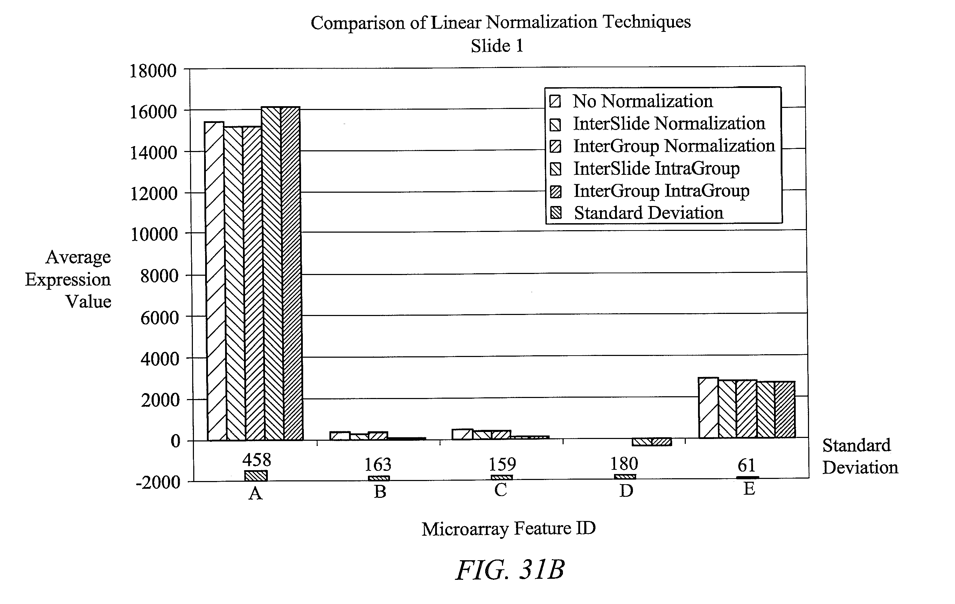

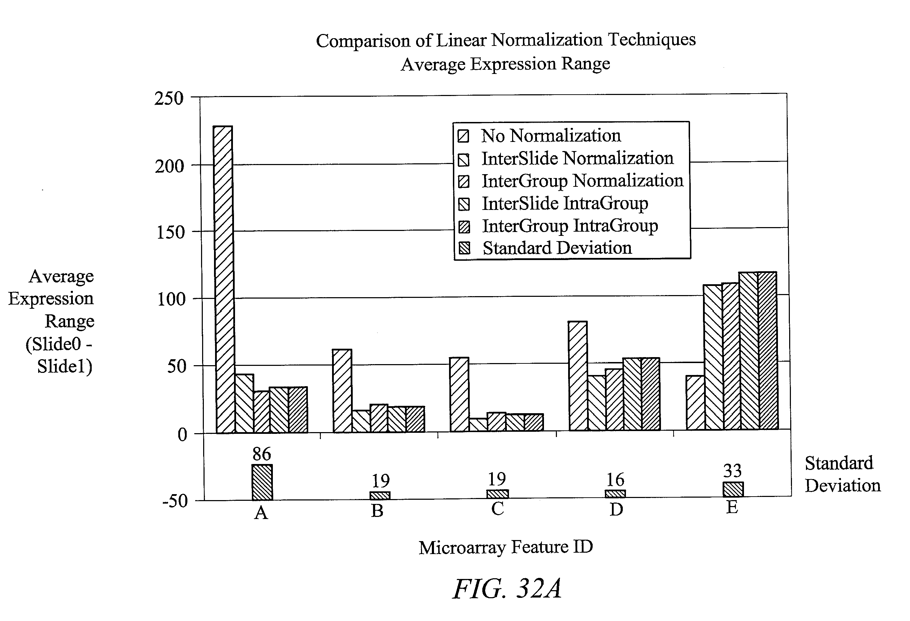

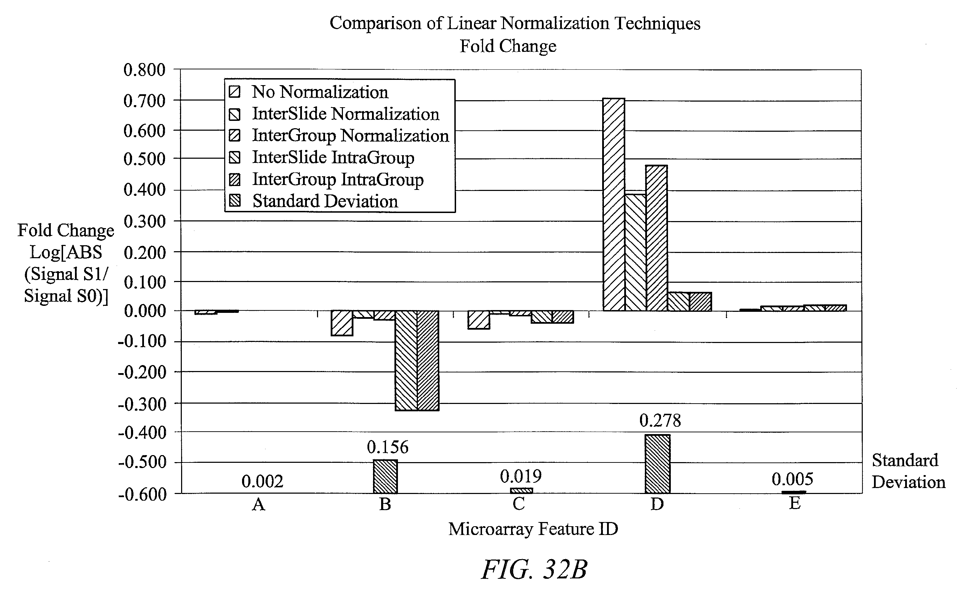

FIGs 31 and 32 show a side-by-side performance comparison between the five normalization strategies for the Ordered data set. As shown in FIGs 31A and 31B, the average expression values for each class vary according to strategy. The standard deviation amongst techniques is included below each comparative set for each feature. Slide 0 shows an average standard deviation of 201 across normalization strategies, while Slide 1 is 204. This implies that Slide 0 and Slide 1 are equally affected by normalization. The magnitude of the deviation is low due to the already established low variance associated with the "Ordered" data set. Once linear normalization is complete, comparative expression analysis is performed resulting in FIGs 32A and 32B.

FIG. 32A shows the average expression range between Slide 0 and Slide 1, indicating the amount of new signal that is contributed to measured density values as a result of normalization. Ideally, in this study, the average expression across slides should be equal and therefore the difference should be zero. It is interesting to note that all of the normalization techniques are in close proximity of one another, but differ significantly from no normalization. The average standard deviation of average expression ranges across normalization strategies is 35. This indicates that normalization made an insignificant contribution to measured density values. Is this true, if so, why do it?

Comparative expression in terms of ratios is shown in FIG. 32B. Similarly, the expected performance of the study should be that relative expression is one-to-one which produces a fold change whose log is equal to zero. FIG. 30B represents the fold change whose log has an average standard deviation of 0.0921 fold across normalization methods. This number is somewhat significant compared to the expected value of zero. Upon closer inspection, one can observe that microarray features A, C and E are very close to 0, while features B and D are not. Thus, this analysis would lead an observer to suspect the quality of expression analysis involving this class of features. It is also interesting to note that in suspect features B and D, the Intra-Group based strategies of normalization gave rise to a result that is contrary to the others. Finally, comparative analysis of the Ordered data set consistently shows low levels of variance throughout the analysis, as expected from the onset.

Example 3: Bi-linear Normalization.

Table B1 shows data sets "a" and "b" for each microarray feature C1 through C25.

Table B 1

| Feature ID | a | b |
|---|---|---|
| C1 | 5447 | 1335 |
| C2 | 7753 | 3783 |
| C3 | 4995 | 2935 |
| C4 | 3953 | 1913 |
| C5 | 5015 | 328 |
| C6 | 7830 | 6077 |
| C7 | 7645 | 3700 |
| C8 | 8362 | 2295 |
| C9 | 5837 | 1264 |
| C10 | 4492 | 3823 |
| C11 | 5773 | 4851 |
| C12 | 9435 | 4318 |
| C13 | 8576 | 2956 |
| C14 | 6270 | 1617 |
| C15 | 7223 | 2643 |
| C16 | 9421 | 7261 |
| C17 | 3683 | 508 |
| C18 | 7661 | 4862 |
| C19 | 2994 | 59 |
| C20 | 6190 | 1119 |
| C21 | 7899 | 3121 |
| C22 | 6772 | 4486 |
| C23 | 3933 | 105 |
| C24 | 914 | 175 |

(continued)

| Feature ID | a | b |
|---|---|---|
| C25 | 5636 | 3977 |

Data sets "a" and "b" are assigned to be the independent and dependent data sets, respectively, and are each normalized and then converted to ratios. Then the data sets reverse roles and the process is repeated. These two results are depicted in Tables B2 and B3.

Table B2

| | Control | Data | Normalized | Normalized Ratio |
|---|---|---|---|---|
| **Feature ID** | **S0** | **S1** | **S1'** | **[S0/S1']** |
| C1 | 5447 | 1335 | 3910 | 1.39 |
| C2 | 7753 | 3783 | 7701 | 1.01 |
| C3 | 4995 | 2935 | 6388 | 0.78 |
| C4 | 3953 | 1913 | 4805 | 0.82 |
| C5 | 5015 | 328 | 2350 | 2.13 |
| C6 | 7830 | 6077 | 11254 | 0.70 |
| C7 | 7645 | 3700 | 7573 | 1.01 |
| C8 | 8362 | 2295 | 5396 | 1.55 |
| C9 | 5837 | 1264 | 3800 | 1.54 |
| C10 | 4492 | 3823 | 7763 | 0.58 |
| C11 | 5773 | 4851 | 9355 | 0.62 |
| C12 | 9435 | 4318 | 8530 | 1.11 |
| C13 | 8576 | 2956 | 6420 | 1.34 |
| C14 | 6270 | 1617 | 4346 | 1.44 |
| C15 | 7223 | 2643 | 5935 | 1.22 |
| C16 | 9421 | 7261 | 13088 | 0.72 |
| C17 | 3683 | 508 | 2629 | 1.40 |
| C18 | 7661 | 4862 | 9372 | 0.82 |
| C19 | 2994 | 59 | 1933 | 1.55 |
| C20 | 6190 | 1119 | 3575 | 1.73 |
| C21 | 7899 | 3121 | 6676 | 1.18 |
| C22 | 6772 | 4486 | 8790 | 0.77 |
| C23 | 3933 | 105 | 2004 | 1.96 |
| C24 | 914 | 175 | 2113 | 0.43 |
| C25 | 5636 | 3977 | 8002 | 0.70 |

Table B3

| | Control | Data | Normalized | Normalized Ratio |
|---|---|---|---|---|
| **Feature ID** | **S0** | **S1** | **S1'** | **[S0/S1']** |
| C1 | 1335 | 5447 | 1847 | 0.72 |
| C2 | 3783 | 7753 | 4917 | 0.77 |
| C3 | 2935 | 4995 | 1245 | 2.36 |
| C4 | 1913 | 3953 | -142 | -13.47 |
| C5 | 328 | 5015 | 1272 | 0.26 |
| C6 | 6077 | 7830 | 5019 | 1.21 |
| C7 | 3700 | 7645 | 4773 | 0.78 |
| C8 | 2295 | 8362 | 5727 | 0.40 |
| C9 | 1264 | 5837 | 2366 | 0.53 |

(continued)

| Feature ID | Control | Data | Normalized | Normalized Ratio |
|---|---|---|---|---|
| | S0 | S1 | S1' | [S0/S1'] |
| C10 | 3823 | 4492 | 576 | 6.64 |
| C11 | 4851 | 5773 | 2281 | 2.13 |
| C12 | 4318 | 9435 | 7156 | 0.60 |
| C13 | 2956 | 8576 | 6012 | 0.49 |
| C14 | 1617 | 6270 | 2942 | 0.55 |
| C15 | 2643 | 7223 | 4211 | 0.63 |
| C16 | 7261 | 9421 | 7137 | 1.02 |
| C17 | 508 | 3683 | -501 | -1.01 |
| C18 | 4862 | 7661 | 4794 | 1.01 |
| C19 | 59 | 2994 | -1419 | -0.04 |
| C20 | 1119 | 6190 | 2836 | 0.39 |
| C21 | 3121 | 7899 | 5111 | 0.61 |
| C22 | 4486 | 6772 | 3611 | 1.24 |
| C23 | 105 | 3933 | -169 | -0.62 |
| C24 | 175 | 914 | -4187 | -0.04 |
| C25 | 3977 | 5636 | 2098 | 1.90 |

Table B4

| Feature ID | Product of Normalized Ratios (Ideal =1) |
|---|---|
| | [S0/S1']*[S0/S1'] |
| C1 | 1.01 |
| C2 | 0.77 |
| C3 | 1.84 |
| C4 | 11.08 |
| C5 | 0.55 |
| C6 | 0.84 |
| C7 | 0.78 |
| C8 | 0.62 |
| C9 | 0.82 |
| C10 | 3.84 |
| C11 | 1.31 |
| C12 | 0.67 |
| C13 | 0.66 |
| C14 | 0.79 |
| C15 | 0.76 |
| C16 | 0.73 |
| C17 | 1.42 |
| C18 | 0.83 |
| C19 | 0.06 |
| C20 | 0.68 |
| C21 | 0.72 |
| C22 | 0.96 |
| C23 | 1.22 |
| C24 | 0.02 |
| C25 | 1.33 |

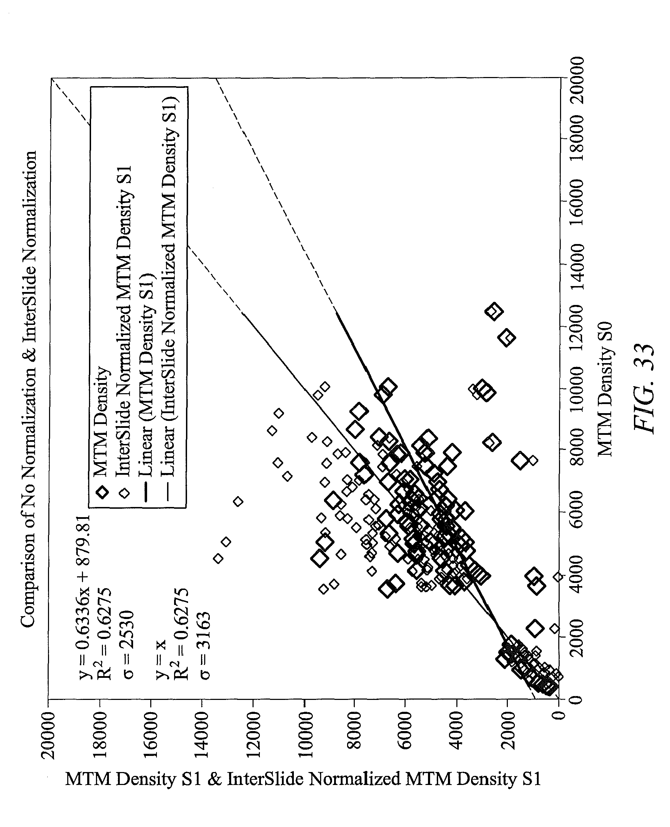

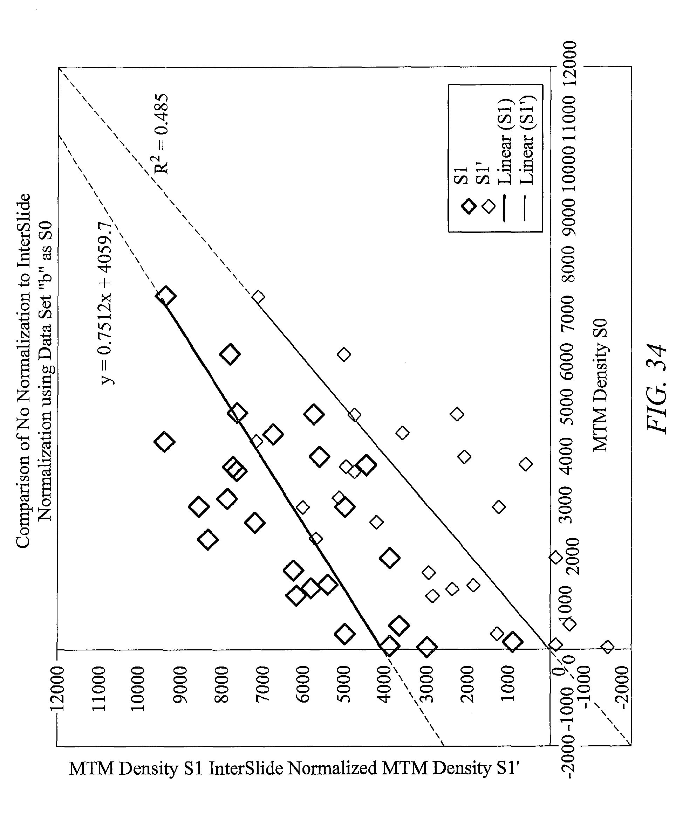

Prior to calculating ratios, linear normalization is performed for which the results are also shown in Tables B2 and B3. FIGs 33 and 34 show the effects of linear normalization on the data set by showing results before and after linear normalization, including the best-fit linear regression line. The linear transform applied to the dependent data values is shown in each figure. The result of applying the transform is also shown, resulting in a new regression line having a slope of 1 and an intercept of 0. There is no change in the R-squared value in either case. Using these two valid relationships, it is clear that their association with each other is reciprocal in nature. That is, the ratios of a/b' and b/a' when multiplied together should be equal to 1:

$$\frac{a}{b'} * \frac{b}{a'} = 1 \qquad (B1)$$

when *a' = a,* and *b' = b.* However, linear normalization shows that a' is not equal to *a,* and *b'* is not equal to *b*, due to contributions from experimental effects which are effectively expressed in individual features. To determine the amount that an experimental error or other effects contribute, on a feature by feature basis, a comparison of the resulting product of normalized ratios can be made.

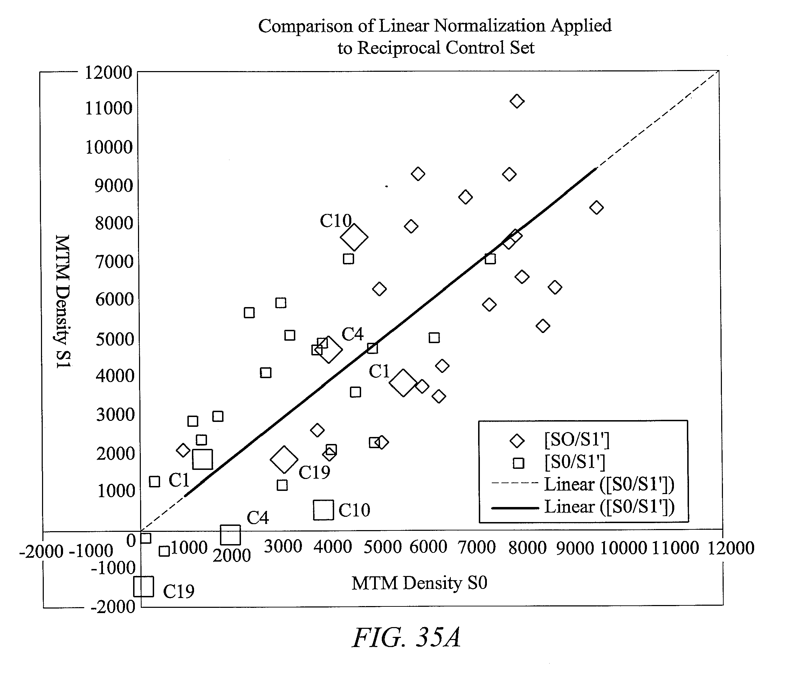

FIG. 35A shows both normalized relationships with data sets showing highlighted features from Table B2. The important concept in this figure is to observe that the regression line for each data set is the same as the other. This condition is the principal concept behind linear normalization which, once met, enables each data set to be compared on equal terms. Without normalization, the reciprocal relationship is exact, *i.e., a/b* and *b/a* when multiplied together are always exactly equal to 1, equation (B2):

$$\frac{a}{b} * \frac{b}{a} = 1 \qquad (B2)$$

Thus, equation (B2) represents the expected relationship between the two data, while equation (B1) represents the actual result. Hence, the contrast between the two can be used as an indication of how well the actual results achieve the desired results. This offers an opportunity to exploit the process to detect features, on an individual basis, that fail to meet the criteria within a given certainty range. Therefore, this approach can act as a quality control for determining the inclusion criteria for individual features in an experimental or comparative analysis.

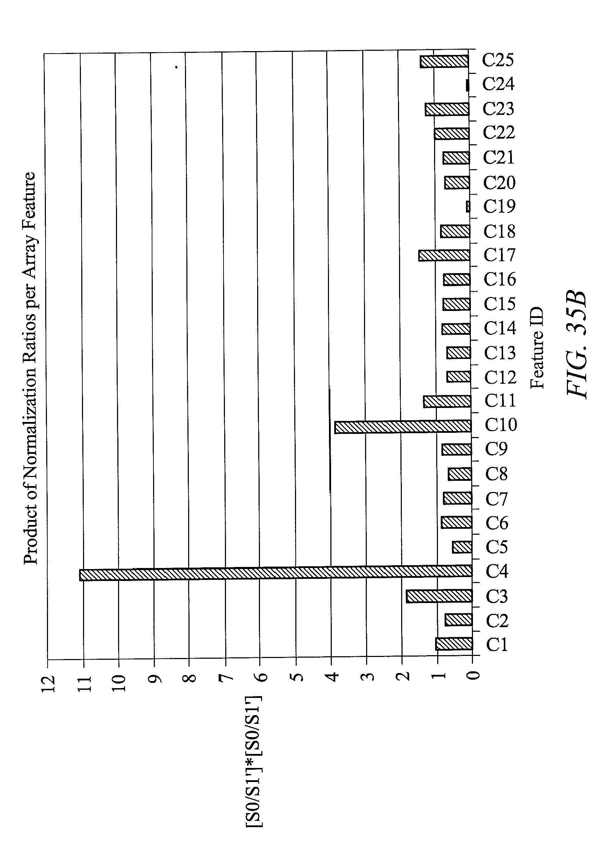

The corresponding bar chart in FIG. 35B shows how the features C1 through C25 perform with this technique. Notice those features which significantly differ from the ideal value of 1. Those are the anomalous features and should be scrutinized by an operator for possible exclusion from analysis and studied further for determining possible causes of experimental error.

Example 4: Application of Two-Color Software to the Reduction of Background Signal in a One Color Protocol.

The purpose of this example was to demonstrate that the Genicon Sciences two-color software was capable of eliminating background signal, generated by light scattered from dust and debris, from microarray slides assayed with the Genicon Sciences one-color protocol.

The following Materials were used: GSD 501 Scanner, equipped with software and filters for two-color analysis; 8 filters (450x70, 480x55, 550x60, 565x70, 580x70, 600x70, 620x70, 850x40); Au Dilution Series Slide (Bar Code #3750; NBN 1128:95); and Genicon Separator Software Tool (Research Systems Inc.).

A single dilution series slide, assayed with the one-color protocol, was imaged with the GSD 501 scanner using eight different filters, all at exposures of one second. The images were analyzed to determine characteristic spectra for Au and dust particles. The images produced by 450, 480, and 600 nm scans were combined in the Genicon Separator software tool. Regions of interest for Au and dust were selected, and end member reference files were created for each particle type. Output intensity images for Au and dust were generated using the software tool.

*Spectral Data of Au and Dust Particles*

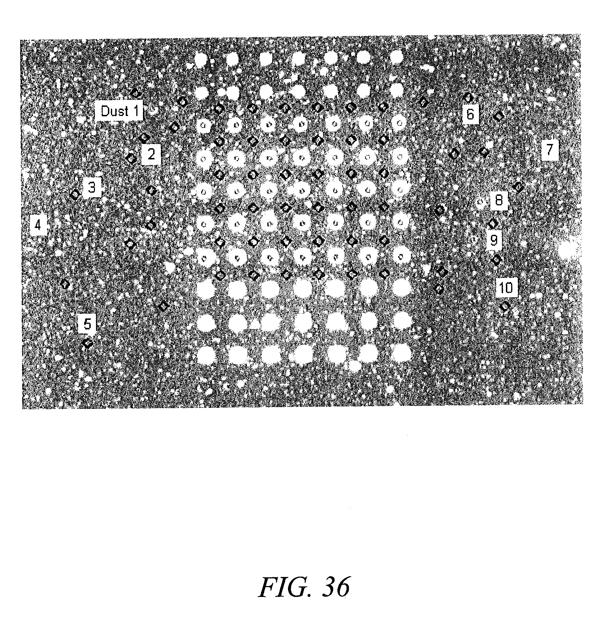

A single slide with Au features and typical dust background was scanned at one-second exposures for all eight wavelengths listed above. FIG. 36 is an image of the "features" selected in the ArrayVision quantification analysis. In particular, FIG. 36 shows spectral data of Au and dust particles. Data was sampled under single template mode to ensure that all intensity values correspond to the same pixel regions of each image (wavelength). Segmentation, as found in the ArrayVision was not applied in this case. Several Au concentrations of gold were sampled, as were dust spots of varying intensity so that a range of gold particles and dust species could be analyzed. The median intensity (MTM Density) of each feature was taken for all wavelengths and a characteristic spectrum was determined for each particle type. The data are presented in Table E-1.

Table E-1. Feature Spectrum Data (MTM Density vs. Filter Band(nm))

| Spot ID | 450X70 | 480X55 | 550X60 | 565X70 | 580X70 | 600X70 | 620X55 | 850X40 |
|---|---|---|---|---|---|---|---|---|
| 2ng/$\mu$l | 921 | 717 | 4103 | 8915 | 8271 | 8510 | 6077 | 218 |
| 5ng/$\mu$l | 1807 | 1365 | 8715 | 19208 | 17807 | 18425 | 13156 | 382 |
| 8ngl$\mu$l | 1677 | 1271 | 8036 | 17982 | 16772 | 17294 | 12360 | 314 |
| 10ng/$\mu$l | 2063 | 1537 | 9899 | 22396 | 21028 | 21858 | 15682 | 386 |
| 20ng/$\mu$l | 3031 | 2270 | 15999 | 36459 | 34655 | 35636 | 25242 | 558 |
| Dust1 | 2265 | 1735 | 1468 | 3942 | 3280 | 3456 | 2612 | 273 |
| Dust2 | 435 | 342 | 302 | 531 | 491 | 502 | 378 | 112 |
| Dust3 | 738 | 604 | 761 | 1227 | 993 | 978 | 765 | 130 |
| Dust4 | 5303 | 3508 | 2824 | 5181 | 4474 | 5044 | 3974 | 1388 |
| Dust5 | 480 | 369 | 503 | 741 | 590 | 578 | 427 | 133 |
| Dust6 | 1297 | 947 | 1077 | 1788 | 1461 | 1498 | 1134 | 188 |
| Dust7 | 1244 | 831 | 1168 | 1606 | 1127 | 1154 | 936 | 205 |
| Dust8 | 20981 | 14686 | 15941 | 28474 | 23524 | 23913 | 17666 | 2413 |
| Dust9 | 2486 | 1951 | 1926 | 3046 | 2193 | 2324 | 2456 | 289 |
| Dust10 | 534 | 398 | 592 | 985 | 851 | 863 | 684 | 122 |

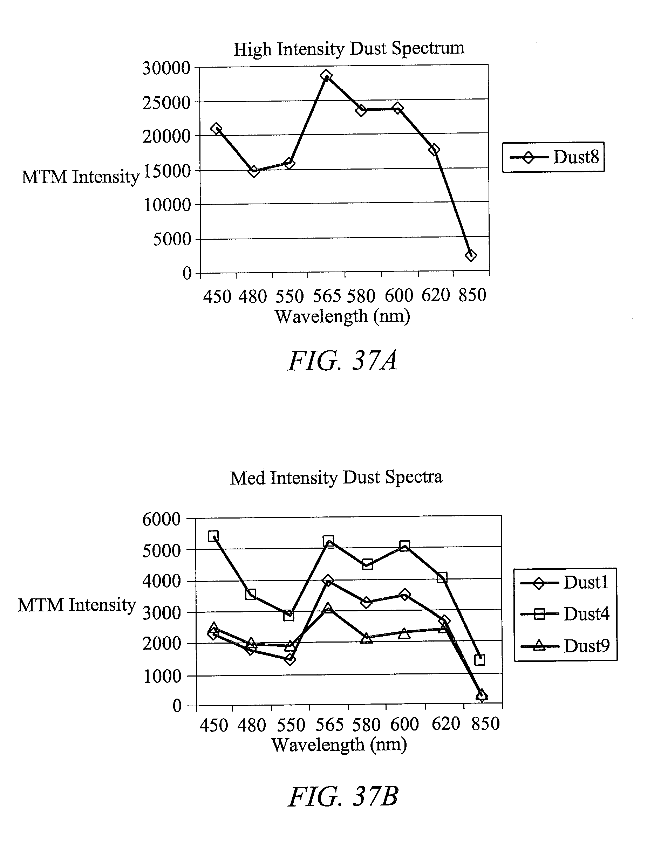

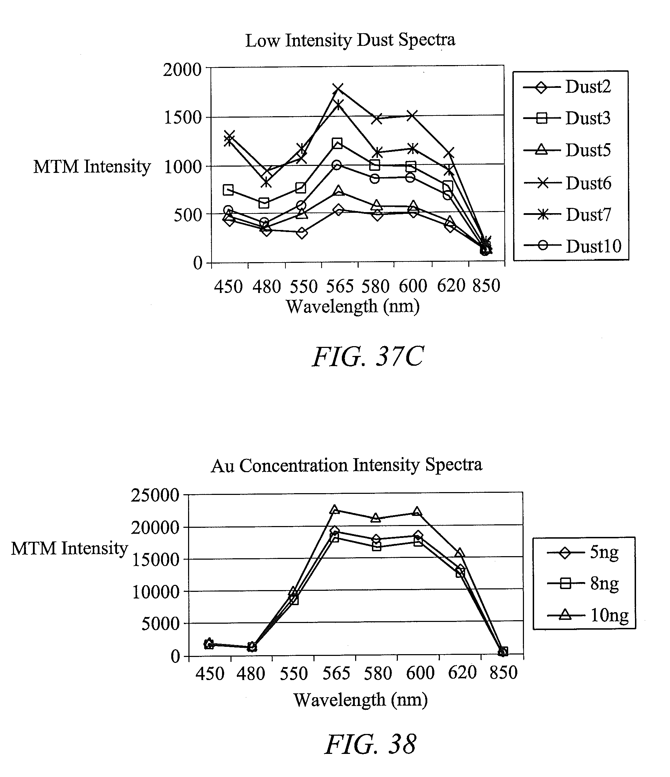

Dust particle data were separated into three regions: high, medium, and low intensity. All particle intensities, qualitatively, possessed similar spectral trends. Of particular interest was the signal increase at 450nm for all intensity groups. See FIGs 37A, B and C.

The spectra of Au features of various concentrations are shown in FIG. 38. The signal is noticeably deficient near 450nm.

*Separation Method and Parameters.*

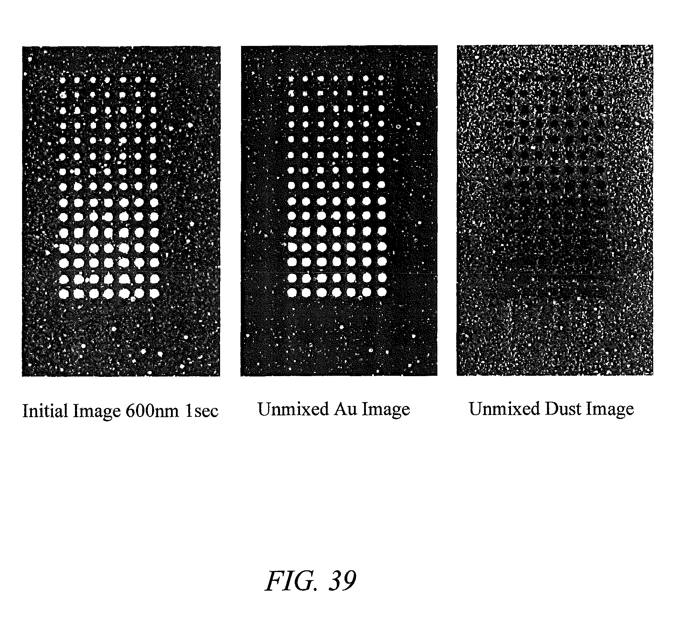

Based on the data hereinabove, the 450, 480, and 600 nm filter images were selected for use and combined in the Genicon separator. A gold endmember file was created sampling only the 8 and 2ng/$\mu$l features (600 nm predominant band). Dust particles of varying size and intensity were also sampled. Although unknown, dust concentration values are a required parameter to create the dust endmember file (450 nm predominant band). Therefore the concentrations were approximated by dividing the mean 450nm-dust-ROI-intensities by the mean Au-8ng-600nm-intensities (all concentrations normalized). Unmixed output intensity Au and dust images were created from Au and dust endmember files; see FIG. 39.

FIG. 39 clearly demonstrates the software's potential to remove background dust from raw images. The unmixed Au image was a definite visual improvement over the original image. Additionally, the unmixed dust image possessed areas where the software has successfully identified signal generated by the gold particles. Table E-2 shows that the de-convolution process did not adversely affect the specific Au signals generated by the output unmix file. Lower concentration features were reduced more greatly than higher concentrations. This is expected since each feature will contain some dust and the removal of that dust will decrease a greater percentage of overall signal in lower concentration features.

Table E-2. Au Feature Data Before and After Separation

| Concentration (ng/μl) | Original MTM Density | Separated MTM Density | Separated/Original Ratio |
|---|---|---|---|
| 0.05 | 432 | 349 | 0.81 |
| 0.08 | 319 | 247 | 0.77 |
| 0.1 | 726 | 623 | 0.86 |
| 0.2 | 909 | 788 | 0.87 |
| 0.5 | 1547 | 1387 | 0.90 |
| 0.8 | 1543 | 1390 | 0.90 |
| 1 | 1896 | 1723 | 0.91 |
| 2 | 7339 | 6915 | 0.94 |
| 5 | 18375 | 18448 | 1.00 |
| 8 | 15432 | 15570 | 1.01 |
| 10 | 19029 | 19279 | 1.01 |
| 20 | 33037 | 33740 | 1.02 |
| 30 | 33705 | 34402 | 1.02 |
| 40 | 28123 | 28752 | 1.02 |
| 50 | 33769 | 34311 | 1.02 |

In summary, the two-color software can identify and separate background dust particles, improving the overall quality of RLS microarray images. The example presented herein shows that it is possible to successfully remove a significant amount of background dust without significantly compromising feature intensity. The success of the method arises because the signal to background ratio is the primary quantity, so that a small drop in signal is offset by a large drop in background signal. Additional parameters that can be adjusted include selection of filter, filter exposure times, dust concentration algorithms, as well as selecting more diverse gold particle, and dust feature sample populations.

The method of filtering out the effects of dust particles can also be applied to bubbles, scratches or other light scattering artifacts with distinguishable spectral properties. It may also be applied to other methods of signal generation and detection including fluorescence labels, and in other assay formats including cellular assays, immunohistochemical systems, *in situ* hybridization and similar approaches.

Example 5: Quantitative Theory for Multiplexing.

To test the two color multiplexed system a model experimental system consisting of mixtures of 60 nm silver and 80 nm gold particles was used. The concentrations of silver and gold particles in these mixtures were determined from scattered light intensity measurements at two different wavelengths, $\lambda_1$ and $\lambda_2$. The validity of the concentrations evaluated in this manner is assessed by comparison with the known concentrations of the particles in the different mixtures.

To determine the concentration of silver and gold particles in the two color multiplexed assays, the sample was illuminated with white light. The scattered light intensity $I(\lambda_1)$ at the wavelength $\lambda_1$ which is optimal for detection of 60 nm silver particles, and the scattered light intensity $I(\lambda_2)$ which is optimal for detection of 80 nm gold particles, were both measured. In addition the following intensities were measured:

$I(\lambda_1)$, $C_{ref,Ag}$) and $I(\lambda_2, C_{ref,Ag})$, the scattered light intensity, at wavelengths $\lambda_1$ and $\lambda_2$ respectively, for a suspension containing 60 nm silver particles at a reference concentration $C_{ref,Ag}$.

$I(\lambda_1, C_{ref,Au})$ and $I(\lambda_2, C_{ref,Au})$, the scattered light intensity, at wavelengths $\lambda_1$ and $\lambda_2$ respectively, for a suspension containing 80 nm gold particles at a reference concentration $C_{ref,Au}$.

These intensities serve to calibrate the system by establishing relations between scattered light intensity and particle concentrations. They can also be used to evaluate the extent of particle overlap.

The principal mathematical relations between scattered light intensities and particle concentrations (assuming a linear relation between particle concentration and scattered light intensity) are as follows:

$$I(\lambda_1) = a_{Ag} I(\lambda_1, C_{ref,Ag}) + a_{Au} I(\lambda_1 C_{ref,Au}) \qquad (M1)$$

$$I(\lambda_2) = a_{Ag} I(\lambda_2, C_{ref,Ag}) + a_{Au} I(\lambda_2 C_{ref,Au}) \qquad (M2)$$

Rearranging equations (M1) and (M2) to solve for the coefficients, $\alpha_{Ag}$ and $\alpha_{Au}$, gives:

$$a_{Ag} = \frac{I(\lambda_1) I(\lambda_2, C_{ref,Au}) - I(\lambda_2) I(\lambda_1, C_{ref,Au})}{I(\lambda_1, C_{ref,Ag}) I(\lambda_2, C_{ref,Au}) - I(\lambda_1, C_{ref,Au}) I(\lambda_2, C_{ref,Ag})} \qquad (M3)$$

$$a_{Au} = \frac{I(\lambda_2) I(\lambda_1, C_{ref,Ag}) - I(\lambda_1) I(\lambda_2, C_{ref,Ag})}{I(\lambda_1, C_{ref,Ag}) I(\lambda_2, C_{ref,Au}) - I(\lambda_1, C_{ref,Au}) I(\lambda_2, C_{ref,Ag})} \qquad (M4)$$

and:

$$a_{Ag} = \frac{C_{Ag}}{C_{ref,Ag}} \qquad (M5)$$

$$a_{Au} = \frac{C_{Au}}{C_{ref,Au}} \qquad (M6)$$

From these equations, the concentrations of gold and silver particles, $C_{Au}$ and $C_{Ag}$ respectively, in other samples can be determined.

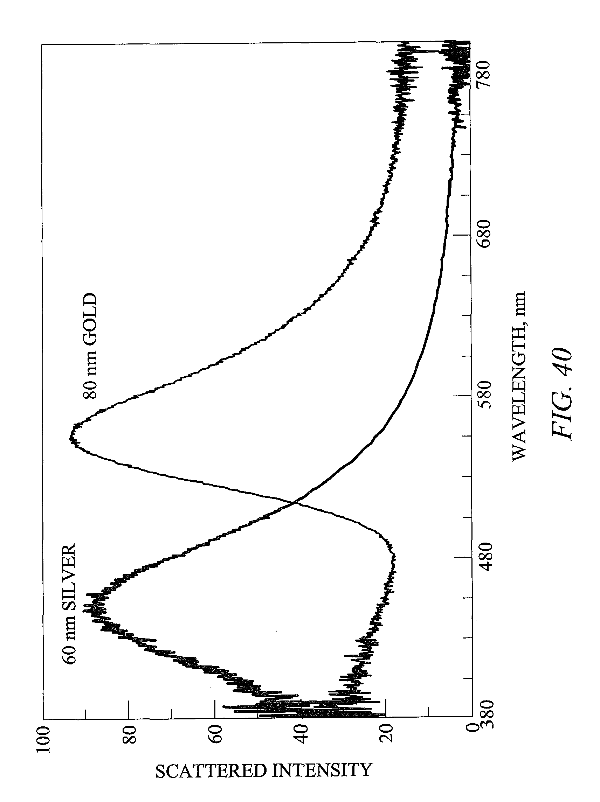

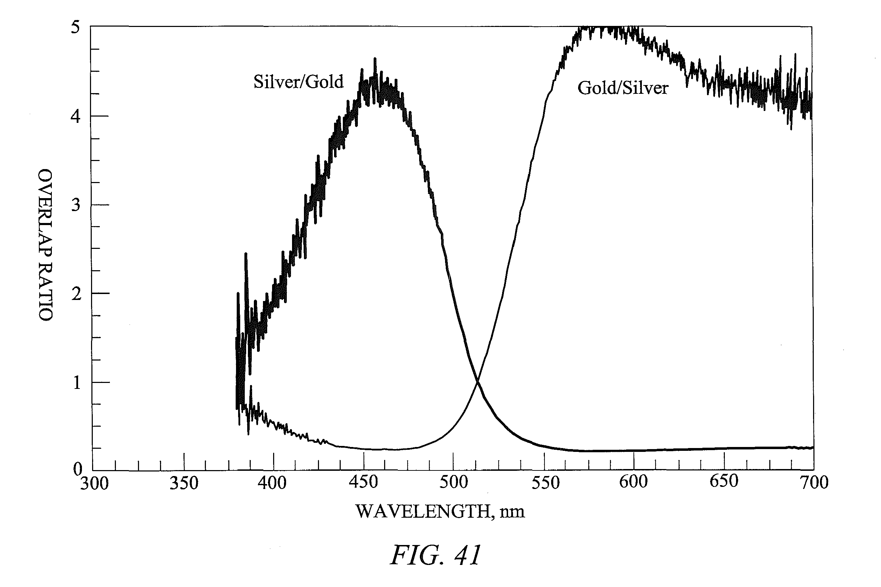

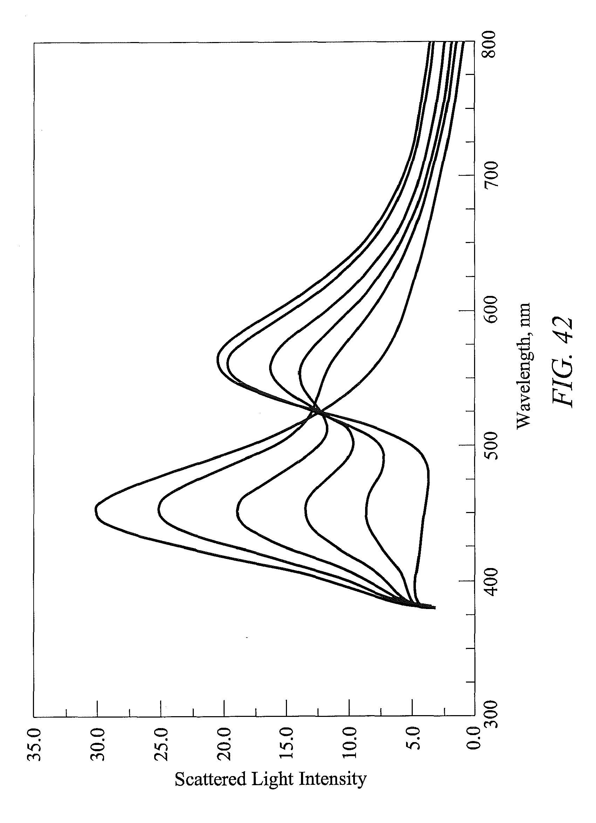

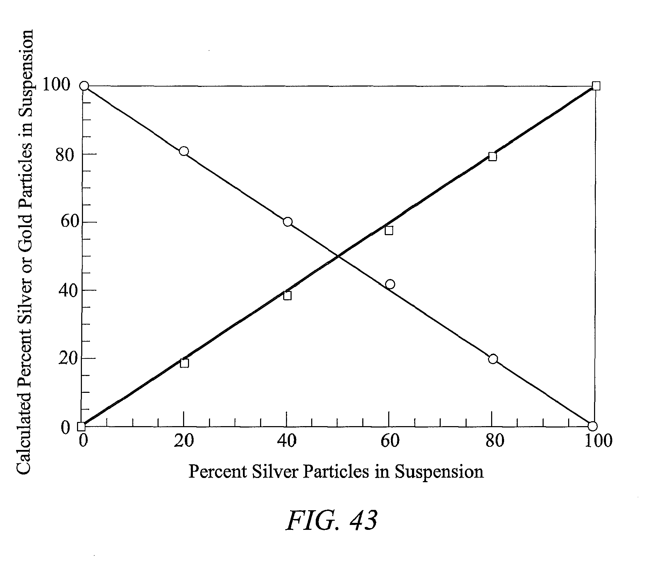

FIGs. 40-43 illustrate the theory of multiplexing. FIG. 40 shows corrected light scattering spectra of 60nm silver and 80 nm gold particles at 1.2 X $10^{-12}$ M concentration in water. FIG. 41 shows a plot of the extent to which the light scattering spectrum of 60nm silver particles overlaps the light scattering spectrum of gold, and vice versa. The overlap ratio = the light scattering intensity of 60nm silver particles/light scattering intensity of 80 nm gold; or light scattering intensity of 80nm gold /light scattering intensity of 60 nm silver particles. FIG. 42 shows scattered light intensity from mixtures of 80nm gold and 60 nm silver particles. Gold particle percentages are 0,20,40,60,80 and 100 in suspension. The 80 nm particle concentration in 100% gold suspension is 0.89 X 10-12 M and the 100% silver suspension is 1.33 X 10 -12 M. FIG. 43 shows a graph of calculated percent silver or gold particles in suspension as determined by applying multiplexed vs. actual percent of silver particles in suspension. The linear graphs with a 45 degree angle show that the calculated percent values agree very well with actual percent values.

Figure 40 shows corrected light scattering spectra of 60 nm (diameter) silver and 80 nm gold particles. Particle concentration is 1.2 x $10^{-12}$ M in water for both the silver and gold particle suspensions. The fact that the amplitudes of the light scattering graphs are approximately the same for both the silver and gold particle suspensions, at equal particle concentrations, indicates that the light scattering cross sections are approximately the same at their respective wavelengths of maximum light scattering.

In FIG. 41, graphs that show the extent to which the 60 nm silver and 80 nm gold particle spectra overlap at equal particle concentrations are presented. These graphs are plots of Silver Spectrum divided by Gold Spectrum and Gold Spectrum divided by Silver Spectrum (or the ratio silver spectrum/gold spectrum and the ratio gold spectrum/silver spectrum). The values of these ratios are a measure of spectral overlap and indicate the wavelengths which are preferred for two color multiplexing. Thus, the ratio silver spectrum/gold spectrum has a maximum at about 450 nm (which is then the preferred wavelength for silver detection) and the intensity of a silver particle at 450 is about 4.2 times greater than that of a gold particle. Similarly, the gold spectrum/silver spectrum ratio shows a maximum at about 580 nm and the intensity of a gold particle is 5 times greater at 580 than that of a silver particle.

FIG. 42 shows corrected light scattering spectra for different mixtures of 80 nm gold and 60 nm silver particles. The percent gold in each mixture is 0, 20, 40, 60, 80 and 100. Percent gold plus percent silver equal to 100%. The concentration of 100% gold is 0.89 x $10^{-12}$ M and the concentration of 100% silver is 1.33 x $10^{-12}$ M. The graphs clearly show the light scattering intensity maxima for the different silver and gold particle mixtures. They also show the spectral changes that occur as percent gold particle is increased. The spectra for the gold and silver particles are independent of each other. Only their amplitudes are affected when their concentrations are changed. The graphs display a scattering light intensity that is constant at the wavelength around 525 nm. This is an isosbestic point or wavelength and is the wavelength where the light scattering powers of the gold and particles are the same.

FIG. 43 shows plots that indicate the ability of the multiplexing methodology to evaluate the concentrations of 80 nm gold particles and 60 nm silver particles, in mixtures of these particles, from lights scattering intensity measurements at two wavelengths. The procedures were as follows. The intensities of the silver and gold mixtures of Fig. 3 were read from the spectra of Fig. 3 at 450 nm and 580 nm. The zero concentrations intensities were used as both sample and reference intensities. For each particle mixture the intensities at 450 nm and 580 nm for the sample and reference were introduced in the equations of 9 and 10 which yielded the values for the concentrations of silver, $C_{ag}$ and gold $C_{au}$ particles. % gold and % silver particle in each mixture was calculated with the expressions:

$$\%Gold = \frac{C_{gold}}{C_{gold} + C_{silver}}$$

$$\%Silver = \frac{C_{silver}}{C_{gold} + C_{silver}}$$

The results plotted in these figure as % Silver and % Gold calculated with the above expressions vs the actual % Silver in each mixture. The experimental points in both plots can be fitted to straight lines with effective slopes of 45˚. There is excellent agreement between calculated and actual percentages.

Example 6: Representative embodiments of Multispectral Deconvolution.

This example describes two representative implementations of multispectral deconvolution algorithms of the present invention. One embodiment is referred to as a stand-alone version, and the other is referred to as an embedded version.

The mathematical algorithms for linear algebra and the supporting facilities for image management were tested within ENVI software (available from Research Systems, Inc.) using the linear spectral unmixing feature. Having ascertained suitability, the linear spectral unmixing feature was extracted and used to generate a stand-alone version, and an embedded version of software for scattered light particle analysis. Reference file generation is a part of both the stand-alone and embedded implementations.

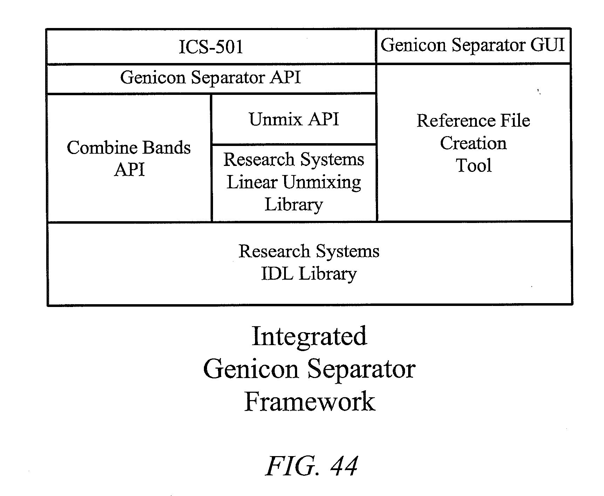

The embedded version unmixes RLS particles in an "on-the-fly" implementation inside RLS instrument control and capture software, such as has been described hereinabove. A framework of software components in an Integrated Genicon Separator is shown in FIG. 44.

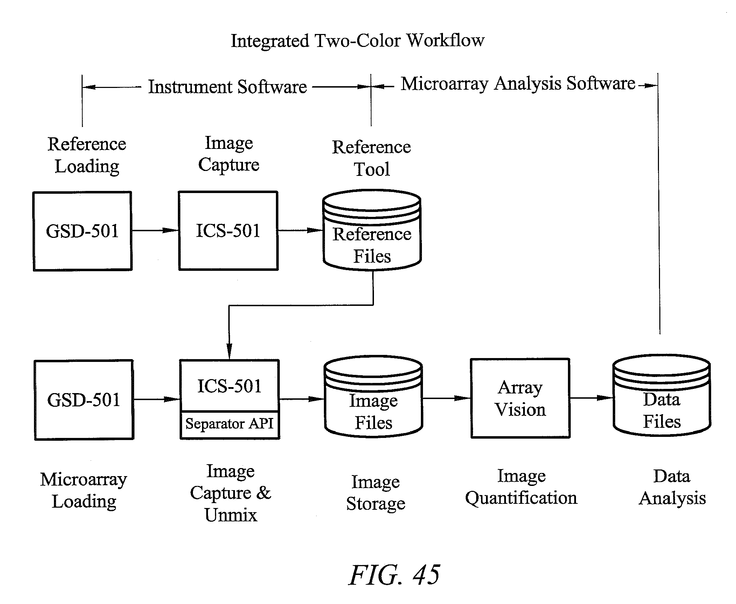

A representative "Integrated 2-Color Workflow", as shown in FIG. 45, uses a separate activity for reference file generation, and is ultimately used by the unmix process.

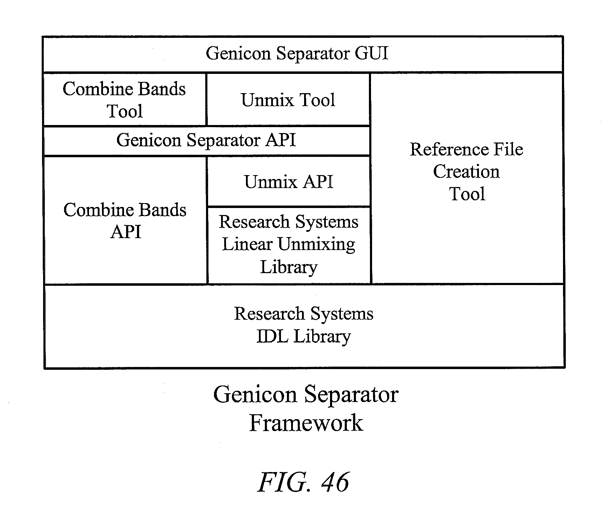

The spectral linear unmix feature from ENVI was initially implemented separately from the RLS instrument in a stand-alone version called "Genicon Separator", a layout of which is shown in FIG. 46, so that independent operation with file-based images could be accomplished.

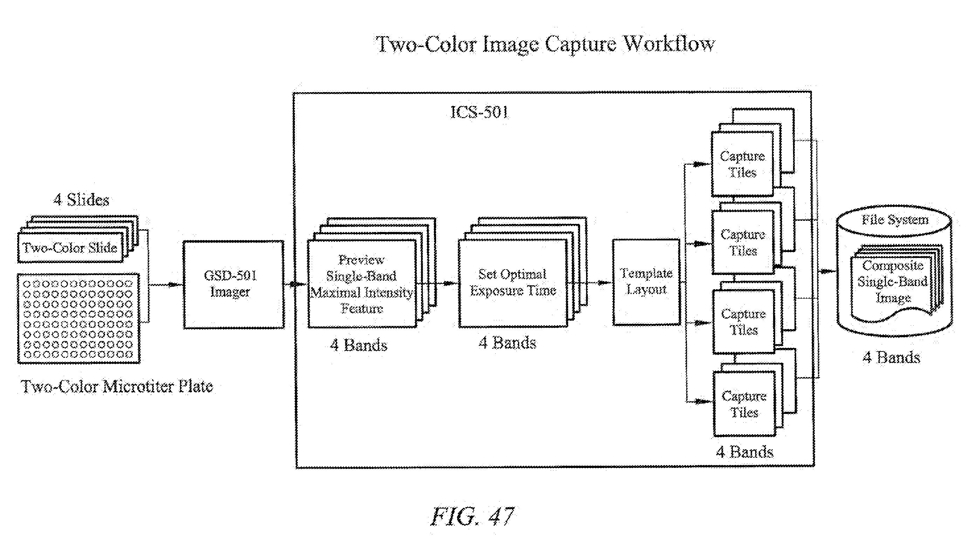

There are three principal activities attributed to the operation of unmixing RLS particles from imagery. One measures the RLS particle reference spectra; one measures the RLS particle mixture spectrum; and finally one solves the linear matrix coefficients in terms of intensity units. This process is accomplished by first building a band-image data cube. The data cube is generated for all multispectral imagery used by the system. This process transforms the raw intensity values into spectral values that have been normalized for exposure time and camera bias. The process of building a data cube is depicted in FIG. 47, which shows four band-images being collected on a tile-by-tile basis across a region (template) and formed into a set of composite images in a data cube structure, in the context of a two-color image capture workflow.

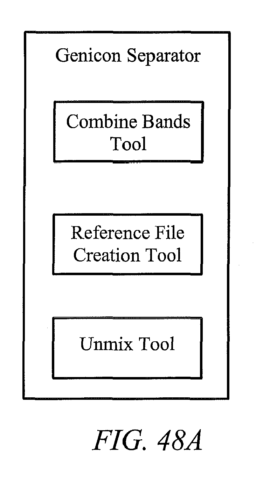

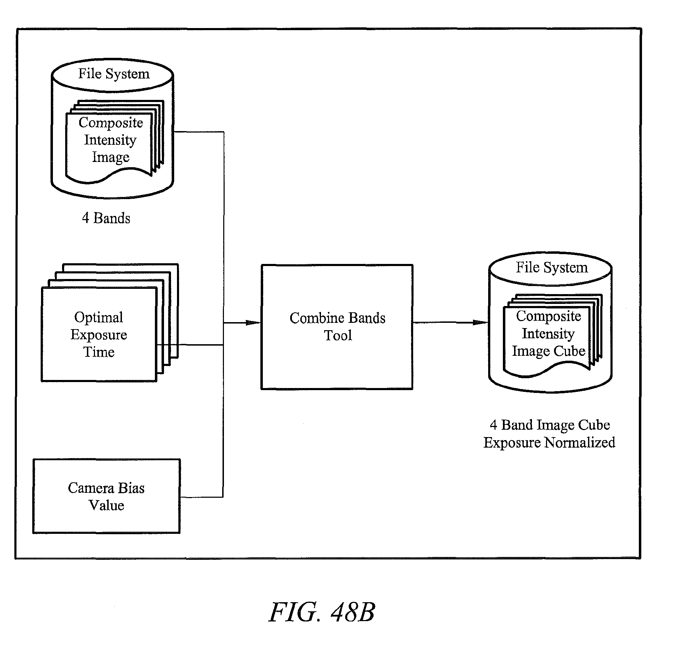

The stand-alone version was designed to operate three functions using a "Genicon Separator Framework" of software components as shown in FIG. 48A. In FIG. 48B, the combine bands tool imports individual filter images, or band-image files, and builds a data cube that is used by the reference file creation tool, and the unmix tool as a principal data structure for representing the spectra. The reference file creation tool is used to collect exemplary regions of interest from a data cube containing "pure" RLS particles. These regions of the image provide a statistical sampling for building the reference spectra. The unmix tool then uses the reference spectra in combination with a mixture image data cube to perform the linear spectral unmix function.

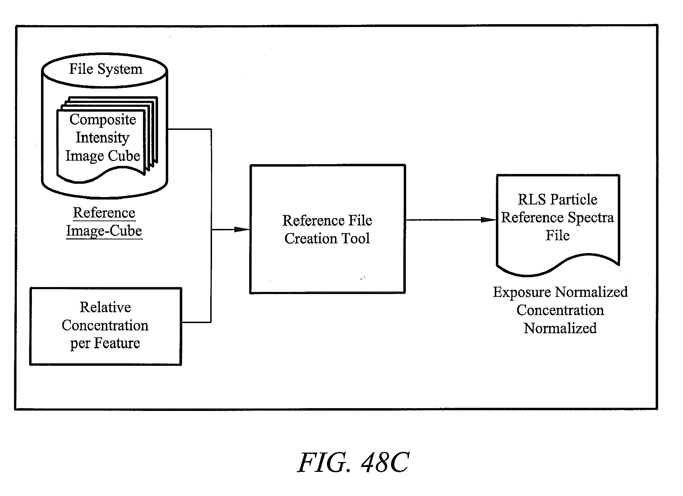

A reference image data cube is created from a pure RLS particle reference image set and sample spectra are derived from it using the reference file creation tool, see FIG. 48C. A relative concentration value is assigned to each feature selected from the reference image to desensitize the spectra concentration variations. However, the relative concentration preferably lies within the linear range of concentrations with respect to intensity, before it can be considered as a useful sample of the reference spectra.

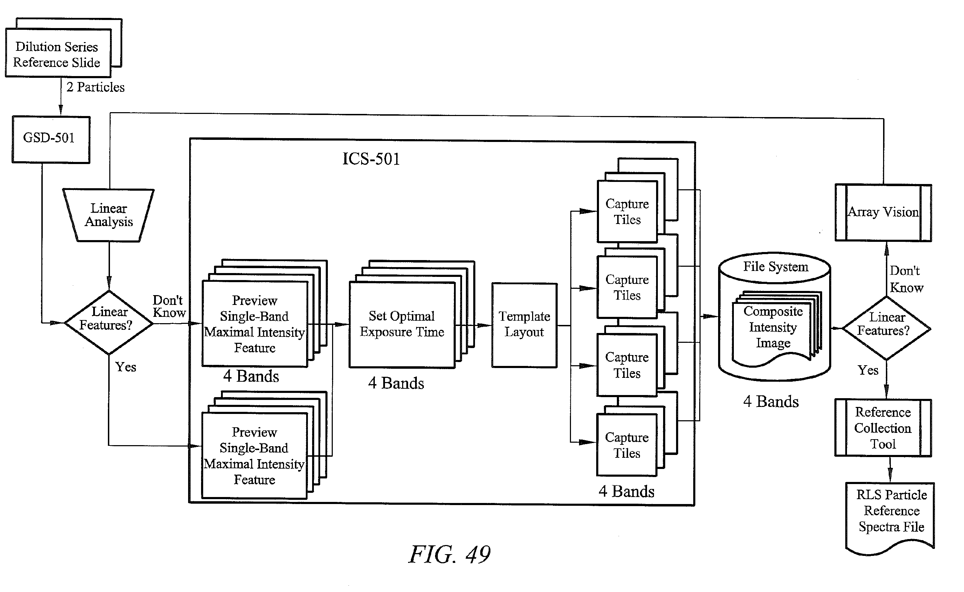

FIG. 49 depicts a process of determining the appropriate samples to use from an RLS particle reference image set. A linear analysis is conducted, via typical quantification means, before a sample is submitted to the reference creation tool for inclusion. As each feature is submitted, a cumulative statistical spectral value is generated for each band, which ultimately is used during the unmix process later on in the workflow.

Once the RLS particle reference file is complete, it may be used universally across mixtures comprised of the reference RLS particles. Spectral references to other types of material, such as dust, may be added to the reference spectra without special treatment.

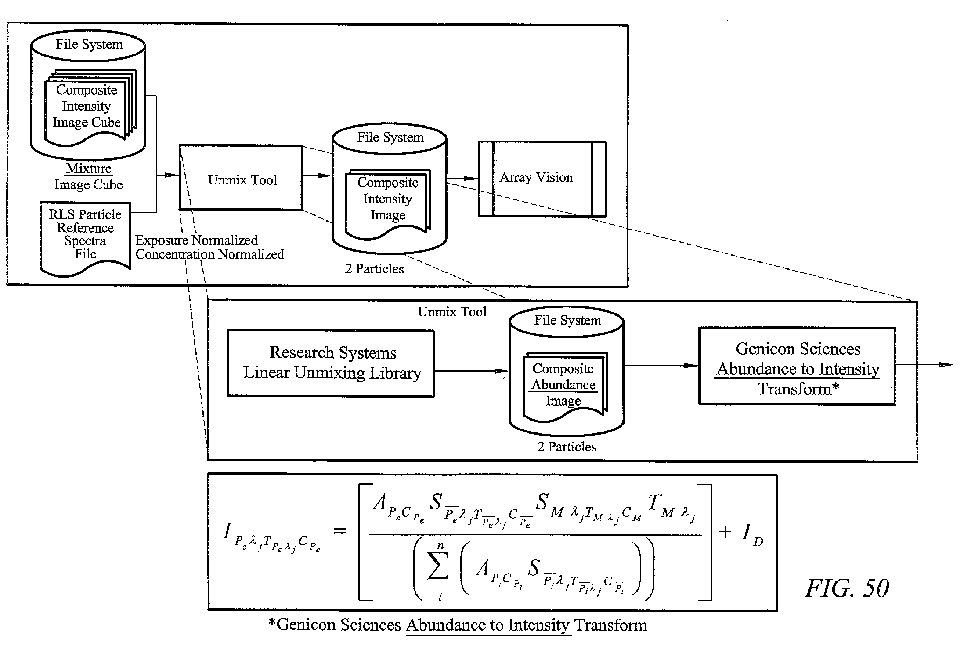

The unmix tool is, as shown in FIG. 50, comprised of commercial software components which generate "abundance" coefficients, then a Genicon-specific algorithm converts the coefficients into intensity units. The resulting transform results in an intensity image that appears "as if" it originated from a single RLS particle image. Although the abundance coefficients are completely accurate for the purpose of ratiometric comparative analysis, they are insufficient for the purpose of intensity-based quantification. Thus, the abundance to intensity transform serves to provide a means for users familiar with the art to quantify RLS particle intensity images in a more customary manner.

The RLS particle unmixing software module is integrated with the instrument control and capture software. The instrument's primary function is to accurately position the RLS particle media with respect to the optical path and to capture a single band-image tile from the CCD camera. A single band-image tile results from a single filter used during imaging. The instrument software organizes the individual tiles into a composite image; displays it to the end-user; and stores it in a computer file. Each band-image tile is also stored as a file during the image capture process and is deleted after the composite image is created.

A pre-selected filter group and spectral reference file is uniquely associated with the RLS particle mixture being unmixed. The software accesses this unique configuration during the "on-the-fly" process. The integrated version of software uses the same, yet reorganized set of software components as the stand-alone version. However, with the addition of the instrument control and capture software (ICS-501) the system is able to conduct all unmixing steps as the images are collected. The reference file creation tool remains as a stand-alone feature, but may be readily incorporated into the embedded version along with a dedicated automation protocol to enable reference spectra collection.

First, the instrument is instructed by the software to collect separate band-images across the specified region, one band-image per filter. The band-images are then combined into a data cube, which is a data structure used by the unmix software module to efficiently access the spatially coincident pixels from each band-image. The reference file, containing the spectral signatures of the RLS particles, along with the data cube is then used by the unmix software to perform the linear spectral unmix process, and abundance to intensity transformation. This results in one intensity image per RLS particle type. Efficient operation of this process, with regard for computer resources such as random access memory (RAM) usage, is achieved by using the unmix software on each set of band-image tiles, rather than using it on the composite image.

The following section of the description consists of numbered paragraphs simply providing statements of the invention already described herein. The numbered paragraphs in this section are not claims. The claims are set forth below in the later section headed "claims".

1. An assay system, comprising: at least one type of light scattering particles configured to be bound to an analyte of interest in a sample; and a scattered light detector configured to analyze analytes in the sample based on detected scattered light of at least first and second colors from the sample.

2. The assay system of 1, wherein the scattered light detector comprises: a sample holder configured to hold and position a sample containing the sample; an illumination system providing light to the sample; a light detector positioned with respect to the sample holder to detect scattered light in response to light provided by the illumination system; and an analyzer communicating with the light detector to analyze the analytes in the sample based on the detected light of the at least first and second colors.

3. The assay system of 1 or 2, wherein said analyzer comprises at least one processor and a memory, wherein said processor is configured to: accept spectral intensity data from a sample, wherein said spectral intensity data comprises signals from at least two types of light scattering particles, and wherein a first particle binds to a first analyte and a second particle binds to a second analyte; convert said spectral intensity data, using multi-spectral deconvolution, into a first intensity that corresponds to an abundance of said first particle, and a second intensity that corresponds to an abundance of said second particle; and quantify said first analyte from said first intensity and quantify said second analyte from said second intensity.

4. The assay system of any one of 1-3, wherein said multi-spectral deconvolution comprises using a first reference spectrum of a pure sample of said first particle, and a second reference spectrum of a pure sample of said second particle, or wherein said spectral intensity data is unmixed into amounts of said first reference spectrum and said second reference spectrum that are in proportion to, respectively, the abundance of said first particle, and the abundance of said second particle, orwherein said sample is in an array, or wherein said spectral values are corrected for spectral properties of the illumination and detection system.

5. The assay system of 4, wherein a spectral value for said sample comprises a linear sum of spectral values from said first and second particle types.

6. The assay system of any one of 2-5 wherein said illumination system is a tunable light source.

7. The assay system of 6 wherein said tunable light source comprises one or more LED's that together emit light of at least two different colors.

8. The assay system of any one of 2-7 wherein said illumination system comprises a broad-band light source in conjunction with at least two individually selectable spectrally discriminative light filters.

9. The assay system of 8 further comprising an illumination path between said illumination system and said sample holder, and a detection path between said sample holder and said detector, wherein said at least two individually selectable spectrally discriminative light filters are placed in one or more of the illumination path and the detection path.

10. The assay system of any one of 2-9 wherein said illumination system comprises a broad-band light source in conjunction with a tunable LCD spectrally discriminative light filter.

11. The assay system of 10 further comprising an illumination path between said illumination system and said sample holder, and a detection path between said sample holder and said detector, wherein said tunable LCD spectrally discriminative light filter is placed in one or more of the illumination path and the detection path.

12. The assay system of 4 wherein said array is a microarray.

13. The assay system of any one of 2-12, wherein said light detector is selected from the group consisting of : an objective lens microscope with a magnification from 0.5 to 500 times, confocal lens, and a photodetector.

14. The assay system of 13, wherein said photodetector is at least one detector selected from the group consisting of : photodiode, photomultiplier tube, photodiode array, charge coupled device, complementary metal oxide semiconductor, and a charge induction device.

15. The assay system of any one of 1-14, wherein said light scattering particles comprises particles of a first type and particles of a second type producing said first and second colors, respectively.

16. The assay system of 15, wherein said two types of particles are configured to be bound to different analytes of interest.

17. The system of any one of 1-16, wherein: said at least one type of particles produce scattered light of at least the first color; and said scattered light of at least the second color is produced by light scattering background sources.

18. The assay system of 17, wherein a first spectral profile of scattered light from said particles differs from a second spectral profile of scattered light from said background sources.

19. The assay system of 18, wherein the difference between said first and second spectral profiles is used to reduce background signal contribution.

20. The assay system of any one of 15-19, wherein said particles of a first type are gold particles, and/or wherein said particles of a second type are silver particles

21. The assay system of 20, wherein said gold particles are about 80 nm in diameter.

22. The assay system of 20, wherein said silver particles are about 60 nm in diameter.

23. The assay system of any one of 15-22, wherein a first spectral profile of scattered light from said particles of a first type differs from a second spectral profile of scattered light from said particles of a second type.

24. The assay system of 23, wherein said first spectral profile has a first peak wavelength, and said second spectral profile has a second peak wavelength at a different wavelength from said first peak wavelength.

25. The assay system of 24 wherein said first peak wavelength differs from said second peak wavelength by at least 100 nm.

26. The assay system of any one of 2-25, wherein said analyzer is configured to quantify at least one analyte through particle counting.

27. The assay system of any one of 2-26, wherein said analyzer is configured to quantify at least one analyte through integrated light intensity measurement, or wherein said analyzer is configured to quantify at least one analyte through particle counting and at least one analyte through integrated intensity measurement.

28. An apparatus for quantifying at least two types of analytes in an assay, said apparatus comprising at least one processor and a memory, wherein said processor is configured to:

   accept spectral intensity data from a sample, wherein said spectral intensity data comprises signals from at least two types of light scattering particle, and

   wherein a first particle binds to a first analyte and a second particle binds to a second analyte;

   convert said spectral intensity data, using multi-spectral deconvolution, into a first intensity that corresponds to an abundance of said first label, and a second intensity that corresponds to an abundance of said second label; and

   quantify said first analyte from said first intensity and a concentration of said second analyte from said second intensity.

29. An apparatus of 28, wherein said processor is additionally configured to use multi- spectral deconvolution to remove a background signal that is not due to a light scattering particle, from a signal due to an light scattering particle, or wherein said spectral intensity data is obtained using the defined isosbestic filter to improve the abundance to intensity conversion, or wherein spectral differences are a function of properties of the illumination and detection system, or wherein the multi-spectral deconvolution comprises using a first reference spectrum of a pure sample of said first particle, and a second reference spectrum of a pure sample of said second particle.

30. An analyzer for quantifying at least two types of analytes in multiplexed assays, comprising at least one processor and a memory, wherein said processor is configured to:

   accept spectral image data from a sample that includes two or more spectrally selective images, wherein said spectral image data is comprised of signals from two labels and wherein a first label binds to a first analyte and a second label binds to a second analyte;

   convert said two or more spectrally selective images into individual images that either contain only said first label or contain only said second label, using multispectral deconvolution; and

quantify said first analyte by means of particle counting from said individual images that contain only said first label, and said second analyte by means of particle counting from said individual images that contain only said second label.

31. A method for normalizing assay data, comprising:

selecting a first population of assay data and a second population of assay data, wherein said first population comprises a dependent set of controls and said second population comprises an independent set of controls;

obtaining a linear relationship between said independent set of controls and said dependent set of controls; and

applying said linear relationship to said first population, thereby producing normalized assay data.

32. The method of 31, wherein said first set of controls and said second set of controls comprise spots in an array, or wherein said first set of controls and said second set of controls are treated as equivalent.

33. The method of 31 or 32, wherein said linear relationship has a slope, m, and an intercept, b.

34. The method of any one of 31-33, wherein said obtaining comprises applying linear regression to said controls, wherein assay data for said first set of controls is treated as a dependent variable and assay data for said second set of controls is treated as an independent variable.

35. The method of 34, further comprising applying the linear relationship with slope $m$, and intercept $b$, to said dependent set of controls, thereby producing a transformed set of controls that, when plotted against the independent control set, results in a straight line that has a slope equal to one and an intercept equal to zero.

36. The method of 34, wherein said applying comprises producing a normalized set of assay data from said assay data for said first population, wherein a value, y' in said normalized set is obtained from a value, $y$, in said first population of assay data by the formula $y' = (y-b)/m.$

37. The method of any one of 31-36, wherein said assay data comprises microarray data.

38. The method of 37, wherein said first set of controls is disposed on a first microarray and said second set of controls is disposed on a second microarray, or wherein said first set of controls and said second set of controls are both disposed on the same microarray, or wherein said first set of controls comprises a first plurality of features, and said second set of controls comprises a second plurality of features such that each feature in said first plurality of features is subject to equivalent experimental conditions to a replicate feature in said second plurality of features.

39. The method of any one of 31-38, wherein said assay data comprises measurements of intensity of light scattered from light scattering particles, or wherein said assay data comprises measurements of intensity of light emitted by fluorescent labels.

40. A method of ratiometric analysis, performed on assay data that comprises an array of features, said method comprising:

selecting a first population of assay data and a second population of assay data, wherein said first population comprises a dependent set of controls and said second population comprises an independent set of controls;

obtaining a linear relationship between said independent set of controls and said dependent set of controls; and

applying said linear relationship to said first population of assay data, thereby producing a first normalized assay data;

obtaining a second linear relationship between said second set of controls and said first set of controls, wherein said first set of controls is treated as an independent variable, and said second set of controls is treated as a dependent variable in said linear relationship;

applying said second linear relationship to said second population of assay data, thereby producing a second normalized assay data;

calculating a ratio of a value of said feature in said normalized assay data to a value of said feature in said second normalized assay data; and

identifying said feature as regulated if said ratio exceeds a threshold value.

41. The method of 40, additionally comprising repeating said calculating for one or more other features.

42. The method of 40 or 41, wherein said threshold value is 2: 1.

43. A method of identifying at least one anomalous feature in assay data, wherein said assay data comprises an array of features, said method comprising:

dividing the assay data into a first population and a second population, wherein said first population comprises a first set of controls and said second population comprises a second set of controls;

obtaining a first linear relationship between said first set of controls and said second set of controls, including setting one set of controls as a dependent variable and the other set of controls as an independent variable;

obtaining a second linear relationship between said second set of controls and said first set of controls, wherein the control set that is treated as the dependent variable in obtaining said first linear relationship is treated as an independent variable in obtaining said second linear relationship;

applying said first linear relationship to said first population, thereby producing a first normalized assay data;

applying said second linear relationship to said second population, thereby producing a second normalized assay data; and

calculating a first ratio of a value of said feature in said first normalized assay data to a value of said feature in said second normalized assay data; and

calculating a second ratio of a value of said feature in said second normalized assay data to a value of said feature in said first normalized assay data; and

multiplying said first ratio by a reciprocal of said second ratio to produce a product;

identifying said feature as anomalous if said product exceeds a threshold value.

44. The method of 43, additionally comprising repeating said calculating and said multiplying for one or more other features.

45. The method of 43 or 44, wherein said threshold is 3.0.

46. The method of any one of 43-45, wherein said assay data comprises gene expression data and said anomalous feature indicates a difference in level of gene expression between first and second data, or wherein said assay data comes from a microarray, and said anomalous feature derives from a defect or artifact in said microarray.

47. A method for comparing a first set of assay data to a second set of assay data, comprising:

identifying a first set of controls in said first set of assay data and a second set of controls in said second set of data, wherein said first set of controls and said second set of controls are treated a equivalent;

obtaining a linear relationship between said first set of controls and said second set of controls;

applying said linear relationship to said first set of assay data, thereby transforming said first set of assay data into a third frame of reference;

applying said linear relationship to said second set of assay data, thereby transforming said second set of assay data into said third frame of reference; and

within said third frame of reference, comparing a feature from said first set of assay data that is not in said first set of controls, to a feature from said second set of assay data that is not in said second set of controls.

48. A method for performing an analyte assay, comprising:

detecting signals from a plurality of sites from an array format thereby producing assay data;

dividing the assay data into a first population and a second population, wherein said first population comprises a first set of controls and said second population comprises a second set of controls;

obtaining a linear relationship between said first set of controls and said second set of controls;

applying said linear relationship to said assay data, thereby producing normalized assay data; and

correlating said signals to an amount of analyte in each of said sites.

49. A system for normalizing assay data, comprising:

a detector that detects signals from discrete areas of said microarray and
produces microarray data; and
a computing device having embedded therein a set of instructions to transform said microarray data by:

selecting a first population of assay data and a second population of assay data, wherein said first population comprises a dependent set of controls and said second population comprises an independent set of controls;

obtaining a linear relationship between said independent set of controls and said dependent set of controls; and

applying said linear relationship to said first population, thereby producing normalized assay data.

50. The method of any one of 31-34, further comprising displaying said normalized microarray data in a visual format.

51. A computer readable medium having recorded therein a set of instructions for providing normalized microarray data, comprising instructions for:

selecting a first population of assay data and a second population of assay data, wherein said first population comprises a dependent set of controls and said second population comprises an independent set of controls;

obtaining a linear relationship between said independent set of controls and said dependent set of controls; and

applying said linear relationship to said first population, thereby producing normalized assay data.

52. The computer readable medium of 51, wherein said medium is selected from the group consisting of random access memory, read only memory, magnetic disk, magnetic tape, and optical disk.

**Claims**

1. A method for normalizing assay data, comprising:

selecting a first population of assay data and a second population of assay data, wherein said first population comprises a dependent set of controls and said second population comprises an independent set of controls;
obtaining a linear relationship between said independent set of controls and said dependent set of controls; and
applying said linear relationship to said first population, thereby producing normalized assay data.

**2.** The method of claim 1, wherein said first set of controls and said second set of controls comprise spots in an array, or wherein said first set of controls and said second set of controls are treated as equivalent.

**3.** The method of claim 1 or 2, wherein said linear relationship has a slope, m, and an intercept, b.

**4.** The method of any one of claim 1- 3, wherein said obtaining comprises applying linear regression to said controls, wherein assay data for said first set of controls is treated as a dependent variable and assay data for said second set of controls is treated as an independent variable.

**5.** The method of claim 4, further comprising applying the linear relationship with slope $m$, and intercept $b$, to said dependent set of controls, thereby producing a transformed set of controls that, when plotted against the independent control set, results in a straight line that has a slope equal to one and an intercept equal to zero.

**6.** The method of claim 4, wherein said applying comprises producing a normalized set of assay data from said assay data for said first population, wherein a value, $y'$ in said normalized set is obtained from a value, $y$, in said first population of assay data by the formula $y'= (y-b)/m$.

**7.** The method of any one of claims 1- 6, wherein said assay data comprises microarray data.

**8.** The method of claim 7, wherein said first set of controls is disposed on a first microarray and said second set of controls is disposed on a second microarray, or wherein said first set of controls and said second set of controls are both disposed on the same microarray, or wherein said first set of controls comprises a first plurality of features, and said second set of controls comprises a second plurality of features such that each feature in said first plurality of features is subject to equivalent experimental conditions to a replicate feature in said second plurality of features.

**9.** The method of any one of claims 1- 8, wherein said assay data comprises measurements of intensity of light scattered from light scattering particles, or wherein said assay data comprises measurements of intensity of light emitted by fluorescent labels.

**10.** A method of ratiometric analysis, performed on assay data that comprises an array of features, said method comprising:

selecting a first population of assay data and a second population of assay data, wherein said first population comprises a dependent set of controls and said second population comprises an independent set of controls; obtaining a linear relationship between said independent set of controls and said dependent set of controls; and applying said linear relationship to said first population of assay data, thereby producing a first normalized assay data; obtaining a second linear relationship between said second set of controls and said first set of controls, wherein said first set of controls is treated as an independent variable, and said second set of controls is treated as a dependent variable in said linear relationship; applying said second linear relationship to said second population of assay data, thereby producing a second normalized assay data; calculating a ratio of a value of said feature in said normalized assay data to a value of said feature in said second normalized assay data; and identifying said feature as regulated if said ratio exceeds a threshold value.

**11.** The method of claim 10, additionally comprising repeating said calculating for one or more other features.

**12.** The method of claim 10 or 11, wherein said threshold value is 2: 1.

**13.** A method of identifying at least one anomalous feature in assay data, wherein said assay data comprises an array of features, said method comprising:

dividing the assay data into a first population and a second population, wherein said first population comprises a first set of controls and said second population comprises a second set of controls; obtaining a first linear relationship between said first set of controls and said second set of controls, including setting one set of controls as a dependent variable and the other set of controls as an independent variable; obtaining a second linear relationship between said second set of controls and said first set of controls, wherein the control set that is treated as the dependent variable in obtaining said first linear relationship is treated as an independent variable in obtaining said second linear relationship;

applying said first linear relationship to said first population, thereby producing a first normalized assay data;

applying said second linear relationship to said second population, thereby producing a second normalized assay data; and

calculating a first ratio of a value of said feature in said first normalized assay data to a value of said feature in said second normalized assay data; and

calculating a second ratio of a value of said feature in said second normalized assay data to a value of said feature in said first normalized assay data; and multiplying said first ratio by a reciprocal of said second ratio to produce a product;

identifying said feature as anomalous if said product exceeds a threshold value.

14. The method of claim 13, wherein said assay data comprises gene expression data and said anomalous feature indicates a difference in level of gene expression between first and second data, or wherein said assay data comes from a microarray, and said anomalous feature derives from a defect or artifact in said microarray.

15. A method for comparing a first set of assay data to a second set of assay data, comprising:

identifying a first set of controls in said first set of assay data and a second set of controls in said second set of data, wherein said first set of controls and said second set of controls are treated a equivalent;

obtaining a linear relationship between said first set of controls and said second set of controls;

applying said linear relationship to said first set of assay data, thereby transforming said first set of assay data into a third frame of reference;

applying said linear relationship to said second set of assay data, thereby transforming said second set of assay data into said third frame of reference; and

within said third frame of reference, comparing a feature from said first set of assay data that is not in said first set of controls, to a feature from said second set of assay data that is not in said second set of controls.

FIG. 1

FIG. 2

FIG. 3

*FIG. 4A*

*FIG. 4B*

EP 2 302 363 A2

10

Scanner

| Memory | CPU |

404 — 402 — 100

30

CPU 102

Memory 108

Operating System ~110

File System ~112

Software Suite ~114

Instrument Software ~400

Scanner Control Module ~410

Image Capture Module ~420

Microarray Analysis Software ~126

Image Quantification Module ~128

Data Analysis Module ~130

Data Files ~450

Image Files ~430

106

User Interface

20

40

150

134

Network Interface

136

Remote Database

FIG 5A

*890*

*888*

Image Processing
Workstation #1
*ArrayVision*

Image Processing
Workstation #2
*ArrayVision*

NETWORK

*886*

*880*

Genicon
RLS
Scanner

*884*

*882*

Image Capture
Workstation
*Maximum DL/CCD
RLS Scanner
ArrayVision*

*FIG. 5B*

*FIG. 6*

*FIG. 7*

652 — Collect spectra of pure particles

654 — Normalize to equal molar concentration

656 — Normalize to 1nm bandwidth

658 — Produce and analyze difference spectra

660 — Select filters in regions of max and min

662 — Capture images using selected filters

664 — Analyze linearity of umixed result

650

*FIG. 8*

Au & Ag Energy Spectra Normalized
to Photon Energy per Mole

*FIG. 9*

FIG. 10

FWHM (nm) vs. Filter Bandwidth (nm)

FIG. 11

Integral Energy (kJ/mol) per Filter
Bandwidth (nm) vs. Wavelength (nm)

*FIG. 12*

Difference of Integral Energy (%)
vs. Wavelength (nm)

*FIG. 13*

FIG. 14

*FIG. 15*

EP 2 302 363 A2

Ag +Au

309-0.39-0.20-0.33-0.14
530nmX40nm.tif

780

Ag

297-0.39-0.20-0.33-0.23
450nmX70nm.tif

782

Au

304-2.40-0.60-0.60-0.14
450nmX70nm.tif

784

*FIG. 16*

EP 2 302 363 A2

*FIG. 17*

FIG. 18

FIG. 19

**Off-Slide References Applied to Mixture**

*Reference spectra are calibrated to Instrument once & remain fixed".*

**On-Slide References Applied to Mixture**

*Reference spectra are calibrated for each slide.*

*FIG. 20*

*Predominate Wavelength*

ROI Tool

End-Member Intensity Image Cubes — 602

Calculate Average Intensity of ROI's per End-Member — 604

Convert Intensity to Spectral Values — 606

End-Member Spectral Values — 608

610

Mixture Intensity Image Cube — 623

Convert Intensity to Spectral Values — 622

Mixture Spectral Values Image Cube — 624

RSI Linear Unmix Module — 626

628

Convert Abundance to Predominate Intensity per End-Member — 630

600

*FIG. 21*

*FIG. 22*

Au
100
80
60
50
40
20
0

640

Gold

Ag
0
20
40
50
60
80
100

642

Silver

*FIG. 23*

EP 2 302 363 A2

Abundance Ratio Image Generation

FIG. 24

Image Capture

Microarray
quantification

Linear Quality Assurance

Linear Transform
Normalization

FIG. 25

FIG. 26

FIG. 27

FIG. 28

Comparison of Linear Normalization Techniques
Slide 0

FIG. 29A

Comparison of Linear Normalization Techniques
Slide 1

*FIG. 29B*

Comparison of Linear Normalization Techniques
Average Expression Range

FIG. 30A

Comparison of Linear Normalization Techniques
Fold Change

Legend:
- No Normalization
- InterSlide Normalization
- InterGroup Normalization
- InterSlide IntraGroup
- InterGroup IntraGroup
- Standard Deviation

Fold Change
Log[ABS
(Signal S1/
Signal S0)]

Microarray Feature ID

Standard Deviation

A: 0.296
B: 0.181
C: 0.053
D: 0.054
E: 0.049

Y-axis values: 0.800, 0.700, 0.600, 0.500, 0.400, 0.300, 0.200, 0.100, 0.000, -0.100, -0.200, -0.300, -0.400, -0.500, -0.600, -0.700

*FIG. 30B*

105

Comparison of Linear Normalization Techniques
Slide 0

Legend:
- ☑ No Normalization
- ◪ InterSlide Normalization
- ▨ InterGroup Normalization
- ◩ InterSlide IntraGroup
- ▨ InterGroup IntraGroup
- ▩ Standard Deviation

Y-axis (left): Average Expression Value — 18000, 16000, 14000, 12000, 10000, 8000, 6000, 4000, 2000, 0, -2000, -4000

X-axis: Microarray Feature ID — A, B, C, D, E

Standard Deviation values: A: 488, B: 154, C: 150, D: 178, E: 37

Right axis: Standard Deviation

*FIG. 31A*

EP 2 302 363 A2

Comparison of Linear Normalization Techniques
Slide 1

FIG. 31B

Comparison of Linear Normalization Techniques
Average Expression Range

FIG. 32A

Comparison of Linear Normalization Techniques
Fold Change

*FIG. 32B*

FIG. 33

Comparison of No Normalization to InterSlide
Normalization using Data Set "b" as S0

$y = 0.7512x + 4059.7$

$R^2 = 0.485$

S1
S1'
Linear (S1)
Linear (S1')

MTM Density S0

MTM Density S1 InterSlide Normalized MTM Density S1'

*FIG. 34*

Comparison of Linear Normalization Applied
to Reciprocal Control Set

FIG. 35A

Product of Normalization Ratios per Array Feature

FIG. 35B

EP 2 302 363 A2

*FIG. 36*

**High Intensity Dust Spectrum**

*FIG. 37A*

**Med Intensity Dust Spectra**

*FIG. 37B*

FIG. 37C

FIG. 38

Initial Image 600nm 1sec     Unmixed Au Image     Unmixed Dust Image

*FIG. 39*

*FIG. 40*

EP 2 302 363 A2

FIG. 41

*FIG. 42*

FIG. 43

| ICS-501 | Genicon Separator GUI |
|---|---|
| Genicon Separator API | |

| Combine Bands API | Unmix API | Reference File Creation Tool |
|---|---|---|
| | Research Systems Linear Unmixing Library | |

| Research Systems IDL Library |
|---|

Integrated
Genicon Separator
Framework

*FIG. 44*

EP 2 302 363 A2

Integrated Two-Color Workflow

|←——— Instrument Software ———→|←— Microarray Analysis Software —→|

Reference
Loading

Image
Capture

Reference
Tool

GSD-501 → ICS-501 → Reference Files

GSD-501 → ICS-501 | Separator API → Image Files → Array Vision → Data Files

Microarray
Loading

Image
Capture &
Unmix

Image
Storage

Image
Quantification

Data
Analysis

*FIG. 45*

| Genicon Separator GUI | | |
|---|---|---|
| Combine Bands Tool | Unmix Tool | Reference File Creation Tool |
| Genicon Separator API | | |
| Combine Bands API | Unmix API | |
| | Research Systems Linear Unmixing Library | |
| Research Systems IDL Library | | |

Genicon Separator
Framework

*FIG. 46*

EP 2 302 363 A2

Two-Color Image Capture Workflow

FIG. 47

Genicon Separator

Combine Bands Tool

Reference File Creation Tool

Unmix Tool

*FIG. 48A*

*FIG. 48B*

*FIG. 48C*

*FIG. 49*

FIG. 50

*Genicon Sciences Abundance to Intensity Transform

$$I_{P_e \lambda_j T_{P_e \lambda_j} C_{P_e}} = \left[ \frac{A_{P_e C_{P_e}} S_{\overline{P_e} \lambda_j T_{\overline{P_e} \lambda_j} C_{\overline{P_e}}} S_{M \lambda_j T_{M \lambda_j} C_M} T_{M \lambda_j}}{\left( \sum_i^n \left( A_{P_i C_{P_i}} S_{\overline{P_i} \lambda_j T_{\overline{P_i} \lambda_j} C_{\overline{P_i}}} \right) \right)} \right] + I_D$$

EP 2 302 363 A2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 31754301 P
- US 60364962 B
- US 60376049 B
- US 6214560 B, Yguerabide
- US 9706584 W
- WO 9740181 A
- US 9823160 W
- WO 9920789 A

- US 317543 P
- US 60376049 P
- US 37604902 P
- US 6214560 A
- US 5350697 A, Swope
- US 10084844 B

**Non-patent literature cited in the description**

- **YGUERABIDE ; YGUERABIDE.** *Anal. Biochem.,* 1998, vol. 261, 157-176
- *Anal. Biochem.,* 1998, vol. 262, 137-156
- **CHEWINGS, V. H. ; PICKUP, G. ; BASTIN, G. N. ; PEARCE, G.** *The potential for hyperspectral data mapping in Australian arid-zone vegetation 10th Australasian Remote Sensing and Photogrammetry Conference,* 2000, 851-863
- **HERMANN, J. A. ; SHETTIGARA, V. K. ; HAWTHORN, C.** Full and partial unmixing of hyperspectral images for target detection. *Proc. 10th Australasian Remote Sensing and Photogrammetry Conference,* 2000
- **SHETTIGARA, V. K. ; O'MARA, D. ; BUBNER, T. ; KEMPINGER, S. G.** Hyperspectral band selection using entropy and target to clutter ratio measures. *Proc. 10th Australasian Remote Sensing and Photogrammetry Conference,* 2000
- **BAO, P. ; FRUTOS, A. ; GREEF, C. ; LAHIRI, J. ; MULLER, U. ; PETERSON, T. ; WARDEN, L. ; XIE, X.** High-Sensitivity detection of DNA hybridization on microarrays using resonance light scattering. *Anal. Chem.,* 2002, vol. 74, 1792-1797
- **SCHENA, M. ; SHALON, D. ; DAVIS, R. W. ; BROWN, P. O.** Quantitative monitoring of gene expression patterns with a complementary DNA microarray. *Science,* 1995, vol. 270, 467-70
- **SCHUCHHARDT, J. ; BEULE, D. ; MALIK, A. et al.** Normalization Strategies for cDNA Microarrays. *Nucl. Acids Res.,* 2000, vol. 28, e47
- **SCHUCHHARDT et al.** *Nucl. Acids Res.,* 2000, vol. 28, e47

- **COLANTUONI, C. ; HENRY, G. ; ZEGER, S. ; PEVSNER, J.** Local mean normalization of microarray element signal intensities across an array surface: quality control and correction of spatially systematic artifacts. *Biotechniques,* June 2002, vol. 32 (6), 1316-20
- **FINKELSTEIN et al.** Iterative linear regression by sector: renormalization of cDNA microarray data and cluster analysis weighted by cross homology. *Critical Assessment of Microarray Data Analysis,* 2000
- **STIMPSON et al.** *Proc. Natl. Acad. Sci. USA,* 1995, vol. 92, 6379-6383
- **DJEMEL ZIOU ; SALVATORE TABBONE.** *Edge Detection Techniques - An Overview, Technical Report,* 1997
- **J. YGUERABIDE ; E. E. YGUERABIDE.** Light-Scattering Submicroscopic particles as Highly Fluorescent Analogs and Their Use as Traced Labels in Clinical and Biological Applications - I. Theory. *Anal. Biochem.,* 1998, vol. 262, 137-156
- **J. YGUERABIDE ; E. E. YGUERABIDE.** Light-Scattering Submicroscopic particles as Highly Fluorescent Analogs and Their Use as Traced Labels in Clinical and Biological Applications. II. Experimental Characterization. *Anal. Biochem.,* 1998, vol. 262, 157-176
- **J. YGUERABIDE ; E. E. YGUERABIDE.** *Anal. Biochem.,* 1998, vol. 262, 157-176
- **E. HECHT ; A. ZAYOC.** Optics. Addison-Wesley Publishing Co, 1979